(19) **Europäisches Patentamt / European Patent Office / Office européen des brevets**

(11) **EP 4 190 353 A1**

(12) **EUROPEAN PATENT APPLICATION**
published in accordance with Art. 153(4) EPC

(43) Date of publication:
**07.06.2023 Bulletin 2023/23**

(21) Application number: **21849923.4**

(22) Date of filing: **30.07.2021**

(51) International Patent Classification (IPC):
***A61K 39/395*** *(2006.01)*     ***A61P 35/00*** *(2006.01)*

(52) Cooperative Patent Classification (CPC):
**A61K 39/395; A61P 35/00**

(86) International application number:
**PCT/CN2021/109438**

(87) International publication number:
**WO 2022/022660 (03.02.2022 Gazette 2022/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.07.2020 CN 202010755954**
**22.07.2021 CN 202110831575**

(72) Inventors:
• **MO, Xiyele**
**Shanghai 200245 (CN)**
• **YAN, Zhen**
**Shanghai 200245 (CN)**
• **LIU, Xun**
**Shanghai 200245 (CN)**

(74) Representative: **Regimbeau**
**20, rue de Chazelles**
**75847 Paris Cedex 17 (FR)**

(71) Applicants:
• **Jiangsu Hengrui Pharmaceuticals Co., Ltd.**
**Lianyungang, Jiangsu 222047 (CN)**
• **Shanghai Hengrui Pharmaceutical Co., Ltd.**
**Shanghai 200245 (CN)**

Remarks:
The complete document including Reference Table(s) and the Sequence Listing(s) can be downloaded from the EPO website

(54) **ANTI-PD-1 ANTIBODY PHARMACEUTICAL COMPOSITION AND USE THEREOF**

(57) The present disclosure relates to an anti-PD-1 antibody pharmaceutical composition and use thereof. Specifically, the present disclosure provides a pharmaceutical composition, comprising an anti-PD-1 antibody and a buffer solution.

EP 4 190 353 A1

**Description**

**TECHNICAL FIELD**

The present disclosure belongs to the field of pharmaceutical formulations, and in particular relates to a pharmaceutical composition comprising an anti-PD-1 antibody, and use thereof as a medicament.

**BACKGROUND**

The statements herein merely provide background information related to the present disclosure and may not necessarily constitute the prior art.

Tumor immunotherapy is a treatment method that fully utilizes and mobilizes killer T cells in a tumor patient to kill the tumor. Meanwhile, the escape of tumor cells is a huge obstacle with which tumor immunotherapy is faced, and the tumor cells promote the rapid growth of tumors by utilizing their inhibition of the immune system. There is a very complex relationship between the immune escape mechanism of tumors and the immune response of the body to tumors. The tumor-specific killer T cells have bioactivity in the early stage of tumor immunotherapy, but as the tumor grows, they lose the killing function in the later stage.

There is a system of two signaling pathways for the activation of T cells in the human body. In addition to providing a first signal to T cells by presenting MHC-antigen peptides via antigen-presenting cells, a series of co-stimulatory molecules are also needed to provide a second signal, thereby enabling the T cells to generate a normal immune response. This dual-signaling pathway system plays a vital role in the homeostasis of the immune system in the body by strictly regulating the body's different immune responses to self and non-self antigens. The absence of a second signal provided by co-stimulatory molecules will result in either no response or a sustained specific immune response of T cells, leading to tolerance. Thus, the second signaling pathway plays a very critical regulatory role throughout the body's immune response. Programmed death-1 (PD-1) is a protein receptor found on the surface of T cells in 1992, which is involved in the process of cell apoptosis. PD-1 belongs to the CD28 family and has 23% amino acid identity with cytotoxic T lymphocyte antigen 4 (CTLA-4). However, PD-L is mainly expressed on activated T cells, B cells and myeloid cells, which is different from CTLA. PD-1 has two ligands, i.e., PD-L1 and PD-L2. PD-L1 is mainly expressed on T cells, B cells, macrophages and dendritic cells (DCs), and its expression can be upregulated on activated cells. In contrast, PD-L2 has a relatively limited expression, and is mainly expressed on antigen-presenting cells, such as activated macrophages and dendritic cells.

The anti-PD-1 antibody can furthest improve the immune system response of a patient to a tumor by blocking the PD-L1/PD-1 binding, thereby achieving the purpose of killing tumor cells.

**SUMMARY**

The present disclosure provides a pharmaceutical composition comprising an anti-PD-1 antibody and use thereof.

In one aspect, the present disclosure provides a pharmaceutical composition comprising an anti-PD-1 antibody and a buffer, wherein the buffer is an acetate buffer, a histidine salt buffer, or a succinate buffer; preferably, the buffer has a pH of about 4.5 to about 6.0, preferably a pH of about 4.7 to about 5.7, and more preferably a pH of about 5.2; wherein the acetate buffer is preferably an acetic acid-sodium acetate buffer, the histidine salt buffer is preferably a histidine-acetate buffer, and the succinate buffer is preferably a succinic acid-sodium succinate buffer; the anti-PD-1 antibody comprises a heavy chain variable region and light chain variable region, wherein the heavy chain variable region comprises an HCDR1 set forth in SEQ ID NO: 65, an HCDR2 set forth in SEQ ID NO: 66, and an HCDR3 set forth in SEQ ID NO: 67, and the light chain variable region comprises an LCDR1 set forth in SEQ ID NO: 68, an LCDR2 set forth in SEQ ID NO: 12, and an LCDR3 set forth in SEQ ID NO: 69; preferably, wherein the heavy chain variable region comprises an HCDR1 set forth in SEQ ID NO: 8, an HCDR2 set forth in SEQ ID NO: 9, and an HCDR3 set forth in SEQ ID NO: 10, and the light chain variable region comprises an LCDR1 set forth in SEQ ID NO: 49, an LCDR2 set forth in SEQ ID NO: 12, and an LCDR3 set forth in SEQ ID NO: 13. The specific sequences are shown in Table 1 below:

Table 1. Antibody CDR sequences

| Heavy chain | | Light chain | |
| --- | --- | --- | --- |
| HCDR1 | DYE $X_1$H, wherein $X_1$ is selected from the group consisting of I and M; (SEQ ID NO: 65) | LCDR1 | RSSQS$X_{13}$VHS$X_{14}X_{15}X_{16}$TY LE, wherein $X_{13}$ is selected from the group consisting of I and L, $X_{14}$ is selected from the group consisting of N, Q, L, T and D, $X_{15}$ is selected from the group consisting of G, A and V, and $X_{16}$ is selected from the group consisting of N and K; (SEQ ID NO: 68) |
| HCDR2 | L$X_2$DPETGG$X_3$VYNQKFK$X_4$, wherein $X_2$ is selected from the group consisting of F and I, $X_3$ is selected from the group consisting of I and T, and $X_4$ is selected from the group consisting of G and D; (SEQ ID NO: 66) | LCDR2 | KVSNRFS; (SEQ ID NO: 12) |
| HCDR3 | E$X_5X_6X_7X_8$Y$X_9X_{10}X_{11}X_{12}$DWYFDV, wherein $X_5$ is selected from the group consisting of G and R, $X_6$ is F or vacancy, $X_7$ is S or vacancy, $X_8$ is Y or vacancy, $X_9$ is G or vacancy, $X_{10}$ is S or vacancy, $X_{11}$ is selected from the group consisting of N and T, and $X_{12}$ is selected from the group consisting of R and S; (SEQ ID NO: 67) | LCDR3 | FQGSHVPY$X_{17}$, wherein $X_{17}$ is selected from the group consisting of A and T; (SEQ ID NO: 69) |

In some alternative embodiments, in the pharmaceutical composition described above, the anti-PD-1 antibody comprises a heavy chain variable region and a light chain variable region, wherein the heavy chain variable region comprises an HCDR1 having a sequence set forth in SEQ ID NO: 8, an HCDR2 having a sequence set forth in SEQ ID NO: 9, and an HCDR3 having a sequence set forth in SEQ ID NO: 10; and the light chain variable region comprises an LCDR2 having a sequence set forth in SEQ ID NO: 12, an LCDR3 having a sequence set forth in SEQ ID NO: 13, and an LCDR1 having a sequence set forth in general formula RSSQS$X_{13}$VHS$X_{14}X_{15}X_{16}$TYLE (SEQ ID NO: 68), wherein $X_{13}$ is selected from L, $X_{14}$ is selected from the group consisting of N, Q, L, T and D, $X_{15}$ is selected from the group consisting of G, A and V, and $X_{16}$ is selected from N.

In some alternative embodiments, in the pharmaceutical composition described above, the anti-PD-1 antibody is an anti-PD-1 antibody selected from any one of the following (a) to (e):

(a) an anti-PD-1 antibody comprising an HCDR1, an HCDR2 and an HCDR3 having sequences set forth in SEQ ID NO: 8, SEQ ID NO: 9 and SEQ ID NO: 10, respectively, an LCDR2 and an LCDR3 having sequences set forth in SEQ ID NO: 12 and SEQ ID NO: 13, respectively, and an LCDR1 having a sequence set forth in SEQ ID NO: 11, 47, 48, 49, 50, 51 or 52;

(b) an anti-PD-1 antibody comprising an HCDR1, an HCDR2 and an HCDR3 having sequences set forth in SEQ ID NO: 14, SEQ ID NO: 15 and SEQ ID NO: 16, respectively, and an LCDR1, an LCDR2 and an LCDR3 having sequences set forth in SEQ ID NO: 17, SEQ ID NO: 12 and SEQ ID NO: 18, respectively;

(c) an anti-PD-1 antibody comprising an HCDR1, an HCDR2 and an HCDR3 having sequences set forth in SEQ ID NO: 21, SEQ ID NO: 22 and SEQ ID NO: 23, respectively, an LCDR1, an LCDR2, and an LCDR3 having sequences set forth in SEQ ID NO: 24, SEQ ID NO: 25 and SEQ ID NO: 26, respectively;

(d) an anti-PD-1 antibody comprising an HCDR1, an HCDR2 and an HCDR3 having sequences set forth in SEQ ID NO: 14, SEQ ID NO: 15 and SEQ ID NO: 16, respectively, an LCDR2 and an LCDR3 having sequences set forth in SEQ ID NO: 12 and SEQ ID NO: 13, respectively, and an LCDR1 having a sequence set forth in SEQ ID NO: 11, 47, 48, 49, 50, 51 or 52; and

(e) a heavy chain variable region comprising an HCDR1, an HCDR2 and an HCDR3 set forth in SEQ ID NO: 8, SEQ ID NO: 9 and SEQ ID NO: 10, respectively, and a light chain variable region comprising an LCDR1, an LCDR2 and an LCDR3 set forth in SEQ ID NO: 17, SEQ ID NO: 12 and SEQ ID NO: 18, respectively.

In some embodiments, in the pharmaceutical composition described above, the anti-PD-1 antibody comprises a heavy chain variable region and a light chain variable region, wherein the heavy chain variable region comprises an HCDR1, an HCDR2 and an HCDR3 having sequences set forth in SEQ ID NO: 8, SEQ ID NO: 9 and SEQ ID NO: 10, respectively, and the light chain variable region comprises an LCDR1, an LCDR2 and an LCDR3 having sequences set forth in SEQ ID NO: 49, SEQ ID NO: 12 and SEQ ID NO: 13, respectively.

In some embodiments, in the pharmaceutical composition described above, the anti-PD-1 antibody comprises a heavy chain variable region and a light chain variable region, wherein the heavy chain variable region comprises an HCDR1, an HCDR2 and an HCDR3 having sequences set forth in SEQ ID NO: 21, SEQ ID NO: 22 and SEQ ID NO: 23, respectively, and the light chain variable region comprises an LCDR1, an LCDR2 and an LCDR3 having sequences set forth in SEQ ID NO: 24, SEQ ID NO: 25 and SEQ ID NO: 26, respectively.

In some embodiments, in the pharmaceutical composition described above of the present disclosure, the anti-PD-1 antibody binds to human PD-1 with a dissociation equilibrium constant (KD value) equal to or less than $10^{-7}$ M, and in some embodiments, with a dissociation equilibrium constant equal to or less than $10^{-8}$ M, $10^{-9}$ M, $10^{-10}$ M, or $10^{-11}$ M. The KD value is determined by surface plasmon resonance (Biacore); for example, assayed by the method described in Test Example 1 of the present disclosure.

In some embodiments, in the pharmaceutical composition described above, the buffer has a pH of about 4.5 to about 6.0, preferably 4.5 to 6.0; in some embodiments, the buffer has a pH of about 4.7 to about 5.7, preferably 4.7 to 5.7; in some other embodiments, the buffer has a pH of about 5.2, preferably 5.2; in some other embodiments, the buffer is an acetate buffer having a pH of about 4.7 to about 5.7; in some other embodiments, the buffer is an acetate buffer having a pH of 5.2. In some other embodiments, non-limiting examples of the pH of the buffer include about 4.5, about 4.6, about 4.7, about 4.8, about 4.9, about 5.0, about 5.1, about 5.2, about 5.3, about 5.4, about 5.5, about 5.6, about 5.7, about 5.8, about 5.9, about 6.0, and any range therebetween. In some embodiments, the pH of the pharmaceutical composition described above is consistent or nearly consistent with the pH of its buffer (it is well known to those skilled in the art that there may be pH drift in some cases (there is some difference between the pH of the formulation and the pH of the buffer, referred to as a pH drift value) in the process of preparing the pharmaceutical formulation, and the pH drift value is usually within ± 0.3, and if not specifically stated, the pH drift value of the pharmaceutical composition of the present disclosure is within ± 0.3, preferably within ± 0.2, and more preferably within ± 0.1).

In some embodiments, in the pharmaceutical composition described above, the buffer has a concentration of about 5 mM to about 30 mM, preferably 5 mM to 30 mM; in some embodiments, the buffer has a concentration of about 5 mM to about 15 mM, preferably 5 mM to 15 mM; in some embodiments, the buffer has a concentration of about 10 mM, preferably 10 mM. Non-limiting examples of the concentration of the buffer include about 5 mM, about 7 mM, about 8 mM, about 9 mM, about 10 mM, about 11 mM, about 12 mM, about 13 mM, about 14 mM, about 15 mM, about 16 mM, about 18 mM, about 20 mM, about 25 mM, about 30 mM, and any range therebetween. In some embodiments, in the pharmaceutical composition described above, the anti-PD-1 antibody has a concentration of about 1 mg/mL to about 150 mg/mL, preferably 1 mg/mL to 150 mg/mL; in some embodiments, the anti-PD-1 antibody has a concentration of about 90 mg/mL to about 150 mg/mL, preferably 90 mg/mL to 150 mg/mL; in some embodiments, the anti-PD-1 antibody has a concentration of about 100 mg/mL to about 120 mg/mL. In some embodiments, the anti-PD-1 antibody has a concentration of about 100 mg/mL. In some embodiments, the anti-PD-1 antibody has a concentration of 100 mg/mL. Non-limiting examples of the concentration of the anti-PD-1 antibody include about 1 mg/mL, about 10 mg/mL, about 20 mg/mL, about 30 mg/mL, about 40 mg/mL, about 50 mg/mL, about 60 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, and any range therebetween.

In some embodiments, the pharmaceutical composition described above further comprises an osmotic pressure regulator. In some embodiments, the osmotic pressure regulator is a sugar (including monosaccharide, disaccharide, trisaccharide, polysaccharide, sugar alcohol, reducing sugar, non-reducing sugar, and the like), an amino acid (including arginine, glycine, cysteine, histidine, and the like) or a salt (sodium chloride, potassium chloride, calcium chloride, and the like). In some embodiments, the sugar is selected from the group consisting of glucose, sucrose, trehalose, lactose, fructose, maltose, dextran, glycerin, erythritol, glycerol, arabitol, sylitol, sorbitol (also known as sorbol), mannitol, melibiose, melezitose, raffinose, mannotriose, stachyose, maltose, lactulose, maltulose, glucitol, maltitol, lactitol, and iso-maltulose. In some embodiments, the sugar is a non-reducing disaccharide; in some embodiments, the sugar is preferably trehalose or sucrose, and most preferably sucrose. In some embodiments, the osmotic pressure regulator is selected from one or more of the group consisting of sucrose, trehalose, sorbitol, arginine, glycine and sodium chloride. In some embodiments, the osmotic pressure regulator preferably has a concentration of about 50 mg/mL to about 100 mg/mL, more preferably about 70 mg/mL to about 90 mg/mL; and most preferably about 80 mg/mL. In some embodiments, non-limiting examples of the concentration of the osmotic pressure regulator include about 50 mg/mL, about 60 mg/mL, about 65 mg/mL, about 70 mg/mL, about 75 mg/mL, about 80 mg/mL, about 85 mg/mL, about 90 mg/mL, about 95 mg/mL, about 100 mg/mL, and any range therebetween. In some embodiments, the pharmaceutical composition described above is an isotonic formulation. In some embodiments, the osmotic pressure regulator controls the pharmaceutical composition described above to have an osmotic pressure of 280-320 mOsm, preferably about 300 mOsm. In some embodiments, the osmotic pressure regulator is preferably sucrose at about 70 mg/mL to about 90 mg/mL, and most preferably sucrose at about 80 mg/mL. In some embodiments, the pharmaceutical composition described above further comprises a surfactant, wherein the surfactant may be selected from the group consisting of polysorbate 20 (also known as tween 20), polysorbate 80 (also known as tween 80), poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramidopropyl-betaine, cocaramidopropyl-betaine, linoleinamidopropyl-betaine, myristylamidopropyl-betaine, palmitamidopropyl-betaine, isostearamidopropyl-betaine, myristylamidopropyl-dimethylamine, palmitamidopropyl-dimethylamine, isostearamidopropyl-dimethylamine, sodium methyl cocoyl, sodium methyl oleyl taurate, polyethylene glycol, polypropylene glycol, copolymer of ethylene and propylene glycol, and the like. The preferred surfactant is polysorbate 80 or polysorbate 20, and the more preferred one is polysorbate 80.

In some embodiments, in the pharmaceutical composition described above, the surfactant has a concentration of about 0.1 mg/mL to about 1.0 mg/mL, preferably about 0.2 mg/mL to about 0.8 mg/mL; in some embodiments, the surfactant has a concentration of about 0.4 mg/mL to about 0.8 mg/mL, preferably about 0.6 mg/mL to about 0.8 mg/mL; in some embodiments, the surfactant has a concentration of about 0.6 mg/mL, preferably 0.6 mg/mL. Non-limiting examples include about 0.1 mg/mL, about 0.2 mg/mL, about 0.3 mg/mL, about 0.4 mg/mL, about 0.45 mg/mL, about 0.5 mg/mL, about 0.55 mg/mL, about 0.6 mg/mL, about 0.7 mg/mL, about 0.8 mg/mL, about 0.9 mg/mL, about 1.0 mg/mL, and any range therebetween.

In some embodiments, the anti-PD-1 antibody in the pharmaceutical composition described above is a murine antibody, a chimeric antibody, a fully human antibody, or a humanized antibody.

In some embodiments, the anti-PD-1 antibody in the pharmaceutical composition described above is a humanized antibody. In some embodiments, the humanized antibody comprises a framework region derived from a human antibody or a framework region variant thereof. In some embodiments, the framework region variant is a back mutation having up to 11 amino acids on the basis of the light chain framework region and/or the heavy chain framework region of a human antibody. In some embodiments, the framework region variant comprises a mutation selected from any one of the following (f) to (h) as compared to a framework region derived from a human antibody:

(f) a 2G amino acid back mutation comprised in the light chain variable region, and/or one or more amino acid back mutations selected from the group consisting of 27Y, 48I, 67T, 69L, 82F and 93T comprised in the heavy chain variable region;
(g) a 2V amino acid back mutation comprised in the light chain variable region, and/or one or more amino acid back mutations selected from the group consisting of 26D, 27F, 30T, 38K, 43H, 48I, 66K, 67A, 69L, 82F and 93T comprised in the heavy chain variable region; and
(h) one or more amino acid back mutations selected from the group consisting of 42G, 44V and 71Y comprised in the light chain variable region, and/or 1K and/or 94S amino acid back mutation comprised in the heavy chain variable region.

In some embodiments, the anti-PD-1 antibody in the pharmaceutical composition described above comprises antibody variable regions selected from the group consisting of the following:

(a2) a heavy chain variable region, comprising an HCDR1, an HCDR2 and an HCDR3 set forth in SEQ ID NO: 8, SEQ ID NO: 9 and SEQ ID NO: 10, respectively, and a heavy chain framework region comprising one or more amino acid back mutations selected from the group consisting of 27Y, 48I, 67T, 69L, 82F and 93T, and
a light chain variable region, comprising an LCDR2 and an LCDR3 set forth in SEQ ID NO: 12 and SEQ ID NO: 13, an LCDR1 set forth in SEQ ID NO: 11, 47, 48, 49, 50, 51 or 52, and a light chain framework region comprising a 2G amino acid back mutation;
(b2) a heavy chain variable region, comprising an HCDR1, an HCDR2 and an HCDR3 set forth in SEQ ID NO: 14, SEQ ID NO: 15 and SEQ ID NO: 16, respectively, and a heavy chain framework region comprising one or more amino acid back mutations selected from the group consisting of 26D, 27F, 30T, 38K, 43H, 48I, 66K, 67A, 69L, 82F and 93T; and
a light chain variable region, comprising an LCDR1, an LCDR2 and an LCDR3 set forth in SEQ ID NO: 17, SEQ ID NO: 12 and SEQ ID NO: 18, respectively, and a light chain framework region comprising a 2V amino acid back mutation;
(c2) a heavy chain variable region, comprising an HCDR1, an HCDR2 and an HCDR3 set forth in SEQ ID NO: 21, SEQ ID NO: 22 and SEQ ID NO: 23, respectively, and a heavy chain framework region comprising a 1K and/or 94S amino acid back mutation, and
a light chain variable region, comprising an LCDR1, an LCDR2 and an LCDR3 set forth in SEQ ID NO: 24, SEQ ID NO: 25 and SEQ ID NO: 26, respectively, and a light chain framework region comprising one or more amino acid back mutations selected from the group consisting of 42G, 44V and 71Y

In some embodiments, the anti-PD-1 antibody in the pharmaceutical composition described above comprises antibody variable regions selected from any one of the following (i) to (o);

(i) a heavy chain variable region, having a sequence set forth in SEQ ID NO: 4 or a sequence having at least 90% sequence identity to SEQ ID NO: 4, and/or

a light chain variable region, having a sequence set forth in SEQ ID NO: 5 or a sequence having at least 90% sequence identity to SEQ ID NO: 5;

(j) a heavy chain variable region, having a sequence set forth in SEQ ID NO: 6 or a sequence having at least 90% sequence identity to SEQ ID NO: 6, and/or a light chain variable region, having a sequence set forth in SEQ ID NO: 7 or a sequence having at least 90% sequence identity to SEQ ID NO: 7;

(k) a heavy chain variable region, having a sequence set forth in SEQ ID NO: 19 or a sequence having at least 90% sequence identity to SEQ ID NO: 19, and/or a light chain variable region, having a sequence set forth in SEQ ID NO: 20 or a sequence having at least 90% sequence identity to SEQ ID NO: 20;

(1) a heavy chain variable region, having a sequence set forth in SEQ ID NO: 27, 30, 31 or 32 or a sequence having at least 90% sequence identity to SEQ ID NO: 27, 30, 31 or 32, and/or

a light chain variable region, having a sequence set forth in SEQ ID NO: 28, 29, 53, 54, 55, 56, 57, 58, 59, 60, 61, 62, 63 or 64 or a sequence having at least 90% sequence identity to SEQ ID NO: 28, 29, 34, 35, 53, 54, 55, 56, 57, 58, 59, 60, 61, 62, 63 or 64;

(m) a heavy chain variable region, having a sequence set forth in SEQ ID NO: 33, 36, 37, 38, 39 or 40 or a sequence having at least 90% sequence identity to SEQ ID NO: 33, 36, 37, 38, 39 or 40, and/or

a light chain variable region, having a sequence set forth in SEQ ID NO: 34, 35, 28, 29, 53, 54, 55, 56, 57, 58, 59, 60, 61, 62, 63 or 64 or a sequence having at least 90% sequence identity to SEQ ID NO: 34, 35, 28, 29, 53, 54, 55, 56, 57, 58, 59, 60, 61, 62, 63 or 64;

(n) a heavy chain variable region, having a sequence set forth in SEQ ID NO: 41, 45 or 46 or a sequence having at least 90% sequence identity to SEQ ID NO: 41, 45 or 46, and/or

a light chain variable region, having a sequence set forth in SEQ ID NO: 42, 43 or 44 or a sequence having at least 90% sequence identity to SEQ ID NO: 42, 43 or 44;

(o) a heavy chain variable region, having a sequence set forth in SEQ ID NO: 70 or a sequence having at 90% sequence identity to SEQ ID NO: 70, and/or a light chain variable region, having a sequence set forth in SEQ ID NO: 71 or a sequence having at least 90% sequence identity to SEQ ID NO: 71; and (p) a heavy chain variable region, having a sequence set forth in SEQ ID NO: 27, 30, 31 or 32 or a sequence having at least 90% sequence identity to SEQ ID NO: 27, 30, 31 or 32, and/or

a light chain variable region, having a sequence set forth in SEQ ID NO: 34 or 35 or a sequence having at least 90% sequence identity to SEQ ID NO: 34 or 35;

wherein the sequences SEQ ID NO: 70 and SEQ ID NO: 71 are general formulas, and the specific sequences are shown in Table 2.

Table 2. Antibody variable region sequences

| | |
|---|---|
| EVQLVQSGAEVKKPGSSVKVSCKASX$_{18}$X$_{19}$TFX$_{20}$D YEX$_1$HWVX$_{21}$QAPGX$_{22}$GLEWX$_{23}$GLX$_2$DPETGGX$_3$V YNQKFKX$_4$X$_{24}$X$_{25}$TX$_{26}$TADKSTSTAYMEX$_{27}$SSLRSE DTAVYYCX$_{28}$REX$_5$X$_6$X$_7$X$_8$YX$_9$X$_{10}$X$_{11}$X$_{12}$DWYFDVW GQGTTVTVSS, wherein X$_1$ is selected from the group | DX$_{29}$VMTQTPLSLPVTPGEPAS ISCRSSQSX$_{13}$VHSX$_{14}$X$_{15}$X$_{16}$TY LEWYLQKPGQSPQLLIYKVS NRFSGVPDRFSGSGSGTDFTL KISRVEAEDVGVYYCFQGSH |
| consisting of I and M, X$_2$ is selected from the group consisting of F and I, X$_3$ is selected from the group consisting of I and T, X$_4$ is selected from the group consisting of G and D, X$_5$ is selected from the group consisting of G and R, X$_6$ is F or vacancy, X$_7$ is S or vacancy, X$_8$ is Y or vacancy, X$_9$ is G or vacancy, X$_{10}$ is S or vacancy, X$_{11}$ is selected from the group consisting of N and T, X$_{12}$ is selected from the group consisting of R and S, X$_{18}$ is selected from the group consisting of G and D, X$_{19}$ is selected from the group consisting of G, F and Y, X$_{20}$ is selected from the group consisting of S and T, X$_{21}$ is selected from the group consisting of R and K, X$_{22}$ is selected from the group consisting of Q and H, X$_{23}$ is selected from the group consisting of M and I, X$_{24}$ is selected from the group consisting of R and K, X$_{25}$ is selected from the group consisting of V, A and T, X$_{26}$ is selected from the group consisting of R and K, X$_{27}$ is selected from the group consisting of L and F, and X$_{28}$ is selected from the group consisting of A and T (SEQ ID NO: 70) | VPYX$_{17}$FGGGTKVEIK, wherein X$_{13}$ is selected from the group consisting of I and L, X$_{14}$ is selected from the group consisting of N, Q, L, T and D, X$_{15}$ is selected from the group consisting of G, A and V, X$_{16}$ is selected from the group consisting of N and K, X$_{17}$ is selected from the group consisting of A and T, and X$_{29}$ is selected from the group consisting of I, V and G (SEQ ID NO: 71) |

In some embodiments, in the pharmaceutical composition described above, the anti-PD-1 antibody comprises a heavy chain variable region set forth in SEQ ID NO: 27 or having at least 90% sequence identity to SEQ ID NO: 27, and a light chain variable region having a sequence set forth in SEQ ID NO: 55 or a sequence having at least 90% sequence identity to SEQ ID NO: 55.

In some embodiments, in the pharmaceutical composition described above, the anti-PD-1 antibody comprises a heavy chain variable region set forth in SEQ ID NO: 46 or having at least 90% sequence identity to SEQ ID NO: 46, and a light chain variable region having a sequence set forth in SEQ ID NO: 43 or a sequence having at least 90% sequence identity to SEQ ID NO: 43.

The "at least 90% identity" described above encompasses at least 90%, 91%, 92%, 93%, 94%, 95%, 96%, 97%, 98%, 99%, or 100% identity.

In some other embodiments, in the pharmaceutical composition described above, the anti-PD-1 antibody further comprises an antibody heavy chain constant region and/or an antibody light chain constant region; in some other embodiments, the antibody heavy chain constant region is selected from the group consisting of constant regions of human IgG1, IgG2, IgG3 and IgG4, and conventional variants thereof, and the antibody light chain constant region is selected from the group consisting of constant regions of human antibody κ and λ chains, and conventional variants thereof; in some other embodiments, the antibody heavy chain constant region comprises a heavy chain constant region of IgG4 incorporating one or more mutations of S228P, F234A and L235A, e.g., three amino acid mutations of S228P, F234A and L235A; in some other embodiments, the antibody comprises a heavy chain constant region having a sequence set forth in SEQ ID NO: 72 or SEQ ID NO: 79 and a light chain constant region having a sequence set forth in SEQ ID NO: 73.

In some embodiments, in the pharmaceutical composition described above, the anti-PD-1 antibody comprises a light chain set forth in SEQ ID NO: 78 or having at least 85%, 86%, 87%, 88%, 89%, 90%, 91%, 92%, 93%, 94%, 95%, 96%, 97%, 98%, 99% or 100% sequence identity to SEQ ID NO: 78, and a heavy chain set forth in SEQ ID NO: 77 or 82 or having at least 85%, 86%, 87%, 88%, 89%, 90%, 91%, 92%, 93%, 94%, 95%, 96%, 97%, 98%, 99% or 100% sequence identity to SEQ ID NO: 77 or 82; or a light chain set forth in SEQ ID NO: 75 or having at least 85%, 86%, 87%, 88%, 89%, 90%, 91%, 92%, 93%, 94%, 95%, 96%, 97%, 98%, 99% or 100% sequence identity to SEQ ID NO: 75, and a heavy chain set forth in SEQ ID NO: 74, 76, 80 or 81 or having at least 85%, 86%, 87%, 88%, 89%, 90%, 91%, 92%, 93%, 94%, 95%, 96%, 97%, 98%, 99% or 100% sequence identity to SEQ ID NO: 74, 76, 80 or 81.

In some embodiments, in the pharmaceutical composition described above, the anti-PD-1 antibody comprises: a heavy chain set forth in SEQ ID: 74 and a light chain set forth in SEQ ID: 75; or a heavy chain set forth in SEQ ID NO: 77 and a light chain set forth in SEQ ID NO: 78.

In some embodiments, the pharmaceutical composition described above comprises:

a) an anti-PD-1 antibody having a concentration of about 1 mg/mL to about 150 mg/mL,
b) an acetate buffer having a concentration of about 5 mM to about 30 mM and a pH of about 4.5 to about 6.0,
c) an osmotic pressure regulator having a concentration of about 50 mg/mL to about 100 mg/mL, wherein the osmotic pressure regulator is selected from the group consisting of sucrose, trehalose, sorbitol, arginine, glycine and sodium chloride; and
d) polysorbate having a concentration of about 0.2 mg/mL to about 0.8 mg/mL.

In some embodiments, the pharmaceutical composition described above comprises:

A1) an anti-PD-1 antibody having a concentration of about 90 mg/mL to about 150 mg/mL,
B1) an acetate buffer having a concentration of about 10 mM to about 30 mM and a pH of about 4.7 to about 5.7,
C1) sucrose having a concentration of about 70 mg/mL to about 90 mg/mL, and D1) polysorbate 80 having a concentration of about 0.6 mg/mL to about 0.8 mg/mL.

In some embodiments, the pharmaceutical composition described above comprises: an acetic acid-sodium acetate buffer having a concentration of about 10 mM and a pH of about 5.2, an anti-PD-1 antibody having a concentration of about 120 mg/mL, sucrose having a concentration of about 80 mg/mL, and polysorbate 80 having a concentration of about 0.6 mg/mL.

In some embodiments, the pharmaceutical composition described above comprises: an acetic acid-sodium acetate buffer having a concentration of about 10 mM and a pH of about 5.0, an anti-PD-1 antibody having a concentration of about 100 mg/mL, sucrose having a concentration of about 80 mg/mL, and polysorbate 80 having a concentration of about 0.6 mg/mL.

In some embodiments, the pharmaceutical composition described above comprises: an acetic acid-sodium acetate buffer having a concentration of about 10 mM and a pH of about 5.2, an anti-PD-1 antibody having a concentration of about 100 mg/mL, sucrose having a concentration of about 80 mg/mL, and polysorbate 80 having a concentration of about 0.6 mg/mL.

In some embodiments, the pharmaceutical composition described above comprises: an acetic acid-sodium acetate buffer having a concentration of about 10 mM and a pH of about 5.5, an anti-PD-1 antibody having a concentration of about 100 mg/mL, sucrose having a concentration of about 80 mg/mL, and polysorbate 80 having a concentration of about 0.6 mg/mL.

In some embodiments, the pharmaceutical composition described above comprises: an acetic acid-sodium acetate buffer having a concentration of about 10 mM and a pH of about 5.7, an anti-PD-1 antibody having a concentration of about 100 mg/mL, sucrose having a concentration of about 80 mg/mL, and polysorbate 80 having a concentration of about 0.6 mg/mL.

In some embodiments, the pharmaceutical composition described above comprises: a histidine-acetate buffer having a concentration of about 10 mM and a pH of about 5.2, an anti-PD-1 antibody having a concentration of about 100 mg/mL, sucrose having a concentration of about 80 mg/mL, and polysorbate 80 having a concentration of about 0.6 mg/mL.

In some embodiments, the pharmaceutical composition described above comprises: an acetic acid-sodium acetate buffer having a concentration of about 30 mM and a pH of about 5.2, an anti-PD-1 antibody having a concentration of about 120 mg/mL, sucrose having a concentration of about 80 mg/mL, and polysorbate 80 having a concentration of about 0.6 mg/mL.

In some embodiments, the pharmaceutical composition described above comprises: an acetic acid-sodium acetate buffer having a concentration of about 10 mM and a pH of about 5.2, an anti-PD-1 antibody having a concentration of about 100 mg/mL, sucrose having a concentration of about 80 mg/mL, and polysorbate 80 having a concentration of about 0.2 mg/mL.

In some embodiments, the pharmaceutical composition described above comprises: an acetic acid-sodium acetate buffer having a concentration of about 10 mM and a pH of about 5.2, an anti-PD-1 antibody having a concentration of about 100 mg/mL, sucrose having a concentration of about 80 mg/mL, and polysorbate 80 having a concentration of about 0.4 mg/mL.

In some embodiments, the pharmaceutical composition described above comprises: an acetic acid-sodium acetate buffer having a concentration of about 10 mM and a pH of about 5.2, an anti-PD-1 antibody having a concentration of about 100 mg/mL, sucrose having a concentration of about 80 mg/mL, and polysorbate 80 having a concentration of about 0.8 mg/mL.

In some embodiments, the pharmaceutical composition described above comprises: an acetic acid-sodium acetate buffer having a concentration of about 10 mM and a pH of about 5.2, an anti-PD-1 antibody having a concentration of about 100 mg/mL, sucrose having a concentration of about 80 mg/mL, and polysorbate 20 having a concentration of about 0.6 mg/mL.

In some embodiments, the pharmaceutical composition described above comprises: an acetic acid-sodium acetate buffer having a concentration of about 10 mM and a pH of about 5.2, an anti-PD-1 antibody having a concentration of about 100 mg/mL, trehalose having a concentration of about 80 mg/mL, and polysorbate 80 having a concentration of about 0.6 mg/mL.

In some embodiments, the pharmaceutical composition described above comprises: an acetic acid-sodium acetate buffer having a concentration of about 10 mM and a pH of about 5.2, an anti-PD-1 antibody having a concentration of about 100 mg/mL, and polysorbate 80 having a concentration of about 0.6 mg/mL.

In some embodiments, the pharmaceutical composition described above comprises: an acetic acid-sodium acetate buffer having a concentration of about 10 mM and a pH of about 5.2, an anti-PD-1 antibody having a concentration of about 100mg/mL, sorbitol having a concentration of about 50mg/mL, and polysorbate 80 having a concentration of about 0.6 mg/mL.

In some embodiments, the pharmaceutical composition described above comprises: an acetic acid-sodium acetate buffer having a concentration of about 10 mM and a pH of about 5.2, an anti-PD-1 antibody having a concentration of about 100 mg/mL, arginine having a concentration of about 100 mM, and polysorbate 80 having a concentration of about 0.6 mg/mL.

In some embodiments, the pharmaceutical composition described above comprises: an acetic acid-sodium acetate buffer having a concentration of about 10 mM and a pH of about 5.2, an anti-PD-1 antibody having a concentration of about 100 mg/mL, glycine having a concentration of about 100 mM, and polysorbate 80 having a concentration of about 0.6 mg/mL.

In some embodiments, the pharmaceutical composition described above comprises: an acetic acid-sodium acetate buffer having a concentration of about 10 mM and a pH of about 5.2, an anti-PD-1 antibody having a concentration of about 100 mg/mL, NaCl having a concentration of about 100 mM, and polysorbate 80 having a concentration of about 0.6 mg/mL.

In some embodiments, the pharmaceutical composition described above comprises: an acetic acid-sodium acetate buffer having a concentration of about 20 mM and a pH of about 5.7, an anti-PD-1 antibody having a concentration of about 150 mg/mL, sucrose having a concentration of about 80 mg/mL, and polysorbate 80 having a concentration of about 0.6 mg/mL.

In some embodiments, the pharmaceutical composition described above comprises: an acetic acid-sodium acetate buffer having a concentration of about 21.9 mM and a pH of about 4.7, an anti-PD-1 antibody having a concentration of about 120 mg/mL, sucrose having a concentration of about 80 mg/mL, and polysorbate 80 having a concentration of about 0.6 mg/mL.

In some embodiments, the pharmaceutical composition described above comprises: an acetic acid-sodium acetate buffer having a concentration of about 10 mM and a pH of about 4.7, an anti-PD-1 antibody having a concentration of about 90 mg/mL, sucrose having a concentration of about 80 mg/mL, and polysorbate 80 having a concentration of about 0.6 mg/mL.

In some embodiments, the pharmaceutical composition described above comprises: an acetic acid-sodium acetate buffer having a concentration of about 20 mM and a pH of about 5.2, an anti-PD-1 antibody having a concentration of about 120 mg/mL, sucrose having a concentration of about 80 mg/mL, and polysorbate 80 having a concentration of about 0.6 mg/mL.

In some embodiments, the pharmaceutical composition described above comprises: an acetic acid-sodium acetate buffer having a concentration of about 20 mM and a pH of about 5.2, an anti-PD-1 antibody having a concentration of about 90 mg/mL, sucrose having a concentration of about 80 mg/mL, and polysorbate 80 having a concentration of about 0.6 mg/mL.

In some embodiments, the pharmaceutical composition described above comprises: an acetic acid-sodium acetate buffer having a concentration of about 10 mM and a pH of about 5.7, an anti-PD-1 antibody having a concentration of about 150 mg/mL, sucrose having a concentration of about 80 mg/mL, and polysorbate 80 having a concentration of about 0.6 mg/mL.

In some embodiments, the pharmaceutical composition described above comprises: an acetic acid-sodium acetate buffer having a concentration of about 30 mM and a pH of about 4.7, an anti-PD-1 antibody having a concentration of about 90 mg/mL, sucrose having a concentration of about 80 mg/mL, and polysorbate 80 having a concentration of about 0.6 mg/mL.

In some embodiments, the pharmaceutical composition described above comprises: an acetic acid-sodium acetate buffer having a concentration of about 10 mM and a pH of about 4.7, an anti-PD-1 antibody having a concentration of about 150 mg/mL, sucrose having a concentration of about 80 mg/mL, and polysorbate 80 having a concentration of about 0.6 mg/mL.

In some embodiments, the pharmaceutical composition described above comprises: an acetic acid-sodium acetate buffer having a concentration of about 30 mM and a pH of about 5.2, an anti-PD-1 antibody having a concentration of about 150 mg/mL, sucrose having a concentration of about 80 mg/mL, and polysorbate 80 having a concentration of about 0.6 mg/mL.

In some embodiments, the pharmaceutical composition described above comprises: an acetic acid-sodium acetate buffer having a concentration of about 10 mM and a pH of about 5.7, an anti-PD-1 antibody having a concentration of about 90 mg/mL, sucrose having a concentration of about 80 mg/mL, and polysorbate 80 having a concentration of about 0.6 mg/mL.

In some embodiments, the pharmaceutical composition described above comprises: an acetic acid-sodium acetate buffer having a concentration of about 30 mM and a pH of about 5.7, an anti-PD-1 antibody having a concentration of about 107.7 mg/mL, sucrose having a concentration of about 80 mg/mL, and polysorbate 80 having a concentration of about 0.6 mg/mL.

In some embodiments, the pharmaceutical composition described above comprises: an acetic acid-sodium acetate buffer having a concentration of 10 mM and a pH of 5.2, an anti-PD-1 antibody having a concentration of 120 mg/mL, sucrose having a concentration of 70 mg/mL, and polysorbate 80 having a concentration of 0.6 mg/mL. In some embodiments, the pharmaceutical composition described above comprises: an acetic acid-sodium acetate buffer having a concentration of 10 mM and a pH of 5.2, an anti-PD-1 antibody having a concentration of 120 mg/mL, sucrose having a concentration of 90 mg/mL, and polysorbate 80 having a concentration of 0.6 mg/mL. In some embodiments, the pharmaceutical composition described above comprises: an acetic acid-sodium acetate buffer having a concentration of 10 mM and a pH of 5.2, an anti-PD-1 antibody having a concentration of 120 mg/mL, sucrose having a concentration of 80 mg/mL, and polysorbate 80 having a concentration of 0.6 mg/mL; wherein the anti-PD-1 antibody has a heavy chain set forth in SEQ ID NO: 74 and a light chain set forth in SEQ ID NO: 75. In some embodiments, the present disclosure provides a method of preparing the pharmaceutical composition described above, which comprises the step of buffer-exchanging an anti-PD-1 antibody stock solution; in some embodiments, the buffer is selected from the group consisting of an acetate buffer, a histidine buffer, and a succinate buffer.

The present disclosure also provides a lyophilized formulation, which can form the pharmaceutical composition according to any one of the embodiments described above after reconstitution.

In some embodiments, the present disclosure provides a lyophilized formulation comprising an anti-PD-1 antibody, wherein the lyophilized formulation is obtained by freeze-drying the pharmaceutical composition described above.

In an alternative embodiment, in the preparation described above, the freeze-drying comprises the steps of pre-freezing, primary drying and secondary drying in sequence.

In some embodiments, the present disclosure provides a lyophilized formulation comprising an anti-PD-1 antibody, wherein the lyophilized formulation can form the pharmaceutical composition described above after reconstitution.

In some embodiments, the present disclosure provides a lyophilized formulation, which is a lyophilized form of the pharmaceutical composition according to any one of the embodiments described above.

In some embodiments, the pharmaceutical composition or the lyophilized formulation described above is a stable formulation; in some embodiments, the pharmaceutical composition or the lyophilized formulation described above shows a decrease in the SEC monomer percentage of no more than 5% after being stored at refrigeration temperature (2-8 °C) for 6 months. In some embodiments, the pharmaceutical composition or the lyophilized formulation described above remains stable at 2-8 °C for at least 3 months, at least 6 months, at least 12 months, at least 18 months, or at least 24 months. In some embodiments, the pharmaceutical composition described above shows a decrease in the formulation SEC monomer percentage of no more than 2% (e.g., less than 2%, less than 1%, less than 0.8%, less than 0.5%, or even less) after being stored at 4 °C for 6 months; in some embodiments, the pharmaceutical composition shows a decrease in SEC of less than or equal to about 10%; preferably less than or equal to about 9%, about 8%, about 7%, about 6%, about 5%, about 4%, about 3%, about 2%, or about 1%; and more preferably 1.1%-2.7% as compared to the SEC value at D0 under a forced degradation condition (40 °C M1). In some embodiments, the pharmaceutical composition described above shows the formulation SEC monomer percentage of greater than 94% (e.g., greater than 94.5%, 95% or 98%) after being stored at 4 °C for 6 months.

In some embodiments, the present disclosure provides a reconstituted solution containing an anti-PD-1 antibody, wherein the reconstituted solution is obtained by reconstituting the lyophilized formulation described above. The solution for reconstitution includes but is not limited to water for injection, normal saline or glucose solution, preferably water for injection.

In some embodiments, the present disclosure provides a reconstituted solution, which is a reconstituted form of the lyophilized formulation according to any one of the embodiments described above.

In some embodiments, the present disclosure provides an article of manufacture, which comprises a container containing the pharmaceutical composition, the lyophilized formulation or the reconstituted solution described above. In some embodiments, the container is a tubular injection vial made of neutral borosilicate glass.

The present disclosure also provides use of the pharmaceutical composition, the lyophilized formulation, the reconstituted solution or the article of manufacture in preparing a medicament for the treatment/prevention of a disease or disorder.

The present disclosure also provides the pharmaceutical composition, the lyophilized formulation, the reconstituted solution or the article of manufacture described above, as a medicament for the treatment/prevention of a disease or disorder.

The present disclosure also provides a method for treating or preventing a disease or disorder, which comprises administering to a subject a therapeutically effective amount or a prophylactically effective amount of the pharmaceutical composition, the lyophilized formulation, the reconstituted solution or the article of manufacture described above.

In some embodiments, the disease or disorder according to any of the embodiments described above is a PD-1-related disease or disorder. In some embodiments, the disease according to any one of the embodiments described above is a tumor. In some other embodiments, the disease according to any one of the above embodiments is selected from the group consisting of head and neck squamous cell carcinoma, head and neck cancer, brain cancer, neuroglioma, glioblastoma multiforme, neuroblastoma, central nervous system carcinoma, neuroendocrine tumor, throat cancer, nasopharyngeal cancer, esophageal cancer, thyroid cancer, malignant pleural mesothelioma, lung cancer, breast cancer, liver cancer, hepatoma, hepatobiliary cancer, pancreatic cancer, stomach cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, leukemia, lymphoma, bone cancer, chondrosarcoma, myeloma, multiple myeloma, myelodysplastic syndrome, myeloproliferative tumor, squamous cell carcinoma, Ewing's sarcoma, systemic light chain amyloidosis and Merkel cell carcinoma; wherein in some embodiments, the lymphoma is selected from the group consisting of Hodgkin's lymphoma, non-Hodgkin's lymphoma, diffuse large B-cell lymphoma, follicular lymphoma, primary mediastinal large B-cell lymphoma, mantle cell lymphoma, small lymphocytic lymphoma, large B-cell lymphoma rich in T-cells/histiocytes and lymphoplasmacytic lymphoma; the lung cancer is selected from the group consisting of non-small cell lung cancer and small cell lung cancer; and the leukemia is selected from the group consisting of chronic myeloid leukemia, acute myeloid leukemia, lymphocytic leukemia, lymphoblastic leukemia, acute lymphoblastic leukemia, chronic lymphocytic leukemia and myeloid cell leukemia; in some embodiments, the disease is selected from the group consisting of PD-L1-positive melanoma, lung cancer, non-small cell lung cancer, breast cancer, stomach cancer, kidney cancer, bladder cancer, intestinal cancer and colon cancer; and in some other embodiments, the disease is selected from the group consisting of melanoma, lung cancer, non-small cell lung cancer, breast cancer, stomach cancer, kidney cancer, bladder cancer, intestinal cancer and colon cancer.

**BRIEF DESCRIPTION OF THE DRAWINGS**

FIG. 1: assay results of the blocking of the binding of PD-1 to the ligand thereof by anti-PD-1 antibodies;
FIG. 2: the effect of anti-PD-1 antibodies on IFNγ secretion by PBMC cells;
FIG. 3: efficacy of anti-PD-1 antibodies on mouse colon cancer MC38 xenograft tumor;
FIG. 4: effect of anti-PD-1 antibodies on volume of mouse colon cancer MC38 tumor; and
FIG. 5: DOE fitted plot of anti-PD-1 antibody formulations.

**DETAILED DESCRIPTION**

**Terms**

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure belongs. "Buffer" or "buffer solution" refers to a buffer that is resistant to pH changes by the action of its acid-base conjugate components. Examples of a buffer that controls the pH in an appropriate range include an acetate buffer, a succinate buffer, a gluconate buffer, a histidine buffer, an oxalate buffer, a lactate buffer, a phosphate buffer, a citrate buffer, a tartrate buffer, a fumarate buffer, a glycylglycine buffer and other organic acid buffers. "Acetate buffer" or "Acetate buffer solution" is a buffer containing acetate ions. Examples of the acetate buffer include an acetic acid-sodium acetate buffer, a histidine-acetate buffer, an acetic acid-potassium acetate buffer, an acetic acid-calcium acetate buffer, an acetic acid-magnesium acetate buffer, and the like. In some embodiments of the present disclosure, the acetate buffer is an acetic acid-sodium acetate buffer (also known as sodium acetate, abbreviated as AA).
"Histidine salt buffer" or "histidine salt buffer solution" is a buffer containing histidine ions. Examples of the histidine buffer include a histidine-hydrochloride buffer, a histidine-acetate buffer, a histidine-phosphate buffer, a histidine-sulfate buffer, and the like. In some embodiments of the present disclosure, the buffer is a histidine-acetate buffer (also known as histidine-acetate, abbreviated His-AA).
"Succinate buffer" or "succinate buffer solution" is a buffer containing succinate ions. Examples of the succinate buffer include a succinic acid-sodium succinate buffer, a succinic acid-potassium succinate buffer, a succinic acid-calcium succinate buffer, and the like. In some embodiments of the present disclosure, the succinate buffer is a succinic acid-sodium succinate buffer (also known as sodium succinate, abbreviated as SA).
"Citrate buffer" or "citrate buffer solution" is a buffer containing citrate ions. Examples of the citrate buffer include a citric acid-sodium citrate buffer, a citric acid-potassium citrate buffer, a citric acid-calcium citrate buffer, a citric acid-magnesium citrate buffer, and the like. The preferred citrate buffer is a citric acid-sodium citrate buffer (also known as sodium citrate, abbreviated as CA).
Osmotic pressure regulator refers to a substance used to regulate the osmotic pressure of a solution. Examples of the osmotic pressure regulator include, but are not limited to, a sugar (including monosaccharide, disaccharide, trisaccharide, polysaccharide, sugar alcohol, reducing sugar, non-reducing sugar, and the like), an amino acid (including arginine, glycine, cysteine, histidine, and the like), a salt (sodium chloride, potassium chloride, calcium chloride, and the like). In some embodiments, the osmotic pressure regulator is a sugar selected from the group consisting of glucose, sucrose, trehalose, lactose, fructose, maltose, dextran, glycerol, erythritol, glycerol, arabitol, xylitol, sorbitol (also known as sorbol), mannitol, melibiose, melezitose, raffinose, mannotriose, stachyose, maltose, lactulose, maltulose, sorbitol, maltitol, lactitol and iso-maltulose; in some embodiments, the sugar is a non-reducing disaccharide; in some embodiments, the sugar is preferably trehalose or sucrose, and most preferably sucrose. In some embodiments, the osmotic pressure regulator is an amino acid, preferably arginine or glycine; in some embodiments, the osmotic pressure regulator is a salt, preferably sodium chloride.
"Isotonic" means that the formulation has substantially the same osmotic pressure as human blood. Isotonicity can be determined by methods known in the art, for example, by using a vapor pressure osmometer or cryoscopic osmometer. When the administration route is subcutaneous injection, the osmotic pressure of the pharmaceutical composition is preferably controlled at 280-320 mOsm, and the osmotic pressure regulator is preferably sucrose at 70-90 mg/mL; and more preferably, the osmotic pressure of the pharmaceutical preparation is controlled at about 300 mOsm, and the osmotic pressure regulator is preferably sucrose with the content of 80 mg/mL.
The term "pharmaceutical composition" refers to a mixture containing one or more of the compounds or the physiologically/pharmaceutically acceptable salts or prodrugs thereof described herein, and other chemical components, for example, physiologically/pharmaceutically acceptable carriers and excipients. The pharmaceutical composition is intended to maintain the stability of the active ingredient of the antibody and promote the administration to an organism, so as to facilitate the absorption of the active ingredient, thereby exerting biological activity. As used herein, a "pharmaceutical composition" and a "formulation" are not mutually exclusive.

"Exchange" refers to the exchange of a solvent system that solubilizes an antibody protein. For example, a high-salt or hypertonic solvent system comprising the antibody protein is exchanged, by physical operations, using a buffer system of a stable formulation, so that the antibody protein is present in the stable formulation. The physical operations include, but are not limited to, ultrafiltration, dialysis or reconstitution following centrifugation.

The pharmaceutical composition or formulation of the present disclosure may be prepared by methods well known in the art. Exemplary, the pharmaceutical composition or formulation comprising an antibody is prepared as follows. Step 1: a certain amount of the purified antibody solution is subjected to solvent exchange (preferably ultrafiltration) with a buffer free of the antibody (e.g., 10 mM sodium acetate buffer at pH 5.2), and concentrated to about 130 mg/mL by at least 6-fold volume exchange over an ultrafiltration membrane. A certain volume of sucrose stock solution is added, and the mixture is uniformly mixed to make a final concentration of 80 mg/mL for sucrose. A certain volume of polysorbate 80 stock solution is added, and the mixture is uniformly mixed to make a final concentration of 0.6 mg/mL for polysorbate 80. A 10 mM sodium acetate buffer solution at pH5.2 is added for making up to a certain volume, to make a concentration of 120 mg/mL for the antibody (other formulations to be tested or stable formulations can be prepared by similar procedures). The product is filtered, subjected to central-control sampling and tested for sterility. The stock solution is allowed to pass through a 0.22 $\mu$m filter element, and the filtrate is collected. Step 2: After the filling volume is adjusted, the filtrate is filled into vials, which are then capped, and central-control samplings are performed at the beginning, in the middle and at the end of filling to test the difference in filling volume. Step 3: The capping machine is started to apply aluminum caps and perform capping. Step 4: Visual inspection is performed to confirm that products have no defects, such as inaccurate filling volume. Vial labels are printed and attached; carton labels are printed, cartons are obtained by folding, packing is performed, and carton labels are attached.

Unless otherwise stated, the solvent in the pharmaceutical composition described herein in solution form is water.

"Lyophilized formulation" refers to a formulation or a pharmaceutical composition obtained by freeze-drying a pharmaceutical composition or a formulation in liquid or solution form in vacuum. The lyophilized formulation can be obtained by freeze-drying the pharmaceutical composition or the formulation in liquid or solution form. The freeze-drying is performed by freezing the formulation and subsequently sublimating the water at a temperature suitable for primary drying. Under this condition, the product is at a temperature lower than the eutectic point or the decomposition temperature of the formulation. Generally, the storage temperature for the primary drying ranges from about -30 °C to 25 °C (assuming the product remains frozen during the primary drying) at a suitable pressure in the range of about 50-250 mTorr. The formulation, the size and type of the container (e.g., glass vial) containing the sample, and the liquid volume determine the time required for drying, which may range from a few hours to several days (e.g., 40-60 hours). The secondary drying may be performed at about 0-40 °C, which mainly depends on the type and size of the container and the type of the protein used. The time for the secondary drying is determined by the desired residual moisture level in the product, and it is generally at least about 5 hours. Generally, the water content of the formulation lyophilized at low pressure is less than about 5%, preferably less than about 3%. The pressure may be the same as that applied to the step of primary drying; preferably, the pressure of the secondary drying is lower than that of the primary drying. The freeze-drying conditions may vary with the formulation and vial size.

The surfactant of the present disclosure may be selected from the group consisting of polysorbate 20, polysorbate 80, poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramido propyl-betaine, cocaramide propyl-betaine, linoleinamide propyl-betaine, myristylamide propyl-betaine, palmitamide propyl-betaine, isostearamide propyl-betaine, myristylamide propyl-dimethylamine, palmitamide propyl-dimethylamine, isostearamide propyl-dimethylamine, sodium methyl cocoyl, sodium methyl oleyl taurate, polyethylene glycol, polypropylene glycol, copolymer of ethylene and propylene glycol, and the like. The preferred surfactant is polysorbate 80 or polysorbate 20, and the more preferred one is polysorbate 80.

The term "about" or "approximately" as used herein mean that a numerical value is within an acceptable error range for the particular value determined by one of ordinary skill in the art, and the numerical value depends in part on how the value is measured or determined (i.e., the limits of the measurement system). For example, "about" may mean a standard deviation within 1 or more than 1 in each practice in the art. Alternatively, "about" or "approximately" may mean a range of up to 20%, for example, $\pm$20%, $\pm$ 19%, $\pm$ 18%, $\pm$ 17%, $\pm$ 16%, $\pm$ 15%, $\pm$ 14%, $\pm$ 13%, $\pm$ 12%, $\pm$ 11%, $\pm$ 10%, $\pm$ 9%, $\pm$ 8%, $\pm$ 7%, $\pm$ 6%, $\pm$ 5%, $\pm$ 4%, $\pm$ 3%, $\pm$ 2%, $\pm$ 1% or less on the basis of a specific value after "about" or "approximately", and the range depends on the assay method, technical conditions, detection reagents and/or detection instruments commonly employed by those skilled in the art. Such a range is well known in the art. Furthermore, particularly for biological systems or processes, the term can mean up to an order of magnitude or up to 5-fold of a numerical value. Unless otherwise stated, when a specific value is provided in the present application and claims, the meaning of "about" or "approximately" should be assumed to be within an acceptable error range for that specific value.

The pharmaceutical composition described herein can achieve a stable effect: a pharmaceutical composition in which the antibody substantially retains its physical and/or chemical stability and/or biological activity after storage; preferably, the pharmaceutical composition substantially retains its physical and chemical stability as well as its biological activity after storage. The storage period is generally selected based on a predetermined shelf life of the pharmaceutical composition. There are a variety of analytical techniques currently available for determining protein stability, and the stability after storage for a selected period of time at a selected temperature can be determined. For example, a stable pharmaceutical antibody formulation is a formulation in which no significant change is observed under the following conditions: stored at refrigeration temperature (2-8 °C) for, e.g., at least 3 months, preferably 6 months, more preferably 1 year, and even more preferably up to 2 years. As an example for a stable formulation, the pharmaceutical antibody formulation is colorless, or clear to slightly opalescent, by visual analysis; the concentration, pH and osmolality of the formulation have no more than $\pm$ 10% change; generally, no more than about 10%, preferably no more than about 5% clipping is observed; generally, no more than about 10%, preferably no more than about 5% aggregation is formed, and the like. In some embodiments, the pharmaceutical composition or the lyophilized formulation described herein remains stable at 2-8 °C for at least 3 months, at least 6 months, at least 12 months, at least 18 months, or at least 24 months. In some embodiments, the relative stability of a formulation can be tested by observing the appearance, SEC, non-reduced CE-SDS, iCIEF and other indices of the formulation, under a forced degradation condition (e.g., 40 °C M1), an accelerated condition (e.g., 25 °C M6), a shaking D7 condition (e.g., 25 °C, 300 rpm, shaking for 10 days), multiple freeze-thaw cycles, and the like.

An antibody "retains its physical stability" in a pharmaceutical formulation if it shows no significant increase in aggregation, precipitation and/or denaturation upon visual inspection of color and/or clarity, or as determined by UV light scattering, size exclusion chromatography (SEC) and dynamic light scattering (DLS). Changes in protein conformation can be assessed by fluorescence spectroscopy (which determines the protein tertiary structure) and by FTIR spectroscopy (which determines the protein secondary structure).

An antibody "retains its chemical stability" in a pharmaceutical formulation if it shows no significant chemical change. Chemical stability can be assessed by detecting and quantifying chemically changed forms of the protein. Degradation processes that often change the chemical structure of proteins include hydrolysis or clipping (assessed by methods such as size exclusion chromatography and SDS-PAGE), oxidation (assessed by methods such as peptide mapping in combination with mass spectroscopy or MALDI/TOF/MS), deamidation (assessed by methods such as ion-exchange chromatography, capillary isoelectric focusing, peptide mapping, and isoaspartic acid determination), and isomerization (assessed by isoaspartic acid content determination, peptide mapping, etc.).

An antibody "retains its biological activity" in a pharmaceutical formulation if the biological activity of the antibody at a given time is within a predetermined range of the biological activity exhibited during the preparation of the pharmaceutical formulation. The biological activity of an antibody can be determined, for example, by an antigen-binding assay.

The terms "programmed death-1", "programmed cell death-1", "protein PD-1", "PD-1", "PDCD1" and "hPD-1" are used interchangeably and include variants, isoforms and species homologs of human PD-1, and analogs having at least one common epitope with PD-1. The complete PD-1 sequence can be found under GenBank accession No. U64863.

The term "programmed death ligand-1 (PD-L1)" is one of two cell surface glycoprotein ligands of PD-1 (the other being PD-L2) that downregulates T cell activation and cytokine secretion upon binding to PD-1. The term "PD-L1" as used herein includes human PD-L1(hPD-L1), variants, isoforms and interspecies homologs of hPD-L1, and 5 analogs having at least one common epitope with hPD-L1. The complete hPD-L1 sequence can be found under GenBank accession No. Q9NZQ7.

The term "cytokine" is a general term for proteins that are released by a cell population to act on other cells as intercellular mediators. Examples of such cytokines include lymphokines, monokines, chemokines, and traditional polypeptide hormones. Exemplary cytokines include: human IL-2, IFN-$\gamma$, IL-6, TNF$\alpha$, IL-17 and IL-5.

The three-letter and single-letter codes for amino acids used herein are as described in J. biol. chem, 243, p3558 (1968).

The term "antibody" described herein is used in the broadest sense herein and includes different antibody structures including, but not limited to, a monoclonal antibody, a polyclonal antibody, a murine antibody, a chimeric antibody, a humanized antibody, a multispecific antibody (e.g., a bispecific antibody) and antibody fragments, so long as they exhibit the desired antigen-binding activity and specificity. The "antibody" described herein includes a "full-length antibody" and an "antigen-binding fragment" thereof. In some embodiments of the present disclosure, the anti-PD-1 antibody is an anti-PD-1 antibody described in International Patent Application PCT/CN2020/074098, e.g., the "Hu23-11.IgG4AA" antibody. International Patent Application PCT/CN2020/074098 is incorporated herein in its entirety.

The terms "full-length antibody", "intact antibody", "complete antibody" and "whole antibody" are used interchangeably herein to refer to an antibody in its substantially intact form, as distinguished from an antigen-binding fragment defined below.

The term "antigen-binding fragment" or "functional fragment" refers to one or more fragments of an intact antibody that retain the ability to specifically bind to an antigen (e.g., PD-1). Examples of the antigen-binding fragment include (i) a Fab fragment, which is a monovalent fragment consisting of VL, VH, CL and CH1 domains; (ii) an F(ab')$_2$ fragment, which is a bivalent fragment comprising two Fab fragments connected by disulfide bridges in the hinge regions; (iii) an Fd fragment consisting of VH and CH1 domains; (iv) an Fv fragment consisting of VH and VL domains of a single arm of an antibody; and (v) a single domain or a dAb fragment (Ward et al., (1989) Nature 341: 544-546) consisting of VH domains. Furthermore, although the two domains of the Fv fragment, VL and VH, are encoded by separate genes, they can be linked by a synthetic linker by a recombination method, so that it is capable of producing a single protein chain in which the VL and VH regions pair to form a monovalent molecule (referred to as single-chain Fv (scFv); see, e.g., Bird et al. (1988) Science 242: 423-426; and Huston et al. (1988) Proc. Natl. Acad. Sci USA 85: 5879-5883). Such single-chain antibodies are also included in the term "antigen-binding fragment" of an antibody. Such antibody fragments are obtained by conventional techniques known to those skilled in the art, and screened for utility in the same manner as for intact antibodies. Antigen-binding moieties may be produced by a recombinant DNA technique or by enzymatic or chemical cleavage of intact immunoglobulins. In some embodiments, the antigen-binding fragment of the present disclosure is selected from the group consisting of a Fab, a Fab', an F(ab')$_2$, a single-chain antibody (scFv), a dimerized V region (diabody), a disulfide-stabilized V region (dsFv), and the like.

In the heavy and light chains of antibody, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (HVRs) and 4 framework regions (FRs) with relatively conservative sequences. The 3 hypervariable regions determine the specificity of the antibody and thus are also known as complementarity determining regions (CDRs). Each of the light chain variable regions (VLs) and the heavy chain variable regions (VHs) consists of 3 CDR regions and 4 FR regions arranged from the amino terminus to the carboxyl terminus in the following order: FR1, CDR1, FR2, CDR2, FR3, CDR3, and FR4.

The term "complementarity determining region", "CDR" or "hypervariable region" refers to one of the 6 hypervariable regions within variable domains of an antibody, which primarily contribute to antigen binding. Generally, there are three CDRs (HCDR1, HCDR2 and HCDR3) in each heavy chain variable region and three CDRs (LCDR1, LCDR2 and LCDR3) in each light chain variable region. Any one of a variety of well-known schemes can be used to determine the amino acid sequence boundaries of the CDRs, including the "Kabat" numbering scheme (see Kabat et al., (1991), "Sequences of Proteins of Immunological Interest", 5th edition, Public Health Service, National Institutes of Health, Bethesda, MD), the "Chothia" numbering scheme (see Al-Lazikani et al., (1997) JMB 273: 927-948), the ImMunoGenTics (IMGT) numbering scheme (Lefranc M. P., Immunologist, 7, 132-136 (1999); Lefranc, M. P., et al., Dev. Comp. Immunol., 27, 55-77 (2003)), etc. For example, for the classical format, according to the Kabat scheme, the CDR amino acid residues in the heavy chain variable domain (VH) are numbered as 31-35 (HCDR1), 50-65 (HCDR2) and 95-102 (HCDR3); the CDR amino acid residues in the light chain variable domain (VL) are numbered as 24-34 (LCDR1), 50-56 (LCDR2) and 89-97 (LCDR3). According to the Chothia scheme, the CDR amino acids in VH are numbered as 26-32 (HCDR1), 52-56 (HCDR2) and 95-102 (HCDR3); and amino acid residues in VL are numbered as 26-32 (LCDR1), 50-52 (LCDR2) and 91-96 (LCDR3). According to the CDR definitions by combining both the Kabat scheme and the Chothia scheme, the CDR is composed of amino acid residues 26-35 (HCDR1), 50-65 (HCDR2) and 95-102 (HCDR3) in the human VH and amino acid residues 24-34 (LCDR1), 50-56 (LCDR2) and 89-97 (LCDR3) in the human VL. According to the IMGT scheme, the CDR amino acid residues in VH are roughly numbered as 26-35 (CDR1), 51-57 (CDR2) and 93-102 (CDR3), and the CDR amino acid residues in VL are roughly numbered as 27-32 (CDR1), 50-52 (CDR2) and 89-97 (CDR3). According to the IMGT scheme, the CDRs of the antibody can be determined using the program IMGT/DomainGap Align. According to the AbM scheme, the CDR amino acids in VH are numbered as 26-32 (HCDR1), 50-58 (HCDR2) and 95-102 (HCDR3); and amino acid residues in VL are numbered as 24-34 (LCDR1), 50-56 (LCDR2) and 89-97 (LCDR3). Unless otherwise stated, the "Kabat" numbering scheme is applied to the sequences of both variable regions and CDRs of the antibody involved in examples of the present disclosure.

The "conventional variant" of the human antibody heavy chain constant region and the human antibody light chain constant region described herein refers to the variant of heavy chain constant region or light chain constant region derived from human that has been disclosed in the prior art and does not change the structure and function of the antibody variable region. Exemplary variants include IgG1, IgG2, IgG3 or IgG4 heavy chain constant region variants with site-directed modifications and amino acid substitutions in the heavy chain constant region. Specific substitutions are, for example, YTE mutation, L234A and/or L235A mutation, or S228P mutation, and/or mutations to obtain a knob-into-hole structure (so that the antibody heavy chain has a combination of knob-Fc and hole-Fc) known in the art. Those mutations have been confirmed to confer new properties on the antibody, but do not change the function of the antibody variable regions.

The antibody of the present disclosure includes a murine antibody, a chimeric antibody, a humanized antibody and a fully human antibody, and preferably a humanized antibody.

The term "murine antibody" herein is a monoclonal antibody against human PD-1 prepared according to the knowledge and skill in the art. The preparation is performed by injecting a test subject with the PD-1 antigen and then isolating hybridomas expressing antibodies with the desired sequences or functional properties. In a preferred embodiment of the present disclosure, the murine anti-PD-1 antibody may further comprise a light chain constant region of a murine κ or λ chain or a variant thereof, or further comprises a heavy chain constant region of murine IgG1, IgG2 or IgG3 or a variant thereof.

The term "chimeric antibody" refers to an antibody obtained by fusing a variable region of a murine antibody to a constant region of a human antibody, which can reduce an immune response induced by the murine antibody. Generally, the chimeric antibody is constructed by firstly establishing hybridoma secreting murine specific monoclonal antibody, then cloning a variable region gene from murine hybridoma cells, cloning a constant region gene of human antibody as required, connecting the murine variable region gene and the human constant region gene into a chimeric gene, inserting the chimeric gene into an expression vector, and finally expressing chimeric antibodies in a eukaryotic system or a prokaryotic system. In a preferred embodiment of the present disclosure, the light chain of the PD-1 chimeric antibody further comprises a light chain constant region of a human κ or λ chain or a variant thereof. The antibody heavy chain of the anti-PD-1 chimeric antibody further comprises a heavy chain constant region of human IgG1, IgG2, IgG3 or IgG4 or a variant thereof, preferably a heavy chain constant region of human IgG1, IgG2 or IgG4, or an IgG1, IgG2 or IgG4 variant using an amino acid mutation (e.g., an L234A and/or L235A mutation, and/or an S228P mutation).

The term "humanized antibody", also known as a CDR-grafted antibody, refers to an antibody produced by grafting murine CDR sequences into a human antibody variable region framework, i.e., a different type of human germline antibody framework sequence. Such an antibody can overcome the heterogeneous reaction induced by the chimeric antibody because of carrying a large amount of mouse protein components. Such framework sequences can be obtained from public DNA databases or published references that include germline antibody gene sequences. For example, germline DNA sequences of genes of the human heavy and light chain variable regions can be found in the "VBase" human germline sequence database (available at the Internet address www.mrccpe.com.ac.uk/vbase), as well as in Kabat, E. A. et al., 1991 Sequences of Proteins of Immunological Interest, 5th edition. To avoid the decrease in activity caused by the decrease in immunogenicity, the FR sequence in human antibody variable region can be subjected to minimum reverse mutation or back mutation to maintain activity. The humanized antibody of the present disclosure also includes humanized antibodies that were further subjected to CDR affinity maturation mutation by yeast display.

"Human antibody" (HuMAb), "humanized antibody", "fully human antibody" and "completely human antibody" herein are used interchangeably and have amino acid sequences corresponding to those of antibodies produced by humans or human cells, or derived from non-human sources using repertoires of human antibodies or other human antibody coding sequences. The definition of such a human antibody specifically excludes humanized antibodies comprising non-human antigen-binding residues. In some technical schemes, the completely human antibody can also be constructed by gene or chromosome transfection methods and phage display techniques, or by *in vitro* activated B cells, all of which are known in the art.

The term "amino acid difference" or "amino acid mutation" refers to the presence of amino acid changes or mutations in the variant protein or polypeptide compared with the original protein or polypeptide, including occurrence of 1, 2, 3 or more amino acid insertions, deletions or substitutions on the basis of the original protein or polypeptide. The term "antibody framework" or "FR" refers to a portion of a variable domain VL or VH, which serves as a framework for the antigen-binding loops (CDRs) of the variable domain. It is essentially a variable domain without CDRs.

The term "epitope" or "antigenic determinant" refers to a site on an antigen (e.g., a specific site on a PD-1 molecule) to which an immunoglobulin or an antibody specifically binds. Epitopes generally comprise at least 3, 4, 5, 6, 7, 8, 9, 10, 11, 12, 13, 14 or 15 contiguous or non-contiguous amino acids in a unique spatial conformation. See, e.g., Epitope Mapping Protocols in Methods in Molecular Biology, volume 66, G.E. Morris, Ed. (1996).

The terms "specific binding", "selective binding", "selectively bind to" and "specifically bind to" refer to the binding of an antibody to an epitope on a predetermined antigen. Generally, the antibody binds with an affinity (KD) of less than about $10^{-7}$ M, e.g., less than about $10^{-9}$ M, $10^{-10}$ M, or $10^{-11}$ M or less.

The term "KD" or "Kd" refers to the dissociation equilibrium constant for particular antibody-antigen interaction. Generally, the antibody of the present disclosure binds to PD-1 with a dissociation equilibrium constant (KD) of less than about $10^{-7}$ M, e.g., less than about $10^{-8}$ M or $10^{-9}$ M, e.g., as determined in a BIACORE instrument by the surface plasmon resonance (SPR) technique.

The term "compete", when used in a case where antigen-binding proteins (e.g., neutralizing antigen-binding proteins or neutralizing antibodies) compete for the same epitope, refers to the competition between the antigen-binding proteins, which is determined by the following assays in which a test antigen-binding protein (e.g., an antibody or an immunologically functional fragment thereof) prevents or inhibits (e.g., reduces) specific binding of a reference antigen-binding protein (e.g., a ligand or a reference antibody) to a common antigen (e.g., a PD-1 antigen or a fragment thereof). Numerous types of competitive binding assays are available for determining whether an antigen-binding protein competes with another, such as: solid phase direct or indirect radioimmunoassay (RIA), solid phase direct or indirect enzyme immunoassay (EIA), and sandwich competition assay (see, e.g., Stahli et al., 1983, Methods in Enzymology 9: 242-253);

solid phase direct biotin-avidin EIA (see, e.g., Kirkland et al., 1986, J. Immunol. 137: 3614-3619), solid phase direct labeled assay, and solid phase direct labeled sandwich assay (see, e.g., Harlow and Lane, 1988, Antibodies: A Laboratory Manual, Cold Spring Harbor Press); solid phase direct labeled RIA with I-125 label (see, e.g., Morel et al., 1988, Molec. Immunol. 25: 7-15); solid phase direct biotin-avidin EIA (see, e.g., Cheung, et al., 1990, Virology 176: 546-552) and direct labeled RIA (Moldenhauer et al., 1990, Scand. J. Immunol. 32: 77-82). Generally, the assay relates to use of a purified antigen binding to a solid surface or a cell bearing any of an unlabeled assayed antigen-binding protein and a labeled reference antigen-binding protein. Competitive inhibition is determined by measuring the amount of label bound to the solid surface or the cell in the presence of the assayed antigen-binding protein. Generally, the assayed antigen-binding protein exists in an excessive amount. Antigen-binding proteins identified by the competitive assay (competitive antigen-binding proteins) include: an antigen-binding protein binding to the same epitope as a reference antigen-binding protein, and an antigen-binding protein binding to an adjacent epitope sufficiently close to a binding epitope of the reference antigen-binding protein, and the two epitopes spatially interfere with each other to prevent the binding. Other detailed information regarding the method for assaying competitive binding is provided in the examples herein. Generally, when the competitive antigen-binding protein exists in an excessive amount, the specific binding of the reference antigen-binding protein to the common antigen will be inhibited (e.g., reduced) by at least 40%-45%, 45%-50%, 50%-55%, 55%-60%, 60%-65%, 65%-70%, 70%-75% or 75% or more. In certain instances, the binding is inhibited by at least 80%-85%, 85%-90%, 90%-95%, 95%-97% or 97% or more.

The term "nucleic acid molecule" used herein refers to a DNA molecule and an RNA molecule. The nucleic acid molecule may be single-stranded or double-stranded, and is preferably a double-stranded DNA, a single-stranded mRNA or a modified mRNA. A nucleic acid is "operably linked" when it is placed into a functional relationship with another nucleic acid sequence. For example, a promoter or enhancer is operably linked to a coding sequence if it affects the transcription of the coding sequence.

The term "vector" refers to a nucleic acid molecule capable of transporting another nucleic acid to which it has been linked. In one embodiment, the vector is a "plasmid" that refers to a circular double-stranded DNA loop into which additional DNA segments can be ligated. In another embodiment, the vector is a viral vector, wherein additional DNA segments may be ligated into the viral genome. The vectors disclosed herein are capable of autonomous replication in a host cell into which they have been introduced (e.g., bacterial vectors having a bacterial origin of replication and episomal mammalian vectors) or being integrated into the genome of a host cell upon introduction into the host cell and thereby replicated along with the host genome (e.g., non-episomal mammalian vectors).

Methods for producing and purifying antibodies and antigen-binding fragments are well known in the art, for example, those described in chapters 5-8 and 15 of "Antibodies: A Laboratory Manual", Cold Spring Harbor Press. For example, mice can be immunized with human PD-1 or a fragment thereof, and the obtained antibodies can be renatured and purified, and amino acid sequencing can be performed by conventional methods. Likewise, antigen-binding fragments can be prepared by conventional methods. The antibody or the antigen-binding fragment described herein is genetically engineered to contain one or more additional human FRs in the non-human CDRs. Human FR germline sequences can be obtained at the website http://imgt.cines.fr of ImMunoGeneTics (IMGT) or from the immunoglobulin journal, 2001ISBN012441351, by comparing the IMGT human antibody variable region germline gene database with the MOE software.

The term "host cell" refers to a cell into which an expression vector has been introduced. Host cells may include bacterial, microbial, plant or animal cells. Bacteria susceptible to transformation include members of the *Enterobacteriaceae* family, such as strains of *Escherichia coli* or *Salmonella;* members of the *Bacillaceae* family, such as *Bacillus subtilis; Pneumococcus; Streptococcus* and *Haemophilus influenzae.* Suitable microorganisms include *Saccharomyces cerevisiae* and *Pichia pastoris.* Suitable animal host cell lines include CHO (Chinese hamster ovary cell line) and NSO cells.

The engineered antibody or antigen-binding fragment of the present disclosure can be prepared and purified by conventional methods. For example, cDNA sequences encoding the heavy and light chains can be cloned and recombined into a GS expression vector. Recombinant immunoglobulin expression vectors can be stably transfected into CHO cells. As a more recommended prior art, mammalian expression systems may result in glycosylation of antibodies, particularly at the highly conserved N-terminal site of the Fc region. Stable clones are obtained by expression of the antibody that specifically binds to human PD-1. Positive clones are expanded in a serum-free medium of a bioreactor to produce antibodies. The culture with the secreted antibody can be purified by conventional techniques. For example, purification is performed using an A or G Sepharose FF column containing an adjusted buffer. Non-specifically bound fractions are washed away. The bound antibody is eluted by the pH gradient method, and the antibody fragments are detected by SDS-PAGE and collected. The antibody can be filtered and concentrated by conventional methods. Soluble mixtures and polymers can also be removed by conventional methods, such as molecular sieves and ion exchange. The resulting product needs to be immediately frozen, e.g., at -70 °C, or lyophilized.

"Administrating", "giving" and "treating", when applied to animals, humans, experimental subjects, cells, tissues, organs or biological fluid, refer to contact of an exogenous drug, a therapeutic agent, a diagnostic agent or composition with the animals, humans, subjects, cells, tissues, organs or biological fluid. "Administrating", "giving" and "treating" can refer to, for example, therapeutic, pharmacokinetic, diagnostic, research and experimental methods. The treatment of cells comprises making the reagent in contact with the cells and making the reagent in contact with fluid, where the fluid is in contact with the cells. "Administrating", "giving" and "treating" also refer to treating, e.g., cells by reagents, diagnosis, binding compositions or by another cell *in vitro* and *ex vivo.* "Treating", when applied to humans, veterinary or research subjects, refers to therapeutic treatment, preventive or prophylactic measures, and research and diagnostic applications.

"Treatment" refers to administering a therapeutic agent, such as a composition comprising any one of the conjugation compounds of the present disclosure, either internally or externally to a patient with one or more symptoms of a disease on which the therapeutic agent is known to have a therapeutic effect. Generally, the therapeutic agent is administered in an amount effective to alleviate one or more symptoms of the disease in the patient or population being treated to induce regression of such symptoms or inhibiting the development of such symptoms to any clinically measurable degree. The amount of therapeutic agent effective to alleviate the symptoms of any particular disease (also known as a "therapeutically effective amount") may vary depending on a variety of factors, such as the disease state, age, and weight of the patient, and the ability of the drug to produce a desired therapeutic effect in the patient. Whether a symptom of a disease has been alleviated can be evaluated by any clinical testing methods commonly used by doctors or other health care professionals to evaluate the severity or progression of the symptom. Although the embodiments of the present disclosure (for example, treatment methods or articles of manufacture) may not be effective in alleviating the symptoms of each disease of interest, they shall reduce the symptoms of a disease of interest in a statistically significant number of patients, as determined according to any statistical testing methods known in the art, such as Student t-test, chi-square test, Mann and Whitney's U test, Kruskal-Wallis test (H test), Jonckheere-Terpstra test and Wilcoxon test.

"Conservative modification" or "conservative replacement or substitution" refers to replacement of amino acids in a protein with other amino acids having similar characteristics (e.g., charge, side-chain size, hydrophobicity/hydrophilicity, or backbone conformation and rigidity), so that changes can be frequently made without changing the biological activity of the protein. Those skilled in the art know that, generally speaking, a single amino acid replacement in a non-essential region of a polypeptide does not substantially change the biological activity (see, e.g., Watson et al. (1987) Molecular Biology of the Gene, The Benjamin/Cummings Pub. Co., p224, (4th edition)). In addition, the replacement of amino acids with similar structure or function is unlikely to disrupt the biological activity. Exemplary conservative substitutions are stated in the Table "Exemplary amino acid conservative substitutions" below.

Table 3. Exemplary amino acid conservative substitutions

| Original residue | Conservative substitution |
|---|---|
| Ala(A) | Gly; Ser |
| Arg(R) | Lys; His |
| Asn(N) | Gln; His; Asp |
| Asp(D) | Glu; Asn |
| Cys(C) | Ser; Ala; Val |
| Gln(Q) | Asn; Glu |
| Glu(E) | Asp; Gln |
| Gly(G) | Ala |
| His(H) | Asn; Gln |
| Ile(I) | Leu; Val |
| Leu(L) | Ile; Val |
| Lys(K) | Arg; His |
| Met(M) | Leu; Ile; Tyr |
| Phe(F) | Tyr; Met; Leu |
| Pro(P) | Ala |
| Ser(S) | Thr |
| Thr(T) | Ser |
| Trp(W) | Tyr; Phe |

(continued)

| Original residue | Conservative substitution |
|---|---|
| Tyr(Y) | Trp; Phe |
| Val(V) | Ile; Leu |

"Effective amount" or "effective dose" refers to the amount of a drug, a compound or a pharmaceutical composition necessary to obtain any one or more beneficial or desired therapeutic results. For preventive use, the beneficial or desired results include elimination or reduction of risk, reduction of severity or delay of the onset of a disorder, including the biochemistry, histology and/or behavioral symptoms of the disorder, complications thereof and intermediate pathological phenotypes that appear during the progression of the disorder. For therapeutic applications, the beneficial or desired results include clinical results, such as reducing the incidence of various disorders related to the target antigen of the present disclosure or alleviating one or more symptoms of the disorder, reducing the dosage of other agents required to treat the disorder, enhancing the therapeutic effect of another agent, and/or delaying the progression of disorders of the patient related to the target antigen of the present disclosure.

"Exogenous" refers to substances produced outside organisms, cells or human bodies according to circumstances. "Endogenous" refers to substances produced inside cells, organisms or human bodies according to circumstances.

"Homology" refers to sequence similarity between two polynucleotide sequences or between two polypeptides. When positions in two compared sequences are occupied by identical bases or amino acid monomer subunits, e.g., if the position of each of two DNA molecules is occupied by adenine, the molecules are homologous at that position. The homology percentage between two sequences is a function of the number of matching or homologous positions shared by the two sequences divided by the number of positions compared × 100%. For example, in the optimal alignment of sequences, if 6 out of 10 positions of two sequences are matched or homologous, the two sequences are 60% homologous, and if 95 out of 100 positions of two sequences are matched or homologous, the two sequences are 95% homologous. Generally, two sequences, when aligned, are compared to give the maximum percent homology. For example, the comparison may be made by the BLAST algorithm, wherein the parameters of the algorithm are selected to give the maximum match between the reference sequences over the entire length of each sequence. The following references relate to the BLAST algorithm often used for sequence analysis: the BLAST algorithms: Altschul, S.F. et al., (1990) J. Mol. Biol., 215: 403-410; Gish, W., et al., (1993) Nature Genet., 3: 266-272; Madden, T.L. et al., (1996) Meth. Enzymol., 266: 131-141; Altschul, S.F. et al., (1997) Nucleic Acids Res., 25: 3389-3402; Zhang, J. et al., (1997) Genome Res., 7: 649-656. Other conventional BLAST algorithms, such as one provided by NCBI BLAST, are also well known to those skilled in the art.

As used herein, the expressions "cell", "cell line" and "cell culture" are used interchangeably, and all such designations include their progenies. Therefore, the words "transformant" and "transformed cell" include primary test cells and cultures derived therefrom, regardless of the number of transfers. It should also be understood that all progenies may not be precisely identical in DNA content due to deliberate or unintentional mutations. Mutant progeny with the same function or biological activity as screened in the original transformed cells is included. When referring to different designations, they will become clear through the context.

"Polymerase chain reaction" or "PCR" used herein refers to a procedure or technique in which a trace amount of a specific moiety of nucleic acid, RNA and/or DNA is amplified as described in, for example, US Patent No. 4,683,195. Generally speaking, it is necessary to obtain sequence information from the end or outside of the target region, so that oligonucleotide primers can be designed; these primers are identical or similar in terms of sequence to the corresponding strand of the template to be amplified. The 5'-terminal nucleotide of 2 primers may coincide with the end of the material to be amplified. PCR can be used to amplify specific RNA sequences, specific DNA sequences from total genomic DNA and cDNA sequences transcribed from total cellular RNA, phage, plasmid sequences, or the like. See generally Mullis, et al., (1987) Cold Spring Harbor Symp. Quant. Biol. 51: 263; Erlich ed. (1989) PCR TECHNOLOGY (Stockton Press, N.Y). The PCR used herein is considered to be an example, but not the only one, of a nucleic acid polymerase reaction method for amplifying a nucleic acid test sample, and the method comprises using known nucleic acids as primers and nucleic acid polymerases to amplify or produce a specific moiety of the nucleic acid.

"Isolated" refers to a purified state, and in this case means that the designated molecule is substantially free of other biomolecules, such as nucleic acids, proteins, lipids, carbohydrates, or other materials (such as cell debris and growth medium). Generally, the term "isolated" does not mean the complete absence of such substances or the absence of water, buffers or salts, unless they are present in amounts that will significantly interfere with the experimental or therapeutic use of the compounds described herein.

The term "optional" or "optionally" means that the event or circumstance subsequently described may, but not necessarily, occur, and that the description includes instances where the event or circumstance occurs or does not occur. For example, "optionally comprising 1-3 antibody heavy chain variable regions" means that the antibody heavy chain variable region of a particular sequence may, but not necessarily, be present.

In addition, the present disclosure includes an agent for treating a disease related to target antigen (e.g., PD-1)-positive cells, and the agent comprises the anti-PD-1 antibody of the present disclosure as an active ingredient.

There is no limitation for a PD-1-related disease in the present disclosure, as long as it is a disease related to PD-1. For example, the therapeutic response induced by the molecule of the present disclosure can be achieved by binding to human PD-1 and then blocking the binding of PD-1 to its ligands PD-L1 or PD-L2, or killing tumor cells over-expressing PD-1. Thus, the molecule of the present disclosure is very useful, when in preparations and formulations suitable for therapeutic applications, for those people who have tumors or cancers, preferably melanoma, colon cancer, breast cancer, lung cancer, stomach cancer, intestinal cancer, kidney cancer, non-small cell lung cancer, bladder cancer, and the like.

In addition, the present disclosure relates to methods for immunodetection or determination of the target antigen (e.g., PD-1), reagents for immunodetection or determination of the target antigen (e.g., PD-1), methods for immunodetection or determination of cells expressing the target antigen (e.g., PD-1) and diagnostic agents for diagnosing diseases related to positive cells of the target antigen (e.g., PD-1), which includes the antibody or the antibody fragment of the present disclosure as an active ingredient, which specifically recognizes the target antigen (e.g., human PD-1) and binds to the amino acid sequence of the extracellular region or a three-dimensional structure thereof.

In the present disclosure, the method for detection or determination of the amount of the target antigen (e.g., PD-1) may be any known method. For example, it includes immunodetection or determination methods.

The immunodetection or determination methods are methods for detecting or determining the amount of antibody or antigen using labeled antigens or antibodies. Examples of immunodetection or determination methods include radioimmunoassay (RIA), enzyme immunoassay (EIA or ELISA), fluorescence immunoassay (FIA), luminescence immunoassay, western blotting, physicochemical methods, etc.

The above diseases related to PD-1-positive cells can be diagnosed by detecting or determining cells expressing PD-1 with the antibody or the antibody fragment of the present disclosure.

In order to detect cells expressing the polypeptide, known immunodetection methods can be used, preferably immunoprecipitation, fluorescent cell staining, immunohistochemical staining, etc. In addition, fluorescent antibody staining method utilizing the FMAT8100HTS system (Applied Biosystem) can be used.

In the present disclosure, there is no particular limitation for the living sample used for detection or determination of the target antigen (e.g., PD-1), as long as it has the possibility of comprising cells expressing the target antigen (e.g., PD-1 ), such as tissue cells, blood, plasma, serum, pancreatic juice, urine, feces, tissue fluid or culture.

According to the required diagnostic method, the diagnostic agent containing the monoclonal antibody or the antibody fragment thereof of the present disclosure can also contain reagents for performing antigen-antibody reaction or reagents for detecting the reaction. The reagents for performing the antigen-antibody reaction include buffers, salts, etc. The reagents for detection include reagents commonly used in immunodetection or determination methods, for example labeled second antibodies that recognize the monoclonal antibody, the antibody fragment thereof or the conjugate thereof, and substrates corresponding to the label, etc.

In some embodiments, the antigen is prepared as follows:

Human PD-1-IgG1Fc fusion protein is designed and synthesized, wherein the N-terminus is 150 amino acids of the human PD-1 extracellular region, and the C-terminus is the human IgG1 Fc segment (hIgG1Fc). The fusion protein is purified using a Protein A affinity column to obtain recombinant PD-1-Fc protein with high purity for detecting the binding of the anti-PD-1 antibody to the antigen.

Human PD-1-IgG1Fc (SEQ ID NO: 1):

MEFGLSWLFLVAILKGVQCPGWFLDSPDRPWNPPTFSPALLVVTEGDNATFTCS
FSNTSESFVLNWYRMSPSNQTDKLAAFPEDRSQPGQDCRFRVTQLPNGRDFH
MSVVRARRNDSGTYLCGAISLAPKAQIKESLRAELRVTERRAEVPTAHPSPSR
PAGQFQTL*EPKSSDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFYPSD
IAVEWESNGQPENNYKTTPPVLDSDGSFFLYSKLTVDKSRWQQGNVFSCSVMHEAL
HNHYTQKSLSLSPGK.*

Note: the underlined part represents the signal peptide, the upright part refers to the human PD-1 extracellular region, and the italic part refers to hIgG1Fc (signal peptide + extracellular region + *hIgG1Fc*).

Human PD-1-his (SEQ ID NO: 2):

MEFGLSWLFLVAILKGVQCPGWFLDSPDRPWNPPTFSPALLVVTEGDNATFTCS
FSNTSESFVLNWYRMSPSNQTDKLAAFPEDRSQPGQDCRFRVTQLPNGRDFH
MSVVRARRNDSGTYLCGAISLAPKAQIKESLRAELRVTERRAEVPTAHPSPSPR
PAGQFQTLVGSSDYKDDDDKHHHHHH.

PD-1 antigen encoded by nucleic acid transfecting cells (SEQ ID NO: 3):

MQIPQAPWPVVWAVLQLGWRPGWFLDSPDRPWNPPTFSPALLVVTEGDNATF
TCSFSNTSESFVLNWYRMSPSNQTDKLAAFPEDRSQPGQDCRFRVTQLPNGRD
FHMSVVRARRNDSGTYLCGAISLAPKAQIKESLRAELRVTERRAEVPTAHPSPS
PRPAGQFQTLVVGVVGGLLGSLVLLVWVLAVICSRAARGTIGARRTGQPLKED
PSAVPVFSVDYGELDFQWREKTPEPPVPCVPEQTEYATIVFPSGMGTSSPARRGS
ADGPRSAQPLRPEDGHCSWPL.

In some embodiments, the anti-human PD-1 antibody can be produced by immunizing mice or can be obtained from an anti-human PD-1 phage mouse immune library.

The method for preparing the anti-human PD-1 antibody by immunizing mice is as follows:

1. Immunization: laboratory SJL white mice, female, 6-8 weeks of age, and Balb/c white mice, female, 6-8 weeks of age. Housing environment: SPF grade. The purchased mice are fed in a laboratory environment for 1 week, in a 12/12 hour light/dark cycle, at a temperature of 20-25 °C, with humidity at 40-60%. The mice adapted to the environment are immunized according to different schemes, with 6-10 mice in each group. The immunization antigen may be purified recombinant protein PD-1-IgG1Fc (see SEQ ID NO: 1), PD-1-his (see SEQ ID NO: 2), or Jurkat/CHO-PD-1 cell transfected with PD-1 as an antigen (see SEQ ID NO: 3), and cross-immunization can be performed with a single antigen in combination with different immunoadjuvants or different types of immunogens. The immunization site may be abdominal cavity or dorsal subcutaneous part, or the two alternative immunization sites. The immunoadjuvants TiterMax® Gold Adjuvant (hereinafter referred to as Titermax, purchased from Sigma, Cat. No. T2684) and Imject Alum Adjuvant (hereinafter referred to as Alum, purchased from Pierce, Cat. No. 77161) are used for cross-immunization. The ratio of the antigen to the adjuvant (Titermax) is 1:1, the ratio of the antigen to the adjuvant (Alum) is 3:1, and the dose is as follows: 25-50 $\mu$g/mouse (primary immunization), 50 $\mu$g/mouse (booster immunization), or $1 \times 10^7$ Jurkat/CHO-PD-1 cells/mouse. On day 0, the emulsified antigen is intraperitoneally injected at 25-50 $\mu$g/mouse, after the primary immunization, the injection is performed once a week or once every two weeks, with Titermax and Alum as alternate antigens, for a total of 5-8 times.

2. Cell fusion: mice with high antibody titers in serum are selected for spleen cell fusion, and the mice after 72 h of booster immunization ("h" is an abbreviation for "hour", the same applies hereinafter) are subjected to bloodletting by picking up eyeballs, sacrificed by cervical dislocation, and sterilized in 75% ethanol. Spleen lymphocytes and myeloma cells, Sp2/0 cells (Chinese Academy of Sciences), are fused by following an optimized PEG-mediated fusion procedure to obtain hybridoma cells. The fused hybridoma cells are resuspended in HAT complete medium (RPMI-1640 medium containing 20% FBS, 1× HAT and 1× OPI), plated in 96-well cell culture plates (1 × 10^5/150 $\mu$L/well), and incubated at 37 °C with 5% $CO_2$, wherein about 10-30 plates are used. On day 5 after the fusion, HAT complete medium is added at 50 $\mu$L/well, and the cells are incubated at 37 °C with 5% $CO_2$. On days 7-8 after the fusion, complete medium change is performed according to cell growth density at 200 $\mu$L/well, and the cells are incubated at 37 °C with 5% $CO_2$.

3. Screening of hybridoma cells: on days 7-9 after the fusion, an assay on the binding of the antibody to PD-1 by ELISA is performed according to cell growth density, and an assay on the blocking of binding of PD-1/PDL1 by ELISA is performed on the detected positive well cells. The positive well cells are subjected to medium change and timely transferred to a 24-well plate for expansion according to the cell density. After the cell strains transferred into the 24-well plate are retested, preservation and first subcloning are performed. The cell strains tested positive after the first subcloning are selected for preservation, and second or third subcloning is performed until single cell clones are obtained. The hybridoma cells that have the effect of blocking the binding of PD-1 to PDL1 are obtained after multiple fusions.

The method for obtaining the anti-human PD-1 antibody through the anti-human PD-1 phage mouse immune library is as follows:

1. Construction of an anti-human PD-1 phage mouse immune library: spleens of mice with high antibody titers in serum are selected, from which total RNA is extracted using Trizol (Invitrogen Cat No. 15596-018). Reverse transcription is performed using PrimeScript™ II 1st Strand cDNA Synthesis Kit (Takara Cat No. 6210A) to obtain cDNA. Primers for constructing the library are designed and synthesized according to the IMGT database. A single-chain antibody fragment is obtained by three rounds of PCR reactions. The single-chain antibody fragment and a modified library construction vector pCantab5E (Amersham Biosciences/GE Cat No. 27-9400-01) are digested by Sfi1 (NEB Cat No. R0123L), and the fragments are purified and extracted using E.Z.N.A.® Gel Extraction Kit (Omega Cat No. D2500-02) after electrophoresis. Then, they are ligated with T4 DNA ligase (NEB Cat No. M0202L) at 16 °C for 16-18 h, purified and extracted with the above kit, and finally eluted with deionized water. 1 μg of the ligation product is mixed with 1 tube of electrotransformation competent TG1 (Lucigen Cat No. 60502-2) and the mixture is electrotransformed with an electroporator (Bio Rad Micropulser), with parameters set as follows: 2.5 kV, 200Ω, 25 μF. After the transformation is repeated 10 times, the cells are plated and cultured in an inverted state at 37 °C for 16-18 h. All colonies are scraped off and mixed together, added with glycerol at a final concentration of 15%, and stored at -80 °C for later use.

2. Screening of anti-human PD-1 phage mouse immune library: 1 mL of a buffer containing 2% skim milk-phosphate buffered saline (abbreviated as MPBS) is added to the packaged anti-human PD-1 phage immune library ($1×10^{12}$-$1×10^{13}$) and 100 μL of streptavidin microbeads (Mi1envi Biotec, Auburn, CA), and the mixture is incubated at room temperature for 1 h and left to stand on a magnetic rack, and the supernatant is collected. The supernatant is added into 10 μg/mL biotinylated human PD-1-ECD-his protein (purchased from Sino Biological) and incubated at room temperature for 1 h, then 100 μL of streptavidin-coated magnetic beads (1 mL of MPBS pre-incubation) are added and incubated at room temperature for 1 h. The tube is loaded on a magnetic rack system for sorting, and the supernatant is removed by pipetting. 1 mL of PBST (0.1% Tween-20 in phosphate buffered saline) is added, and the tube is flipped up and down many times. The liquid is removed by pipetting, and a fresh washing solution is added, which are repeated 11 times to remove unbound antibody fragments. 0.5 mL of eluent (50 μL of 10 mg/mL trypsin stock solution in 450 μL of PBS) is added. The tube is shaken at room temperature for 15 min ("min" is an abbreviation for "minute", the same applies hereinafter) and left to stand on a magnetic rack, and the supernatant is pipetted and added into a new EP tube. TG1 is seeded into 2YT medium and expanded to a cultured bacterial density of OD600 = 0.4. 1.75 mL of TG1(OD600 = 0.4) is added to each tube, followed by the addition of 250 μL of eluted phage (phase), and the mixture is incubated for 30 min under a water bath at 37 °C, diluted in a gradient, and plated for titer determination. The remaining TG1 solution is centrifuged, plated and incubated at 37 °C overnight.

The phage mouse immune library is subjected to 2-3 rounds of MACS screening (streptomycin magnetic beads, Invitrogen) using biotinylated human PD-1-ECD-his antigen, so that a monoclonal antibody that binds to PD-1 and blocks the binding of PD-1 to PD-L1 is finally obtained, which is then sequenced and verified to obtain the variable region sequences of the antibody.

In some embodiments, the purification method for the antibody or antigen protein is as follows:

1. Separation and purification/Protein G affinity chromatography of hybridoma supernatant

For the purification of mouse hybridoma supernatant, Protein G is preferred for affinity chromatography. The cultured hybridoma is centrifuged to obtain the supernatant, and 10%-15% by volume of 1 M Tris-HCl (pH 8.0-8.5) is added based on the volume of the supernatant to adjust the pH of the supernatant. The Protein G column is washed with 3-5 column volumes of 6 M guanidine hydrochloride, then with 3-5 column volumes of pure water; the column is equilibrated with 3-5 column volumes of, for example, 1× PBS (pH 7.4) buffer system as an equilibration buffer; the cell supernatant is loaded on the column at a low flow rate for binding, with the flow rate controlled to allow for retention time of about 1 min or longer; the column is washed with 3-5 column volumes of 1× PBS (pH 7.4) until the UV absorbance falls back to baseline; the sample is eluted with a 0.1 M acetic acid/sodium acetate (pH 3.0) buffer, the elution peaks are collected by UV detection, and the eluted product is rapidly adjusted to pH 5-6 with 1 M Tris-HCl (pH 8.0) and temporarily stored. For the eluted product, solution exchange may be performed by methods well known to those skilled in the art, such as ultrafiltration concentration using an ultrafiltration tube to exchange the solution to a desired buffer system, or exchange with a desired buffer system by size exclusion (e.g., G-25 desalination), or removal of polymer components from the eluted product using a high-resolution molecular exclusion column such as Superdex 200 to improve sample purity. 2. Purification of protein or antibody by Protein A affinity chromatography Firstly, the cell culture supernatant expressing the antigen protein or antibody is centrifuged at a high speed to collect the supernatant. The Protein A affinity column is washed with 3-5 column volumes of 6 M guanidine hydrochloride, then with 3-5 column volumes of pure water. The column is equilibrated with 3-5 column volumes of, for example, 1× PBS (pH 7.4) buffer system as an equilibration buffer. The supernatant is loaded on the column at a low flow rate for binding, with the flow rate controlled to allow for retention time of about 1 min or longer. After the binding is completed, the column is washed with 3-5 column volumes of 1× PBS (pH 7.4) until the UV absorbance falls back to baseline. The sample is eluted with a 0.1 M acetic acid/sodium acetate (pH 3.0-3.5) buffer, the elution peaks are collected by UV detection, and the eluted product is rapidly adjusted to pH 5-6 with 1 M Tris-HCl (pH 8.0) and temporarily stored. For the eluted product, solution exchange may be performed by methods well known to those skilled in the art, such as ultrafiltration concentration using an ultrafiltration tube to exchange the solution to a desired buffer system, or exchange with a desired buffer system by size exclusion (e.g., G-25 desalination), or removal of polymer components from the eluted product using a high-resolution molecular exclusion column such as Superdex 200 to improve sample purity.

The present disclosure is further described with reference to the following examples, which, however, do not limit the present disclosure. The experimental methods in the examples of the present disclosure in which specific conditions are not specified are generally performed under conventional conditions such as Antibodies: A Laboratory Manual and Molecular Cloning: A Laboratory Manual by Cold Spring Harbor Laboratory, or under conditions recommended by the manufacturers of the starting materials or commercial products. Reagents without specific origins indicated are commercially available conventional reagents.

**Example 1. Acquisition of Anti-Human PD-1 Murine Antibodies**

The anti-human PD-1 murine antibodies obtained by the above method were subjected to an antigen binding assay, and screened to obtain a plurality of antibodies with good activity, including M23, M32 and M33. Single cell clones were expanded, from which RNA was extracted, and reverse transcription amplification (RT-PCR) was performed using a degenerate primer of mouse-Ig to obtain the variable region sequences of the antibody. The variable region sequences of the murine antibody were linked to the constant region sequences of the human antibody, and the chimeric antibody of the murine monoclonal antibody was cloned and recombinantly expressed. An *in vitro* activity assay was performed to confirm that the variable region sequences of the obtained monoclonal antibody were correct.

The obtained variable region sequences of murine antibodies M23, M32 and M33 are as follows:

Heavy chain variable region of murine antibody M23 (SEQ ID NO: 4):

QVQLQQSGAELVRPGASVTLSCKASGYTFT<u>DYEMH</u>WVKQTPIHGLEWIG<u>LIDP ETGGTVYNQKFKD</u>KTILTADKSSSTAYMEFRSLTSEDSAVYHCTR<u>ERFSYYGSTS DWYFDV</u>WGTGTTVTVSS.

Light chain variable region of murine antibody M23 (SEQ ID NO: 5):

DGLMTQTPLSLPVSLGDHASISC<u>RSSQSLVHSNGNTYLE</u>WYLQKPGQSPKLLIY <u>KVSNRFS</u>GVPDRFSGSGSGTDFTLKISRVEAEDLGIYYC<u>FQGSHVPYT</u>FGGGTKL EIK.

Heavy chain variable region of murine antibody M32 (SEQ ID NO: 6):

QVQLQQSGAELVRPGASVTLSCKASDFTFT<u>DYEIH</u>WVKQTPVHGLEWIG<u>LFDPE TGGIVYNQKFKG</u>KAILTADKSSNTAYMEFRSLTSEDSAVYYCTR<u>EGYNRDWYFD V</u>WGTGTTVTVSS.

Light chain variable region of murine antibody M32 (SEQ ID NO: 7):

DVLMTQTPLSLPVSLGDQASISC<u>RSSQSIVHSNGNTYLE</u>WYLQKPGQSPKLLIY<u>K</u><u>VSNRFS</u>GVPDRFSGSGSGTDFTLKISRVEAEDLGIYYC<u>FQGSHVPYA</u>FGGGTKLE IK.

Heavy chain variable region of murine antibody M33: (SEQ ID NO: 19)

KVMLVESGGGLVKPGGSLKLSCAASGFTFS<u>SYAMS</u>WVRQTPEKRLEWVA<u>TISG</u><u>GGVDTYYQDNVQG</u>RFTISRDNAKNTLYLQMSSLRSEDTALYYCAS<u>PYGHGYFD</u><u>V</u>WGTGTTVTVSS.

Light chain variable region of murine antibody M33: (SEQ ID NO: 20)

DIQMTQTTSSLSASLGDRVTISC<u>RASQDINNFLN</u>WYQQKPDGTVKLLIY<u>YTSSLH</u><u>S</u>GVPSRFSGSGSGTDYSLTISNLEQEDIATYFC<u>QQGNTLPWT</u>FGGGTKLEIK.

Note: in the sequences of the heavy chain variable regions and the light chain variable regions of the above antibodies, the underlined parts represent CDR sequences determined according to the Kabat numbering scheme, and the regions were arranged in the following order: FR1-CDR1-FR2-CDR2-FR3-CDR3-FR4.

Table 4. CDR sequences of heavy chain and light chain of murine antibodies M23, M32, and M33

| Name of antibody | Heavy chain | | Light chain | |
|---|---|---|---|---|
| M23 | HCDR1 | DYEMH (SEQ ID NO: 8) | LCDR1 | RSSQSLVHSNGNTYLE (SEQ ID NO: 11) |
| | HCDR2 | LIDPETGGTVYNQKFKD (SEQ ID NO: 9) | LCDR2 | KVSNRFS (SEQ ID NO: 12) |
| | HCDR3 | ERFSYYGSTSDWYFDV (SEQ ID NO: 10) | LCDR3 | FQGSHVPYT (SEQ ID NO: 13) |
| M32 | HCDR1 | DYEIH (SEQ ID NO: 14) | LCDR1 | RSSQSIVHSNGNTYLE (SEQ ID NO: 17) |
| | HCDR2 | LFDPETGGIVYNQKFKG (SEQ ID NO: 15) | LCDR2 | KVSNRFS (SEQ ID NO: 12) |
| | HCDR3 | EGYNRDWYFDV (SEQ ID NO: 16) | LCDR3 | FQGSHVPYA (SEQ ID NO: 18) |
| M33 | HCDR1 | SYAMS (SEQ ID NO: 21) | LCDR1 | RASQDINNFLN (SEQ ID NO: 24) |
| | HCDR2 | TISGGGVDTYYQDNVQG (SEQ ID NO: 22) | LCDR2 | YTSSLHS (SEQ ID NO: 25) |
| | HCDR3 | PYGHGYFDV (SEQ ID NO: 23) | LCDR3 | QQGNTLPWT (SEQ ID NO: 26) |
| Note: the CDR sequences of the antibodies in the table were determined according to the Kabat numbering scheme. | | | | |

## Example 2. Humanization of Anti-Human PD-1 Monoclonal Antibodies

By aligning the IMGT human antibody heavy and light chain variable region germline gene database with the MOE software, human germline heavy and light chain variable region germline genes with high sequence identity to light and heavy chain sequences of M23, M32 and M33 were selected as templates, CDRs of the 3 murine antibodies were separately grafted into corresponding human antibody templates to construct corresponding humanized antibodies.

1. Humanization of murine antibody M23

1.1 Humanized framework selection of murine antibody M23

For the murine antibody M23, the humanized light chain templates were IGKV2-40*01 and IGKJ4*01, and the humanized heavy chain templates were IGHV1-69*02 and IGHJ6*01. The humanized variable region sequences are as follows (the underlined parts represent CDR sequences):

Hu23VH-CDR grafted: (SEQ ID NO: 27)

EVQLVQSGAEVKKPGSSVKVSCKASGGTFS<u>DYEMH</u>WVRQAPGQGLEWMG<u>LID PETGGTVYNQKFKD</u>RVTITADKSTSTAYMELSSLRSEDTAVYYCAR<u>ERFSYYGS TSDWYFDV</u>WGQGTTVTVSS.

Hu23VL-CDR grafted: (SEQ ID NO: 28)

DIVMTQTPLSLPVTPGEPASISC<u>RSSQSLVHSNGNTYLE</u>WYLQKPGQSPQLLIYK<u>VSNRFS</u>GVPDRFSGSGSGTDFTLKISRVEAEDVGVYYC<u>FQGSHVPYT</u>FGGGTKV EIK.

1.2 Humanized template selection and back mutation design of murine antibody M23

Table 5. Back mutations of humanized antibodies of murine antibody M23

| VL | | VH | |
|---|---|---|---|
| Hu23VL1 | Grafted | Hu23VH1 | Grafted |
| Hu23VL2 | I2G | Hu23VH2 | G27Y I69L |
| | | Hu23VH3 | G27Y M48I V67T I69L L82F |
| | | Hu23VH4 | G27Y M48I V67T I69L L82F A93T |
| Note: grafted indicates that the CDRs of the murine antibody are grafted into the human germline FR region sequences, wherein amino acid residues are determined and annotated according to the Kabat numbering scheme, for example, I2G indicates that I at position 2 (numbered by the Kabat numbering scheme) is back-mutated to G according to the Kabat numbering scheme. | | | |

The light/heavy chain variable region sequences of the humanized antibodies of M23 are as follows:

> Hu23VL1 (same as Hu23VL-CDR grafted): (SEQ ID NO: 28)

DIVMTQTPLSLPVTPGEPASISCRSSQSLVHSNGNTYLEWYLQKPGQSPQLLIYK VSNRFSGVPDRFSGSGSGTDFTLKISRVEAEDVGVYYCFQGSHVPYTFGGGTKV EIK.

> Hu23VL2 (SEQ ID NO: 29)

DGVMTQTPLSLPVTPGEPASISCRSSQSLVHSNGNTYLEWYLQKPGQSPQLLIYK VSNRFSGVPDRFSGSGSGTDFTLKISRVEAEDVGVYYCFQGSHVPYTFGGGTKV EIK.

> Hu23VH1 (same as Hu23VH-CDR grafted): (SEQ ID NO: 27)

EVQLVQSGAEVKKPGSSVKVSCKASGGTFSDYEMHWVRQAPGQGLEWMGLID PETGGTVYNQKFKDRVTITADKSTSTAYMELSSLRSEDTAVYYCARERFSYYGS TSDWYFDVWGQGTTVTVSS.

> Hu23VH2 (SEQ ID NO: 30)

EVQLVQSGAEVKKPGSSVKVSCKASGYTFSDYEMHWVRQAPGQGLEWMGLID PETGGTVYNQKFKDRVTLTADKSTSTAYMELSSLRSEDTAVYYCARERFSYYGS TSDWYFDVWGQGTTVTVSS.

> Hu23VH3 (SEQ ID NO: 31)

EVQLVQSGAEVKKPGSSVKVSCKASGYTFSDYEMHWVRQAPGQGLEWIGLIDP ETGGTVYNQKFKDRTTLTADKSTSTAYMEFSSLRSEDTAVYYCARERFSYYGST SDWYFDVWGQGTTVTVSS.

> Hu23VH4 (SEQ ID NO: 32) EVQLUQ SGAEVKKPGS SVKV SCKASGYTF SDYEMHW VRQAPGQGLEWIGLIDP ETGGTVYNQKFKDRTTLTADKSTSTAYMEFSSLRSEDTAVYYCTRERFSYYGSTS DWYFD VWGQGTT VT VS S.

1.3 Humanized sequence combinations of murine antibody M23

The antibody obtained after humanization of murine antibody M23 and variable region combinations thereof are shown in the table below.

Table 6. Variable region combinations of humanized antibody Hu23

| VH \ VL | Hu23VL1 | Hu23VL2 |
|---|---|---|
| Hu23VH1 | Hu23-1 | Hu23-5 |
| Hu23VH2 | Hu23-2 | Hu23-6 |
| Hu23VH3 | Hu23-3 | Hu23-7 |
| Hu23VH4 | Hu23-4 | Hu23-8 |

Note: "Hu23-1" refers to an antibody in which the light chain variable region is Hu23VL1 and the heavy chain variable region is Hu23VH1, and so on for others.

The antibody light/heavy chain variable region combinations referred to in the above table (e.g., Hu23-1) can be separately linked to antibody light/heavy chain constant regions to form full-length antibodies; in the present disclosure, in the formation of a full-length antibody, unless otherwise specifically stated, the light chain variable region was linked to the Kappa chain constant region set forth in SEQ ID NO: 73 to form an antibody light chain, and the heavy chain variable region was linked to the IgG4-AA heavy chain constant region set forth in SEQ ID NO: 72 or the IgG4-P heavy chain constant region set forth in SEQ ID NO: 79 to form an antibody heavy chain, and the name referring to the antibody light/heavy chain variable region combinations in the table (e.g., Hu23-1) was suffixed ".IgG4AA" to indicate a full-length antibody formed by linking the heavy chain variable region to the IgG4-AA heavy chain constant region, and suffixed ".IgG4P" to indicate a full-length antibody formed by linking the heavy chain variable region to the IgG4-P heavy chain constant region. For example, "Hu23-1.IgG4AA" indicates a full-length antibody consisting of a heavy chain formed by linking the Hu23VH1 heavy chain variable region to the IgG4-AA heavy chain constant region set forth in SEQ ID NO: 72, and a light chain formed by linking the Hu23VL1 light chain variable region to the Kappa chain constant region set forth in SEQ ID NO: 73. "Hu23-1.IgG4P" indicates a full-length antibody consisting of a heavy chain formed by linking the Hu23VH1 heavy chain variable region to the IgG4-P heavy chain constant region set forth in SEQ ID NO: 79, and a light chain formed by linking the Hu23VL1 light chain variable region to the Kappa chain constant region set forth in SEQ ID NO: 73.

2. Humanization of murine antibody M32

2.1 Humanized framework selection of murine antibody M32

For the murine antibody M32, the humanized light chain templates were IGKV2-40*01 and IGKJ4*01, and the humanized heavy chain templates were IGHV1-69*02 and IGHJ6*01. The humanized variable region sequences are as follows (the underlined parts represent CDR sequences):

Hu32VH-CDR grafted: (SEQ ID NO: 33) IGHV1-69*02 and IGHJ6*01

EVQLVQSGAEVKKPGSSVKVSCKASGGTFS<u>DYEIH</u>WVRQAPGQGLEWMG<u>LFD PETGGIVYNQKFKG</u>RVTITADKSTSTAYMELSSLRSEDTAVYYCAR<u>EGYNRDWY FDV</u>WGQGTTVTVSS.

Hu32VL-CDR grafted: (SEQ ID NO: 34)

DIVMTQTPLSLPVTPGEPASISC<u>RSSQSIVHSNGNTYLE</u>WYLQKPGQSPQLLIY<u>KV SNRFS</u>GVPDRFSGSGSGTDFTLKISRVEAEDVGVYYC<u>FQGSHVPYA</u>FGGGTKVEI K.

2.2 Humanized template selection and back mutation design of murine antibody M32

Table 7. Back mutations of humanized antibodies of murine antibody M32

| VL | | VH | |
|---|---|---|---|
| Hu32VL1 | Grafted | Hu32VH1 | Grafted |
| Hu32VL2 | I2V | Hu32VH2 | G27F, I69L, A93T |
| | | Hu32VH3 | G26D, G27F, I69L, A93T |
| | | Hu32VH4 | G27F, M48I, V67A, I69L, L82F, A93T |
| | | Hu32VH5 | G26D, G27F, S30T, M48I, V67A, I69L, L82F, A93T |
| | | Hu32VH6 | G26D, G27F, S30T, R38K, Q43H, M48I, R66K, V67A, I69L, L82F, A93T |
| Note: grafted indicates that the CDRs of the murine antibody are grafted into the human germline FR region sequences, wherein amino acid residues are determined and annotated according to the Kabat numbering scheme, for example, I2V indicates that I at position 2 (numbered by the Kabat numbering scheme) is back-mutated to V according to the Kabat numbering scheme. | | | |

The light chain and heavy chain variable region sequences of the humanized antibodies of the murine antibody M32 are as follows:

> Hu32VL1 (same as Hu32VL-CDR grafted): (SEQ ID NO: 34)

DIVMTQTPLSLPVTPGEPASISCRSSQSIVHSNGNTYLEWYLQKPGQSPQLLIYKV
SNRFSGVPDRFSGSGSGTDFTLKISRVEAEDVGVYYCFQGSHVPYAFGGGTKVEI
K.

> Hu32VL2 (SEQ ID NO: 35)

DVVMTQTPLSLPVTPGEPASISCRSSQSIVHSNGNTYLEWYLQKPGQSPQLLIYK
VSNRFSGVPDRFSGSGSGTDFTLKISRVEAEDVGVYYCFQGSHVPYAFGGGTKV
EIK.

> Hu32VH1 (same as Hu32VH-CDR grafted): (SEQ ID NO: 33)

EVQLVQSGAEVKKPGSSVKVSCKASGGTFSDYEIHWVRQAPGQGLEWMGLFD
PETGGIVYNQKFKGRVTITADKSTSTAYMELSSLRSEDTAVYYCAREGYNRDWY
FDVWGQGTTVTVSS.

> Hu32VH2 (SEQ ID NO: 36)

EVQLVQSGAEVKKPGSSVKVSCKASGFTFSDYEIHWVRQAPGQGLEWMGLFDP
ETGGIVYNQKFKGRVTLTADKSTSTAYMELSSLRSEDTAVYYCTREGYNRDWYF
DVWGQGTTVTVSS.

> Hu32VH3 (SEQ ID NO: 37)

EVQLVQSGAEVKKPGSSVKVSCKASDFTFSDYEIHWVRQAPGQGLEWMGLFDP
ETGGIVYNQKFKGRVTLTADKSTSTAYMELSSLRSEDTAVYYCTREGYNRDWYF
DVWGQGTTVTVSS.

> Hu32VH4 (SEQ ID NO: 38)

EVQLVQSGAEVKKPGSSVKVSCKASGFTFSDYEIHWVRQAPGQGLEWIGLFDP
ETGGIVYNQKFKGRATLTADKSTSTAYMEFSSLRSEDTAVYYCTREGYNRDWYF
DVWGQGTTVTVSS.

> Hu32VH5 (SEQ ID NO: 39)

EVQLVQSGAEVKKPGSSVKVSCKASDFTFTDYEIHWVRQAPGQGLEWIGLFDP
ETGGIVYNQKFKGRATLTADKSTSTAYMEFSSLRSEDTAVYYCTREGYNRDWYF
DVWGQGTTVTVSS.

> Hu32VH6 (SEQ ID NO: 40)

EVQLVQSGAEVKKPGSSVKVSCKASDFTFTDYEIHWVKQAPGHGLEWIGLFDP
ETGGIVYNQKFKGKATLTADKSTSTAYMEFSSLRSEDTAVYYCTREGYNRDWYF
DVWGQGTTVTVSS.

2.3 Humanized sequence combinations of murine antibody M32

The antibody obtained after humanization of murine antibody M32 and variable region combinations thereof are shown in the table below.

Table 8. Light/heavy chain variable region combinations of humanized antibody Hu32

| VH \ VL | Hu32VL1 | Hu32VL2 |
|---|---|---|
| Hu32VH1 | Hu32-1 | Hu32-7 |
| Hu32VH2 | Hu32-2 | Hu32-8 |
| Hu32VH3 | Hu32-3 | Hu32-9 |
| Hu32VH4 | Hu32-4 | Hu32-10 |
| Hu32VH5 | Hu32-5 | Hu32-11 |
| Hu32VH6 | Hu32-6 | Hu32-12 |

Note: in the table, for example, "Hu32-1" refers to an antibody light/heavy chain variable region combination in which the light chain variable region is Hu32VL1 and the heavy chain variable region is Hu32VH1, and so on for others.

The antibody light/heavy chain variable region combinations referred to in the above table (e.g., Hu32-1) can be separately linked to antibody light/heavy chain constant regions to form full-length antibodies; in the present disclosure, in the formation of a full-length antibody, unless otherwise specifically stated, the light chain variable region was linked to the Kappa chain constant region set forth in SEQ ID NO: 73 to form an antibody light chain, and the heavy chain variable region was linked to the IgG4-AA heavy chain constant region set forth in SEQ ID NO: 72 or the IgG4-P heavy chain constant region set forth in SEQ ID NO: 79 to form an antibody heavy chain, and the name referring to the antibody light/heavy chain variable region combinations in the table (e.g., Hu32-1) was suffixed ".IgG4AA" to indicate a full-length antibody formed by linking the heavy chain variable region to the IgG4-AA heavy chain constant region, and suffixed ".IgG4P" to indicate a full-length antibody formed by linking the heavy chain variable region to the IgG4-P heavy chain constant region. For example, "Hu32-1.IgG4AA" indicates a full-length antibody consisting of a heavy chain formed by linking the Hu32VH1 heavy chain variable region to the IgG4-AA heavy chain constant region set forth in SEQ ID NO: 72, and a light chain formed by linking the Hu32VL1 light chain variable region to the Kappa chain constant region set forth in SEQ ID NO: 73. "Hu32-1.IgG4P" indicates a full-length antibody consisting of a heavy chain formed by linking the Hu32VH1 heavy chain variable region to the IgG4-P heavy chain constant region set forth in SEQ ID NO: 79, and a light chain formed by linking the Hu32VL1 light chain variable region to the Kappa chain constant region set forth in SEQ ID NO: 73.

3. Humanization of murine antibody M33

3.1 Humanized framework selection of murine antibody M33

For the murine antibody M33, the humanized light chain templates were IGKV1-39*01 and IGKJ4*01, and the humanized heavy chain templates were IGHV3-7 and IGHJ6*01. The humanized variable region sequences are as follows:

Hu33VH-CDR grafted (SEQ ID NO: 41):

EVQLVESGGGLVQPGGSLRLSCAASGFTFSSYAMSWVRQAPGKGLEWVATISGG GVDTYYQDNVQGRFTISRDNAKNSLYLQMNSLRAEDTAVYYCARPYGHGYFD VWGQGTTVTVSS.

Hu33VL-CDR grafted (SEQ ID NO: 42):

DIQMTQSPSSLSASVGDRVTITCRASQDINNFLNWYQQKPGKAPKLLIYYTSSLH SGVPSRFSGSGSGTDFTLTISSLQPEDFATYYCQQGNTLPWTFGGGTKVEIK.

3.2 Humanized template selection and back mutation design of murine antibody M33

Table 9. Back mutations of humanized antibodies of murine antibody M33

| VL | | VH | |
|---|---|---|---|
| Hu33VL1 | Grafted | Hu33VH1 | Grafted |
| Hu33VL2 | F71Y | Hu33VH2 | R94S |
| Hu33VL3 | K42G P44V F71Y | Hu33VH3 | E1K R94S |
| Note: grafted indicates that the CDRs of the murine antibody are grafted into the human germline FR region sequences, wherein amino acid residues are determined and annotated according to the Kabat numbering scheme, for example, F71Y indicates that F at position 71 (numbered by the Kabat numbering scheme) is back-mutated to Y according to the Kabat numbering scheme. | | | |

Light chain variable region and heavy chain variable region sequences of the humanized antibodies of the murine antibody M33 are as follows:

> Hu33VL1 (same as Hu33VL-CDR grafted): (SEQ ID NO: 42)

DIQMTQSPSSLSASVGDRVTITCRASQDINNFLNWYQQKPGKAPKLLIYYTSSLH SGVPSRFSGSGSGTDFTLTISSLQPEDFATYYCQQGNTLPWTFGGGTKVEIK.

> Hu33VL2 (SEQ ID NO: 43)

DIQMTQSPSSLSASVGDRVTITCRASQDINNFLNWYQQKPGKAPKLLIYYTSSLH SGVPSRFSGSGSGTDYTLTISSLQPEDFATYYCQQGNTLPWTFGGGTKVEIK.

> Hu33VL3 (SEQ ID NO: 44)

DIQMTQSPSSLSASVGDRVTITCRASQDINNFLNWYQQKPGGAVKLLIYYTSSLH SGVPSRFSGSGSGTDYTLTISSLQPEDFATYYCQQGNTLPWTFGGGTKVEIK.

> Hu33VH1 (same as Hu33VH-CDR grafted): (SEQ ID NO: 41)

EVQLVESGGGLVQPGGSLRLSCAASGFTFSSYAMSWVRQAPGKGLEWVATISGG
GVDTYYQDNVQGRFTISRDNAKNSLYLQMNSLRAEDTAVYYCARPYGHGYFD
VWGQGTTVTVSS.

> Hu33VH2 (SEQ ID NO: 45)

EVQLVESGGGLVQPGGSLRLSCAASGFTFSSYAMSWVRQAPGKGLEWVATISGG
GVDTYYQDNVQGRFTISRDNAKNSLYLQMNSLRAEDTAVYYCASPYGHGYFD
VWGQGTTVTVSS.

> Hu33VH3 (SEQ ID NO: 46)

KVQLVESGGGLVQPGGSLRLSCAASGFTFSSYAMSWVRQAPGKGLEWVATISG
GGVDTYYQDNVQGRFTISRDNAKNSLYLQMNSLRAEDTAVYYCASPYGHGYF
DVWGQGTTVTVSS.

3.3 Humanized sequence combinations of murine antibody M33

Table 10. Light/heavy chain variable region combinations of humanized antibody

| VL / VH | Hu33VL1 | Hu33VL2 | Hu33VL3 |
|---------|---------|---------|---------|
| Hu33VH1 | Hu33-1 | Hu33-4 | Hu33-7 |
| Hu33VH2 | Hu33-2 | Hu33-5 | Hu33-8 |
| Hu33VH3 | Hu33-3 | Hu33-6 | Hu33-9 |

Note: in the table, for example, "Hu33-6" refers to an antibody light/heavy chain variable region combination in which the light chain variable region is Hu33VL2 and the heavy chain variable region is Hu33VH3, and so on for others.

The antibody light/heavy chain variable region combinations referred to in the above table (e.g., Hu33-6) can be separately linked to antibody light/heavy chain constant regions to form full-length antibodies; in the present disclosure, in the formation of a full-length antibody, unless otherwise specifically stated, the light chain variable region was linked to the Kappa chain constant region set forth in SEQ ID NO: 73 to form an antibody light chain, and the heavy chain variable region was linked to the IgG4-AA heavy chain constant region set forth in SEQ ID NO: 72 or the IgG4-P heavy chain constant region set forth in SEQ ID NO: 79 to form an antibody heavy chain, and the name referring to the antibody light/heavy chain variable region combinations in the table (e.g., Hu33-6) was suffixed ".IgG4AA" to indicate a full-length antibody formed by linking the heavy chain variable region to the IgG4-AA heavy chain constant region, and suffixed ".IgG4P" to indicate a full-length antibody formed by linking the heavy chain variable region to the IgG4-P heavy chain constant region. For example, "Hu33-6.IgG4AA" indicates a full-length antibody consisting of a heavy chain formed by linking the Hu33VH3 heavy chain variable region to the IgG4-AA heavy chain constant region set forth in SEQ ID NO: 72, and a light chain formed by linking the Hu33VL2 light chain variable region to the Kappa chain constant region set forth in SEQ ID NO: 73. "Hu33-6.IgG4P" indicates a full-length antibody consisting of a heavy chain formed by linking the Hu33VH3 heavy chain variable region to the IgG4-P heavy chain constant region set forth in SEQ ID NO: 79, and a light chain formed by linking the Hu33VL2 light chain variable region to the Kappa chain constant region set forth in SEQ ID NO: 73.

4. Mutants of humanized antibodies

4.1 Mutant antibodies of humanized antibody Hu23

Site-directed mutagenesis was performed on amino acids at particular positions of the light chain LCDR1 (SEQ ID NO: 11) of the humanized antibody Hu23 by computer simulation. The specific mutations are shown in Table 11.

Table 11. Mutated sequences of light chain LCDR1 of Hu23:

| Name | Sequence (SEQ ID NO) |
|---|---|
| Hu23LCDR1 (N28Q) | RSSQSLUHSQGNTYLE (SEQ ID NO: 47) |
| Hu23LCDR1 (N28L) | RSSQSLVHSLGNTYLE (SEQ ID NO: 48) |
| Hu23LCDR1 (N28T) | RSSQSLVHSTGNTYLE (SEQ ID NO: 49) |
| Hu23LCDR1 (N28D) | RSSQSLVHSDGNTYLE (SEQ ID NO: 50) |
| Hu23LCDR1 (G29A) | RSSQSLVHSNANTYLE (SEQ ID NO: 51) |
| Hu23LCDR1 (G29V) | RSSQSLVHSNVNTYLE (SEQ ID NO: 52) |
| Note: Hu23LCDR1(N28Q) indicates an LCDR1 mutated sequence in which N at position 28 (according to the Kabat numbering scheme) of the light chain variable region Hu23VL1 or Hu23VL2 of humanized antibody Hu23 is mutated to Q, and Hu23LCDR1(G29A) indicates an LCDR1 mutated sequence in which G at position 29 (according the Kabat numbering scheme) of the light chain variable region Hu23VL1 or Hu23VL2 of humanized antibody Hu23 is mutated to A (CDRs are determined according to the Kabat numbering scheme). | |

The light chain variable region sequences of the humanized antibody Hu23 after LCDR1 mutation are as follows:

> Sequence of Hu23VL1(N28Q):

DIVMTQTPLSLPVTPGEPASISCRSSQSLVHSQGNTYLEWYLQKPGQSPQLLIYK
VSNRFSGVPDRFSGSGSGTDFTLKISRVEAEDVGVYYCFQGSHVPYTFGGGTKV
EIK     (SEQ ID NO: 53)

> Sequence of >Hu23VL1(N28L):

DIVMTQTPLSLPVTPGEPASISCRSSQSLVHSLGNTYLEWYLQKPGQSPQLLIYK
VSNRFSGVPDRFSGSGSGTDFTLKISRVEAEDVGVYYCFQGSHVPYTFGGGTKV
EIK     (SEQ ID NO: 54)

> Sequence of Hu23VL1(N28T):

DIVMTQTPLSLPVTPGEPASISCRSSQSLVHSTGNTYLEWYLQKPGQSPQLLIYK
VSNRFSGVPDRFSGSGSGTDFTLKISRVEAEDVGVYYCFQGSHVPYTFGGGTKV
EIK     (SEQ ID NO: 55)

> Sequence of Hu23VL1(N28D):

DIVMTQTPLSLPVTPGEPASISCRSSQSLVHSDGNTYLEWYLQKPGQSPQLLIYK
VSNRFSGVPDRFSGSGSGTDFTLKISRVEAEDVGVYYCFQGSHVPYTFGGGTKV
EIK     (SEQ ID NO: 56)

> Sequence of Hu23VL1(G29A):

DIVMTQTPLSLPVTPGEPASISCRSSQSLVHSNANTYLEWYLQKPGQSPQLLIYK
VSNRFSGVPDRFSGSGSGTDFTLKISRVEAEDVGVYYCFQGSHVPYTFGGGTKV
EIK    (SEQ ID NO: 57)

> Sequence of Hu23VL1(G29V):

DIVMTQTPLSLPVTPGEPASISCRSSQSLVHSNVNTYLEWYLQKPGQSPQLLIYK
VSNRFSGVPDRFSGSGSGTDFTLKISRVEAEDVGVYYCFQGSHVPYTFGGGTKV
EIK    (SEQ ID NO: 58)

> Sequence of Hu23VL2(N28Q):

DGVMTQTPLSLPVTPGEPASISCRSSQSLVHSQGNTYLEWYLQKPGQSPQLLIYK
VSNRFSGVPDRFSGSGSGTDFTLKISRVEAEDVGVYYCFQGSHVPYTFGGGTKV
EIK    (SEQ ID NO: 59)

> Sequence of Hu23VL2(N28L):

DGVMTQTPLSLPVTPGEPASISCRSSQSLVHSLGNTYLEWYLQKPGQSPQLLIYK
VSNRFSGVPDRFSGSGSGTDFTLKISRVEAEDVGVYYCFQGSHVPYTFGGGTKV
EIK    (SEQ ID NO: 60)

> Sequence of Hu23VL2(N28T):

DGVMTQTPLSLPVTPGEPASISCRSSQSLVHSTGNTYLEWYLQKPGQSPQLLIYK
VSNRFSGVPDRFSGSGSGTDFTLKISRVEAEDVGVYYCFQGSHVPYTFGGGTKV
EIK    (SEQ ID NO: 61)

> Sequence of Hu23VL2(N28D):

DGVMTQTPLSLPVTPGEPASISCRSSQSLVHSDGNTYLEWYLQKPGQSPQLLIYK
VSNRFSGVPDRFSGSGSGTDFTLKISRVEAEDVGVYYCFQGSHVPYTFGGGTKV
EIK    (SEQ ID NO: 62)

> Sequence of Hu23VL2(G29A):

DGVMTQTPLSLPVTPGEPASISCRSSQSLVHSNANTYLEWYLQKPGQSPQLLIYK
VSNRFSGVPDRFSGSGSGTDFTLKISRVEAEDVGVYYCFQGSHVPYTFGGGTKV
EIK    (SEQ ID NO: 63)

> Sequence of Hu23VL2(G29V):

DGVMTQTPLSLPVTPGEPASISCRSSQSLVHSNVNTYLEWYLQKPGQSPQLLIYK
VSNRFSGVPDRFSGSGSGTDFTLKISRVEAEDVGVYYCFQGSHVPYTFGGGTKV
EIK　　(SEQ ID NO: 64)

Table 12. Light/heavy chain variable region combinations of humanized antibody Hu23

| VH / VL | Hu23VH1 | Hu23VH2 | Hu23VH3 | Hu23VH4 |
|---|---|---|---|---|
| Hu23VL1(N28Q) | Hu23-9 | Hu23-21 | Hu23-33 | Hu23-45 |
| Hu23VL1(N28L) | Hu23-10 | Hu23-22 | Hu23-34 | Hu23-46 |
| Hu23VL1(N28T) | Hu23-11 | Hu23-23 | Hu23-35 | Hu23-47 |
| Hu23VL1(N28D) | Hu23-12 | Hu23-24 | Hu23-36 | Hu23-48 |
| Hu23VL1(G29A) | Hu23-13 | Hu23-25 | Hu23-37 | Hu23-49 |
| Hu23VL1(G29V) | Hu23-14 | Hu23-26 | Hu23-38 | Hu23-50 |
| Hu23VL2(N28Q) | Hu23-15 | Hu23-27 | Hu23-39 | Hu23-51 |
| Hu23VL2(N28L) | Hu23-16 | Hu23-28 | Hu23-40 | Hu23-52 |
| Hu23VL2(N28T) | Hu23-17 | Hu23-29 | Hu23-41 | Hu23-53 |
| Hu23VL2(N28D) | Hu23-18 | Hu23-30 | Hu23-42 | Hu23-54 |
| Hu23VL2(G29A) | Hu23-19 | Hu23-31 | Hu23-43 | Hu23-55 |
| Hu23VL2(G29V) | Hu23-20 | Hu23-32 | Hu23-44 | Hu23-56 |

Note: in the table, for example, "Hu23-11" refers to an antibody light/heavy chain variable region combination in which the light chain variable region is Hu23VL1(N28T) and the heavy chain variable region is Hu23VH1, and so on for others.

The antibody light/heavy chain variable region combinations referred to in the above table (e.g., Hu23-11) can be separately linked to antibody light/heavy chain constant regions to form full-length antibodies; in the present disclosure, in the formation of a full-length antibody, unless otherwise specifically stated, the light chain variable region was linked to the Kappa chain constant region set forth in SEQ ID NO: 73 to form an antibody light chain, and the heavy chain variable region was linked to the IgG4-AA heavy chain constant region set forth in SEQ ID NO: 72 or the IgG4-P heavy chain constant region set forth in SEQ ID NO: 79 to form an antibody heavy chain, and the name referring to the antibody light/heavy chain variable region combinations in the table (e.g., Hu23-11) was suffixed ".IgG4AA" to indicate a full-length antibody formed by linking the heavy chain variable region to the IgG4-AA heavy chain constant region, and suffixed ".IgG4P" to indicate a full-length antibody formed by linking the heavy chain variable region to the IgG4-P heavy chain constant region. For example, "Hu23-11.IgG4AA" indicates a full-length antibody consisting of a heavy chain formed by linking the Hu23VH1 heavy chain variable region to the IgG4-AA heavy chain constant region set forth in SEQ ID NO: 72, and a light chain formed by linking the Hu23VL1(N28T) light chain variable region to the Kappa chain constant region set forth in SEQ ID NO: 73. "Hu23-11.IgG4P" indicates a full-length antibody consisting of a heavy chain formed by linking the Hu23VH1 heavy chain variable region to the IgG4-P heavy chain constant region set forth in SEQ ID NO: 79, and a light chain formed by linking the Hu23VL1(N28T) light chain variable region to the Kappa chain constant region set forth in SEQ ID NO: 73.

The experimental results showed that the humanized antibodies after site-directed mutagenesis of Hu23LCDR1(N28Q), Hu23LCDR1(N28L), Hu23LCDR1(N28T), Hu23LCDR1(N28D), Hu23LCDR1(G29A) and Hu23LCDR1(G29V) all retained the binding ability to PD-1 (Table 16).

4.2 Mutant antibodies of humanized antibody Hu32

Sequence analysis showed that the series of humanized antibodies Hu23 derived from M23 had high sequence identity to the series of humanized antibodies Hu32 derived from M32. The light chain variable region of Hu23 and the heavy chain variable region of Hu32 were combined to form a new light chain and heavy chain variable region combination. The experimental results showed that the humanized antibodies comprising the new light chain and heavy chain variable region combination all retained the binding ability to the PD-1 antigen (Table 16).

Table 13. General formulas of variable region consensus sequences of antibodies Hu32 and Hu23

| Heavy chain | | | Light chain | |
|---|---|---|---|---|
| HCDR1 | DYEX$_1$H, wherein X$_1$ is selected from the group consisting of I and M; (SEQ ID NO: 65) | | LCDR1 | RSSQSX$_{13}$VHSX$_{14}$X$_{15}$X$_{16}$TYLE, wherein X$_{13}$ is selected from the group consisting of I and L, X$_{14}$ is selected from the group consisting of N, Q, L, T and D, X$_{15}$ is selected from the group consisting of G, A and V, and X$_{16}$ is selected from the group consisting of N and K; (SEQ ID NO: 68) |
| HCDR2 | LX$_2$DPETGGX$_3$VYNQKFKX$_4$, wherein X$_2$ is selected from the group consisting of F and I, X$_3$ is selected from the group consisting of I and T, and X$_4$ is selected from the group consisting of G and D; (SEQ ID NO: 66) | | LCDR2 | KVSNRFS; (SEQ ID NO: 12) |
| HCDR3 | EX$_5$X$_6$X$_7$X$_8$YX$_9$X$_{10}$X$_{11}$X$_{12}$DWYFDV, wherein X$_5$ is selected from the group consisting of G and R, X$_6$ is F or vacancy, X$_7$ is S or vacancy, X$_8$ is Y or vacancy, X$_9$ is G or vacancy, X$_{10}$ is S or vacancy, X$_{11}$ is selected from the group consisting of N and T, and X$_{12}$ is selected from the group consisting of R and S; (SEQ ID NO: 67) | | LCDR3 | FQGSHVPYX$_{17}$, wherein X$_{17}$ is selected from the group consisting of A and T; (SEQ ID NO: 69) |
| VH | EVQLUQSGAEVKKPGSSVKVSCKASX$_{18}$X$_{19}$TFX$_{20}$DYEX$_1$HWVX$_{21}$QAPGX$_{22}$GLEWX$_{23}$GLX$_2$DPETGGX$_3$VYNQKFKX$_4$X$_{24}$X$_{25}$TX$_{26}$TADKSTSTAYMEX$_{27}$SSLRSEDTAVYYCX$_{28}$REX$_5$X$_6$X$_7$X$_8$YX$_9$X$_{10}$X$_{11}$X$_{12}$DWYFDVWGQGTTVTVSS, wherein X$_1$ is selected from the group consisting of I and M, X$_2$ is selected from the group consisting of F and I, X$_3$ is selected from the group consisting of I and T, X$_4$ is selected from the group consisting of G and D, X$_5$ is selected from the group consisting of G and R, X$_6$ is F or vacancy, X$_7$ is S or vacancy, X$_8$ is Y or vacancy, X$_9$ is G or vacancy, X$_{10}$ is S or vacancy, X$_{11}$ is selected from the group consisting of N and T, X$_{12}$ is selected from the group consisting of R and S, X$_{18}$ is selected from the group consisting of G and D, X$_{19}$ is selected from the group consisting of G, F and Y, X$_{20}$ is selected from the group consisting of S and T, X$_{21}$ is selected from the group consisting of R and K, X$_{22}$ is selected from the group consisting of Q and H, X$_{23}$ is selected from the group consisting of M and I, X$_{24}$ is selected from the group consisting of R and K, X$_{25}$ is selected from the group consisting of V, A and T, X$_{26}$ is selected from the group consisting of R and K, X$_{27}$ is selected from the group consisting of L and F, and X$_{28}$ is selected from the group consisting of A and T. (SEQ ID NO: 70) | | VL | DX$_{29}$VMTQTPLSLPVTPGEPASISCRSSQSX$_{13}$VHSX$_{14}$X$_{15}$X$_{16}$TYLEWYLQKPGQSPQLLIYKVSNRFSGVPDRFSGSGSGTDFTLKISRVEAEDVGVYYCFQGSHVPYX$_{17}$FGGGTKVEIK, wherein X$_{13}$ is selected from the group consisting of I and L, X$_{14}$ is selected from the group consisting of N, Q, L, T and D, X$_{15}$ is selected from the group consisting of G, A and V, X$_{16}$ is selected from the group consisting of N and K, X$_{17}$ is selected from the group consisting of A and T, and X$_{29}$ is selected from the group consisting of I, V and G. (SEQ ID NO: 71) |

Table 14. Combination of heavy chain variable region of Hu32 and light chain variable region of Hu23

| VL \ VH | Hu32VH1 | Hu32VH2 | Hu32VH3 | Hu32VH4 | Hu32VH5 | Hu32VH6 |
|---|---|---|---|---|---|---|
| Hu23VL1(N28Q) | Hu32a-13 | Hu32a-27 | Hu32a-41 | Hu32a-55 | Hu32a-69 | Hu32a-83 |

| | | | | | | |
|---|---|---|---|---|---|---|
| Hu23VL1(N28L) | Hu32a-14 | Hu32a-28 | Hu32a-42 | Hu32a-56 | Hu32a-70 | Hu32a-84 |
| Hu23VL1(N28T) | Hu32a-15 | Hu32a-29 | Hu32a-43 | Hu32a-57 | Hu32a-71 | Hu32a-85 |
| Hu23VL1(N28D) | Hu32a-16 | Hu32a-30 | Hu32a-44 | Hu32a-58 | Hu32a-72 | Hu32a-86 |
| Hu23VL1(G29A) | Hu32a-17 | Hu32a-31 | Hu32a-45 | Hu32a-59 | Hu32a-73 | Hu32a-87 |
| Hu23VL1(G29V) | Hu32a-18 | Hu32a-32 | Hu32a-46 | Hu32a-60 | Hu32a-74 | Hu32a-88 |
| Hu23VL2(N28Q) | Hu32a-19 | Hu32a-33 | Hu32a-47 | Hu32a-61 | Hu32a-75 | Hu32a-89 |
| Hu23VL2(N28L) | Hu32a-20 | Hu32a-34 | Hu32a-48 | Hu32a-62 | Hu32a-76 | Hu32a-90 |
| Hu23VL2(N28T) | Hu32a-21 | Hu32a-35 | Hu32a-49 | Hu32a-63 | Hu32a-77 | Hu32a-91 |
| Hu23VL2(N28D) | Hu32a-22 | Hu32a-36 | Hu32a-50 | Hu32a-64 | Hu32a-78 | Hu32a-92 |
| Hu23VL2(G29A) | Hu32a-23 | Hu32a-37 | Hu32a-51 | Hu32a-65 | Hu32a-79 | Hu32a-93 |
| Hu23VL2(G29V) | Hu32a-24 | Hu32a-38 | Hu32a-52 | Hu32a-66 | Hu32a-80 | Hu32a-94 |
| Hu23VL1 | Hu32a-25 | Hu32a-39 | Hu32a-53 | Hu32a-67 | Hu32a-81 | Hu32a-95 |
| Hu23VL2 | Hu32a-26 | Hu32a-40 | Hu32a-54 | Hu32a-68 | Hu32a-82 | Hu32a-96 |

Note: in the table, for example, "Hu32a-85" refers to an antibody light/heavy chain variable region combination in which the light chain variable region is Hu23VL1(N28T) and the heavy chain variable region is Hu32VH6, and so on for others.

The antibody light/heavy chain variable region combinations referred to in the above table (e.g., Hu32a-85) can be separately linked to antibody light/heavy chain constant regions to form full-length antibodies; in the present disclosure, in the formation of a full-length antibody, unless otherwise specifically stated, the light chain variable region was linked to the Kappa chain constant region set forth in SEQ ID NO: 73 to form an antibody light chain, and the heavy chain variable region was linked to the IgG4-AA heavy chain constant region set forth in SEQ ID NO: 72 or the IgG4-P heavy chain constant region set forth in SEQ ID NO: 79 to form an antibody heavy chain, and the name referring to the antibody light/heavy chain variable region combinations in the table (e.g., Hu32a-85) was suffixed ".IgG4AA" to indicate a full-length antibody formed by linking the heavy chain variable region to the IgG4-AA heavy chain constant region, and suffixed ".IgG4P" to indicate a full-length antibody formed by linking the heavy chain variable region to the IgG4-P heavy chain constant region. For example, "Hu32a-85.IgG4AA" indicates a full-length antibody consisting of a heavy chain formed by linking the Hu32VH6 heavy chain variable region to the IgG4-AA heavy chain constant region set forth in SEQ ID NO: 72, and a light chain formed by linking the Hu23VL1(N28T) light chain variable region to the Kappa chain constant region set forth in SEQ ID NO: 73. "Hu32a-85.IgG4P" indicates a full-length antibody consisting of a heavy chain formed by linking the Hu32VH6 heavy chain variable region to the IgG4-P heavy chain constant region set forth in SEQ ID NO: 79, and a light chain formed by linking the Hu23VL1(N28T) light chain variable region to the Kappa chain constant region set forth in SEQ ID NO: 73.

Table 15. Combination of heavy chain variable region of Hu23 and light chain variable region of Hu32

| VL \ VH | Hu23VH1 | Hu23VH2 | Hu23VH3 |
|---------|---------|---------|---------|
| Hu32VL1 | Hu23a-57 | Hu23a-59 | Hu23a-61 |
| Hu32VL2 | Hu23a-58 | Hu23a-60 | Hu23a-62 |

Note: in the table, for example, "Hu23a-57" refers to an antibody light/heavy chain variable region combination in which the light chain variable region is Hu32VL1 and the heavy chain variable region is Hu23VH1, and so on for others.

The antibody light/heavy chain variable region combinations referred to in the above table (e.g., Hu23a-57) can be separately linked to antibody light/heavy chain constant regions to form full-length antibodies; in the present disclosure, in the formation of a full-length antibody, unless otherwise specifically stated, the light chain variable region was linked to the Kappa chain constant region set forth in SEQ ID NO: 73 to form an antibody light chain, and the heavy chain variable region was linked to the IgG4-AA heavy chain constant region set forth in SEQ ID NO: 72 or the IgG4-P heavy chain constant region set forth in SEQ ID NO: 79 to form an antibody heavy chain, and the name referring to the antibody light/heavy chain variable region combinations in the table (e.g., Hu32a-85) was suffixed ".IgG4AA" to indicate a full-length antibody formed by linking the heavy chain variable region to the IgG4-AA heavy chain constant region, and suffixed ".IgG4P" to indicate a full-length antibody formed by linking the heavy chain variable region to the IgG4-P heavy chain constant region. For example, "Hu23a-57.IgG4AA" indicates a full-length antibody consisting of a heavy chain formed by linking the Hu23VH1 heavy chain variable region to the IgG4-AA heavy chain constant region set forth in SEQ ID NO: 72, and a light chain formed by linking the Hu32VL1 light chain variable region to the Kappa chain constant region set forth in SEQ ID NO: 73. "Hu23a-57.IgG4P" indicates a full-length antibody consisting of a heavy chain formed by linking the Hu23VH1 heavy chain variable region to the IgG4-P heavy chain constant region set forth in SEQ ID NO: 79, and a light chain formed by linking the Hu32VL1 light chain variable region to the Kappa chain constant region set forth in SEQ ID NO: 73.

5. Screening of humanized antibodies

An affinity assay was performed on different humanized antibodies by Biacore (see Test Example 3 for the method). The results are shown in Table 16. The results showed that different humanized antibodies retained the binding ability to PD-1, and the affinity of part of humanized antibodies was even substantially similar to that of their murine antibodies.

Table 16. Affinity of humanized antibodies Hu23 for human PD-1

| Antibody | ka (1/Ms) | kd (1/s) | KD (M) |
|----------|-----------|----------|--------|
| M23 | 4.57E+04 | 1.32E-04 | 2.89E-09 |
| Hu23-1.IgG4AA | 3.43E+04 | 1.10E-04 | 3.20E-09 |
| Hu23-5.IgG4AA | 2.99E+04 | 1.14E-04 | 3.82E-09 |
| Hu23-2.IgG4AA | 2.74E+04 | 1.61E-04 | 5.86E-09 |
| Hu23-6.IgG4AA | 2.79E+04 | 1.55E-04 | 5.57E-09 |
| Hu23-3.IgG4AA | 2.93E+04 | 1.60E-04 | 5.46E-09 |
| Hu23-7.IgG4AA | 2.77E+04 | 1.66E-04 | 5.97E-09 |
| Hu23-4.IgG4AA | 4.24E+04 | 1.44E-04 | 3.39E-09 |
| Hu23-8.IgG4AA | 4.11E+04 | 1.47E-04 | 3.56E-09 |
| Hu23-9.IgG4AA | 6.16E+04 | 1.32E-04 | 2.15E-09 |
| Hu23-10.IgG4AA | 5.51E+04 | 1.53E-04 | 2.77E-09 |
| Hu23-11.IgG4AA | 4.22E+04 | 1.14E-04 | 2.71E-09 |
| Hu23-12.IgG4AA | 5.45E+04 | 1.10E-04 | 2.02E-09 |
| Hu23-13.IgG4AA | 4.24E+04 | 1.22E-04 | 2.88E-09 |
| Hu23-14.IgG4AA | 7.23E+04 | 1.61E-04 | 2.22E-09 |

(continued)

| Antibody | ka (1/Ms) | kd (1/s) | KD (M) |
|---|---|---|---|
| M32 | 7.83E+04 | 4.15E-04 | 5.3E-09 |
| Hu32a-15.IgG4AA | 4.89E+04 | 2.52E-03 | 5.14E-08 |
| Hu32a-29.IgG4AA | 7.89E+04 | 6.20E-04 | 7.86E-09 |
| Hu32a-43.IgG4AA | 8.39E+04 | 6.85E-04 | 8.16E-09 |
| Hu32a-57.IgG4AA | 7.94E+04 | 6.24E-04 | 7.85E-09 |
| Hu32a-71.IgG4AA | 8.60E+04 | 3.96E-04 | 4.61E-09 |
| Hu32a-85.IgG4AA | 9.90E+04 | 3.15E-04 | 3.18E-09 |
| M33 | 3.08E+05 | 2.27E-04 | 7.37E-10 |
| Hu33-1.IgG4AA | 6.61E+04 | 1.28E-03 | 1.93E-08 |
| Hu33-4.IgG4AA | 8.11E+04 | 2.55E-03 | 3.14E-08 |
| Hu33-7.IgG4AA | 7.69E+04 | 2.60E-03 | 3.38E-08 |
| Hu33-2.IgG4AA | 1.35E+05 | 1.43E-04 | 1.06E-09 |
| Hu33-5.IgG4AA | 1.46E+05 | 1.35E-04 | 9.26E-10 |
| Hu33-8.IgG4AA | 1.53E+05 | 1.62E-04 | 1.06E-09 |
| Hu33-3.IgG4AA | 1.25E+05 | 1.36E-04 | 1.09E-09 |
| Hu33-6.IgG4AA | 1.30E+05 | 1.40E-04 | 1.08E-09 |
| Hu33-9.IgG4AA | 1.40E+05 | 1.53E-04 | 1.09E-09 |

**Example 3. Construction and Expression of PD-1 Humanized Antibody**

Primers were designed for constructing VH/VK gene fragments of various humanized antibodies, which were subjected to homologous recombination with an expression vector pHr (with a signal peptide and a constant region gene (CH1-Fc/CL) fragment) to construct an expression vector VH-CH1-Fc-pHr/VK-CL-pHr for full-length antibodies. IgG4-P represents the S228P (corresponding to position 108 of the sequence SEQ ID NO: 72 or SEQ ID NO: 79) mutation, IgG4-AA represents the F234A (corresponding to position 114 of the sequence SEQ ID NO: 72 or SEQ ID NO: 79), L235A (corresponding to position 115 of the sequence SEQ ID NO: 72 or SEQ ID NO: 79) and S228P (corresponding to position 108 of the sequence SEQ ID NO: 72 or SEQ ID NO: 79) mutations, and IgG4-AA and IgG4-P antibody forms can be obtained by simple site-directed mutagenesis of the IgG4 antibody form.

Heavy chain constant region sequence of IgG4-AA (SEQ ID NO: 72):

ASTKGPSVFPLAPCSRSTSESTAALGCLVKDYFPEPVTVSWNSGALTSGVHTFPA
VLQSSGLYSLSSVVTVPSSSLGTKTYTCNVDHKPSNTKVDKRVESKYGPPCPP̲CP
APEA̲A̲GGPSVFLFPPKPKDTLMISRTPEVTCVVVDVSQEDPEVQFNWYVDGVE
VHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNY
KTTPPVLDSDGSFFLYSRLTVDKSRWQEGNVFSCSVMHEALHNHYTQKSLSLSL
GK.

Light chain (Kappa chain) constant region sequence of the antibody (SEQ ID NO: 73):

RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC.

An exemplary list of sequences of full-length antibodies constructed in the form of IgG4AA is as follows:

Heavy chain of Hu23-1 LIgG4AA antibody (SEQ ID NO: 74):

EVQLVQSGAEVKKPGSSVKVSCKASGGTFSDYEMHWVRQAPGQGLEWMGLID
PETGGTVYNQKFKDRVTITADKSTSTAYMELSSLRSEDTAVYYCARERFSYYGS
TSDWYFDVWGQGTTVTVSSASTKGPSVFPLAPCSRSTSESTAALGCLVKDYFPE
PVTVSWNSGALTSGVHTFPAVLQSSGLYSLSSVVTVPSSSLGTKTYTCNVDHKPS
NTKVDKRVESKYGPPCPPCPAPEAAGGPSVFLFPPKPKDTLMISRTPEVTCVVVD
VSQEDPEVQFNWYVDGVEVHNAKTKPREEQFNSTYRVVSVLTVLHQDWLNGK
EYKCKVSNKGLPSSIEKTISKAKGQPREPQVYTLPPSQEEMTKNQVSLTCLVKGF
YPSDIAVEWESNGQPENNYKTTPPVLDSDGSFFLYSRLTVDKSRWQEGNVFSCS
VMHEALHNHYTQKSLSLSLGK.

Light chain of Hu23-11.IgG4AA(SEQ ID NO: 75):

DIVMTQTPLSLPVTPGEPASISCRSSQSLVHSTGNTYLEWYLQKPGQSPQLLIYK
VSNRFSGVPDRFSGSGSGTDFTLKISRVEAEDVGVYYCFQGSHVPYTFGGGTKV
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC.

Heavy chain of Hu32a-85.IgG4AA (SEQ ID NO: 76):

EVQLVQSGAEVKKPGSSVKVSCKASDFTFTDYEIHWVKQAPGHGLEWIGLFDP
ETGGIVYNQKFKGKATLTADKSTSTAYMEFSSLRSEDTAVYYCTREGYNRDWYF
DVWGQGTTVTVSSASTKGPSVFPLAPCSRSTSESTAALGCLVKDYFPEPVTVSW
NSGALTSGVHTFPAVLQSSGLYSLSSVVTVPSSSLGTKTYTCNVDHKPSNTKVDK
RVESKYGPPCPPCPAPEAAGGPSVFLFPPKPKDTLMISRTPEVTCVVVDVSQEDP
EVQFNWYVDGVEVHNAKTKPREEQFNSTYRVVSVLTVLHQDWLNGKEYKCK
VSNKGLPSSIEKTISKAKGQPREPQVYTLPPSQEEMTKNQVSLTCLVKGFYPSDIA
VEWESNGQPENNYKTTPPVLDSDGSFFLYSRLTVDKSRWQEGNVFSCSVMHEA
LHNHYTQKSLSLSLGK.

Light chain of Hu32a-85.IgG4AA (same as the light chain of Hu23-11.IgG4AA, SEQ ID NO: 75):

 DIVMTQTPLSLPVTPGEPASISCRSSQSLVHSTGNTYLEWYLQKPGQSPQLLIYK

VSNRFSGVPDRFSGSGSGTDFTLKISRVEAEDVGVYYCFQGSHVPYTFGGGTKV
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC.

Heavy chain of Hu33-6.IgG4AA (SEQ ID NO: 77):

KVQLVESGGGLVQPGGSLRLSCAASGFTFSSYAMSWVRQAPGKGLEWVATISG GGVDTYYQDNVQGRFTISRDNAKNSLYLQMNSLRAEDTAVYYCASPYGHGYF DVWGQGTTVTVSSASTKGPSVFPLAPCSRSTSESTAALGCLVKDYFPEPVTVSW NSGALTSGVHTFPAVLQSSGLYSLSSVVTVPSSSLGTKTYTCNVDHKPSNTKVDK RVESKYGPPCPPCPAPEAAGGPSVFLFPPKPKDTLMISRTPEVTCVVVDVSQEDP EVQFNWYVDGVEVHNAKTKPREEQFNSTYRVVSVLTVLHQDWLNGKEYKCK VSNKGLPSSIEKTISKAKGQPREPQVYTLPPSQEEMTKNQVSLTCLVKGFYPSDIA VEWESNGQPENNYKTTPPVLDSDGSFFLYSRLTVDKSRWQEGNVFSCSVMHEA LHNHYTQKSLSLSLGK.

Light chain of Hu33-6.IgG4AA (SEQ ID NO: 78):

DIQMTQSPSSLSASVGDRVTITCRASQDINNFLNWYQQKPGKAPKLLIYYTSSLH SGVPSRFSGSGSGTDYTLTISSLQPEDFATYYCQQGNTLPWTFGGGTKVEIKRTV AAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC.

Heavy chain constant region sequence of IgG4-P (SEQ ID NO: 79):

ASTKGPSVFPLAPCSRSTSESTAALGCLVKDYFPEPVTVSWNSGALTSGVHTFPA VLQSSGLYSLSSVVTVPSSSLGTKTYTCNVDHKPSNTKVDKRVESKYGPPCP_PCP APEFLGGPSVFLFPPKPKDTLMISRTPEVTCVVVDVSQEDPEVQFNWYVDGVEV HNAKTKPREEQFNSTYRVVSVLTVLHQDWLNGKEYKCKVSNKGLPSSIEKTISK AKGQPREPQVYTLPPSQEEMTKNQVSLTCLVKGFYPSDIAVEWESNGQPENNYK TTPPVLDSDGSFFLYSRLTVDKSRWQEGNVFSCSVMHEALHNHYTQKSLSLSLG K.

An exemplary list of sequences of full-length antibodies constructed in the form of IgG4-P is as follows:

Heavy chain of Hu23-11.IgG4P antibody (SEQ ID NO: 80):

EVQLVQSGAEVKKPGSSVKVSCKASGGTFSDYEMHWVRQAPGQGLEWMGLID PETGGTVYNQKFKDRVTITADKSTSTAYMELSSLRSEDTAVYYCARERFSYYGS TSDWYFDVWGQGTTVTVSSASTKGPSVFPLAPCSRSTSESTAALGCLVKDYFPE PVTVSWNSGALTSGVHTFPAVLQSSGLYSLSSVVTVPSSSLGTKTYTCNVDHKPS NTKVDKRVESKYGPPCPPCPAPEFLGGPSVFLFPPKPKDTLMISRTPEVTCVVVD VSQEDPEVQFNWYVDGVEVHNAKTKPREEQFNSTYRVVSVLTVLHQDWLNGK EYKCKVSNKGLPSSIEKTISKAKGQPREPQVYTLPPSQEEMTKNQVSLTCLVKGF YPSDIAVEWESNGQPENNYKTTPPVLDSDGSFFLYSRLTVDKSRWQEGNVFSCS VMHEALHNHYTQKSLSLSLGK.

Light chain of Hu23-11.IgG4P (same as the light chain of Hu23-11.IgG4AA, SEQ ID NO: 75):

DIVMTQTPLSLPVTPGEPASISCRSSQSLVHSTGNTYLEWYLQKPGQSPQLLIYK
VSNRFSGVPDRFSGSGSGTDFTLKISRVEAEDVGVYYCFQGSHVPYTFGGGTKV
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC.

Heavy chain of Hu32a-85.IgG4P (SEQ ID NO: 81):

EVQLVQSGAEVKKPGSSVKVSCKASDFTFTDYEIHWVKQAPGHGLEWIGLFDP
ETGGIVYNQKFKGKATLTADKSTSTAYMEFSSLRSEDTAVYYCTREGYNRDWYF
DVWGQGTTVTVSSASTKGPSVFPLAPCSRSTSESTAALGCLVKDYFPEPVTVSW
NSGALTSGVHTFPAVLQSSGLYSLSSVVTVPSSSLGTKTYTCNVDHKPSNTKVDK
RVESKYGPPCPPCPAPEFLGGPSVFLFPPKPKDTLMISRTPEVTCVVVDVSQEDPE
VQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIAV
EWESNGQPENNYKTTPPVLDSDGSFFLYSRLTVDKSRWQEGNVFSCSVMHEAL
HNHYTQKSLSLSLGK.

Light chain of Hu32a-85. IgG4P (same as the light chain of Hu23-11.IgG4AA, SEQ ID NO: 75):

DIVMTQTPLSLPVTPGEPASISCRSSQSLVHSTGNTYLEWYLQKPGQSPQLLIYK
VSNRFSGVPDRFSGSGSGTDFTLKISRVEAEDVGVYYCFQGSHVPYTFGGGTKV
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC.

Heavy chain of Hu33-6.IgG4P (SEQ ID NO: 82):

KVQLVESGGGLVQPGGSLRLSCAASGFTFSSYAMSWVRQAPGKGLEWVATISG
GGVDTYYQDNVQGRFTISRDNAKNSLYLQMNSLRAEDTAVYYCASPYGHGYF
DVWGQGTTVTVSSASTKGPSVFPLAPCSRSTSESTAALGCLVKDYFPEPVTVSW
NSGALTSGVHTFPAVLQSSGLYSLSSVVTVPSSSLGTKTYTCNVDHKPSNTKVDK
RVESKYGPPCPPCPAPEFLGGPSVFLFPPKPKDTLMISRTPEVTCVVVDVSQEDPE
VQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIAV
EWESNGQPENNYKTTPPVLDSDGSFFLYSRLTVDKSRWQEGNVFSCSVMHEAL
HNHYTQKSLSLSLGK.

Light chain of Hu33-6.IgG4P (same as the light chain of Hu33-6.IgG4AA, SEQ ID NO: 78):

DIQMTQSPSSLSASVGDRVTITCRASQDINNFLNWYQQKPGKAPKLLIYYTSSLH
SGVPSRFSGSGSGTDYTLTISSLQPEDFATYYCQQGNTLPWTFGGGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC.

**Test Examples**

**Test Example 1. Assay on the Binding of Anti-PD-1 Antibodies to PD-1 Ligand *In Vitro* and the Blocking of Binding by ELISA**

PD-L1 on the surface of tumor cells has an inhibitory effect on the proliferation of T cells by binding to PD-1 on the surface of T cells. PD-1 antibodies can block the signaling pathway of PD-L1/PD-1 by binding to PD-1, thereby stimulating the proliferation of T cells. The assay on the blocking of PD-1/PD-L1 binding is used to detect the blocking activity of anti-PD-1 antibodies on the signaling pathway.

In this experiment, a 96-well plate was coated with PD-1-His protein (Cat.# 10377H08H, Sino Biological), and test anti-PD-1 antibodies (including antibodies: Hu23-11.IgG4AA, Hu32a-85.IgG4AA and Hu33-6.IgG4AA, and a positive control antibody: H005-1 (see H005-1 antibody in WO2015085847)) were separately added for an incubation reaction, followed by the addition of HRP-labeled goat anti-human IgG (H+L) antibody (Cat.# 109-035-003, Jackson ImmunoResearch) for an incubation reaction. After the plate was washed, the binding amount of HRP-labeled goat anti-human IgG (H+L) was determined, and $EC_{50}$ values of the anti-PD-1 antibodies for the ligand PD-1 binding were calculated.

In this experiment, a 96-well plate was coated with PD-1 protein with the extracellular region fused to Fc (PD-1-Fc, with the sequence set forth in SEQ ID NO: 1), and test anti-PD-1 antibodies (including antibodies: Hu23-11.IgG4AA, Hu32a-85.IgG4AA and Hu33-6.IgG4AA, and a positive control antibody: H005-1 (see H005-1 antibody in WO2015085847)) were separately added for an incubation reaction, followed by the addition of biotin-labeled PD-L1/PD-L2 for an incubation reaction. After the plate was washed, the binding amount of the biotin-labeled PD-L1/PD-L2 was determined, and $IC_{50}$ values of the anti-PD-1 antibodies for the blocking of the ligand PD-L1/PD-L2 binding were calculated.

PD-1-Fc was diluted to 1 $\mu$g/mL with a CB buffer at pH 9.6 (1.59 g of $Na_2CO_3$ and 2.93 g of $NaHCO_3$ in 1 L of distilled water) and added to a 96-well plate at 100 $\mu$L/well, and the plate was left to stand at 4 °C for 16-20 h. The PBS buffer was removed by pipetting from the 96-well plate. After the plate was washed once with a PBST (PBS containing 0.05% tween 20, pH 7.4) buffer, PBST/1% milk was added at 120 $\mu$L/well, and the mixture was incubated at room temperature for 1 h for blocking. The blocking solution was removed. After the plate was washed once with a PBST buffer, the test anti-PD-1 antibody diluted to appropriate concentrations with a sample diluent (PBS containing 5% BSA and 0.05% Tween 20, pH 7.4) was added at 90 $\mu$L/well, and the mixture was pre-incubated at 4 °C for 1 h. 10× biotin-labeled PD-L1/PD-L2 (Sino Biological) (10 $\mu$g/mL) was added at 10 $\mu$L/well, and the mixture was shaken on a shaker and mixed well, and incubated at 37 °C for 1 h. The reaction system was removed. After the plate was washed 6 times with PBST, streptavidin-peroxidase polymer diluted in a 1:400 ratio with a PBST buffer was added at 100 $\mu$L/well, and the mixture was incubated at room temperature for 50 min with shaking. After the plate was washed 6 times with PBST, TMB was added at 100 $\mu$L/well, and the mixture was incubated at room temperature for 5-10 min. The reaction was stopped by adding 1 M $H_2SO_4$ at 100 $\mu$L/well. The absorbance was read at 450 nm with a microplate reader, and $IC_{50}$ value of the anti-PD-1 antibodies for the blocking of the ligand PD-L1/PD-L2 binding was calculated. The data are detailed in Table 17 below.

Table 17. Assay on the binding of the anti-PD-1 antibodies of the present disclosure to PD-1 and blocking of ligand PD-L1/PD-L2 binding by ELISA

| Antibody | Antigen binding | | Blocking of binding of PD-1 to PD-L1/PD-L2 | |
|---|---|---|---|---|
| | huPD-1-his OD value MAX | hu PD-1-his EC50 (ng/mL) | Hu PD-1-Fc/ PD-L1 IC50 (ng/mL) | Hu PD-1-Fc/ PD-L2 IC50 (ng/mL) |
| Hu23-11.IgG4AA | 1.46 | 237.3 | 92.35 | 245.1 |
| Hu32a-85.IgG4AA | 1.265 | 135.9 | 78.63 | 205.9 |
| Hu33-6.IgG4AA | 1.706 | 205.6 | 103.5 | 207.2 |
| H005-1 | 1.21 | 113.1 | 109.6 | 225.3 |

The exemplary anti-PD-1 antibodies Hu23-11.IgG4AA, Hu32a-85.IgG4AA and Hu33-6.IgG4AA of the present disclosure were all able to effectively block the binding of PD-1 to PD-L1/PD-L2 with blocking activity similar to the positive control antibody.

**Test Example 2. Assay on the Blocking of Ligand by Exemplary Antibodies**

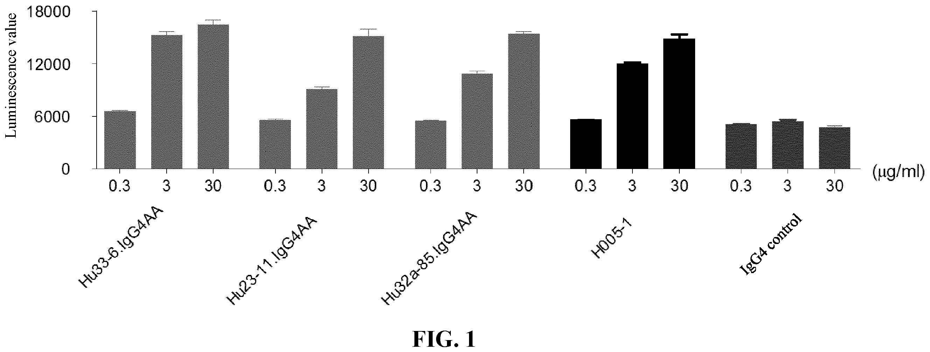

The blocking effect of the antibodies on the binding of PD-1 to PD-L1 was investigated. The experimental procedures were briefly described as follows:
CHOK1/PD-L1 cells (Promega) were digested and added to a 96-well plate at 100 $\mu$L/well, and the plate was incubated in an incubator at 37 °C with 5% $CO_2$ for 24 h. The control and sample were diluted to the desired concentration with PBS. Jurkat/PD-1 cells (Jurkat cells stably transformed with PD-1) were counted, and seeded into a cell culture plate containing CHOK1/PD-L1 cells in a certain ratio (90 $\mu$L/well), and diluted antibodies (antibodies: Hu23-11.IgG4AA, Hu32a-85.IgG4AA and Hu33-6.IgG4AA, a positive control antibody: H005-1, and a negative control IgG4 protein, antibody gradient dilution concentrations of 0.3 mg/mL, 3 mg/mL and 30 mg/mL) were added at 10 $\mu$L/well. The plate was incubated in an incubator at 37 °C with 5% $CO_2$ for 5 h. The cell culture plate was taken out and left to stand at room temperature for 5 min, then 50 $\mu$L of Bio-Glo™ Reagent was added to each well, and the plate was incubated at room temperature for 5 min and read. The experimental results are shown in FIG. 1.
The results showed that the exemplary anti-PD-1 antibodies Hu23-11.IgG4AA, Hu32a-85.IgG4AA and Hu33-6.IgG4AA of the present disclosure were able to effectively block the binding of PD-1 to PD-L1.

**Test Example 3. Assay on Affinity of Exemplary Antibodies by BIAcore**

IgG was subjected to affinity capture using a Protein A biosensor chip (Cat.# 29127556, GE), human PD-1 antigen (Cat.# 10377H08H, Sino Biological) and Cyno PD-1 antigen (purchased from Sino Biological) were allowed to flow through the surface of the chip, and reaction signals of PD-1 antibodies and PD-1 antigens were detected in real time using a Biacore T200 instrument to obtain association and dissociation curves. After the dissociation was completed for each assay cycle, the biosensor chip was washed and regenerated with a 10 mM Glycine-HCl buffer at pH 1.5. The experimental buffer system was a 1× HBS-EP buffer solution (Cat# BR-1001-88, GE). After the experiment was completed, the data were fitted with a (1:1) Langmuir model using GE Biacore T200 Evaluation version 3.0 software to obtain affinity values. The results are shown in Table 18.

Table 18. Affinity of anti-PD-1 antibodies for human PD-1 and monkey PD-1

| Antibody | Antigen | ka (1/Ms) | kd (1/s) | KD (M) |
|---|---|---|---|---|
| Hu23-11.IgG4AA | hPD-1-His | 6.06E+04 | 1.13E-04 | 1.86E-09 |
| | Cyno PD-1 | 4.79E+04 | 1.26E-04 | 2.62E-09 |
| Hu32a-85.IgG4AA | hPD-1-His | 8.56E+04 | 2.78E-04 | 3.25E-09 |
| | Cyno PD-1 | 1.16E+05 | 4.75E-04 | 4.11E-09 |
| Hu33-6.IgG4AA | hPD-1-His | 1.24E+05 | 1.14E-04 | 9.18E-10 |
| | Cyno PD-1 | 2.51E+05 | 2.41E-04 | 9.60E-10 |

The results showed that the exemplary anti-PD-1 antibodies Hu23-11.IgG4AA, Hu32a-85.IgG4AA and Hu33-6.IgG4AA of the present disclosure were all able to bind to human PD-1 and monkey PD-1.

**Test Example 4. Effect of Antibodies on the Secretion of IFN$\gamma$ from Cells in PBMC-T Lymphocyte Activation Assay**

In order to investigate the effect of anti-PD-1 antibodies on the function of human primary T lymphocytes, human peripheral blood mononuclear cells (PBMCs) were collected and purified, stimulated with tuberculin (TB) for 5 days *in vitro,* and determined for the secretion level of cytokine IFN$\gamma$. The experimental procedures were briefly described as follows:
Fresh blood was subjected to density gradient centrifugation (Stem Cell Technologies) using Ficoll-Hypaque (17-5442-02, GE) to obtain PBMCs, which were cultured in an RPMI 1640 (SH30809.01, GE) medium (supplemented with 10% (v/v) FBS (10099-141, Gibco)) at 37 °C with 5% $CO_2$.
Freshly isolated and purified PBMCs were adjusted to a density of $2 \times 10^6$ cells/mL in an RPMI 1640 medium, and 20 mL of cell suspension was added with 40 $\mu$L tuberculin (97-8800, Synbiotics) and cultured in an incubator at 37

°C with 5% $CO_2$ for 5 days. On day 5, the cells cultured above were collected, centrifuged, resuspended in a fresh RPMI 1640 medium, adjusted to a density of $1.1 \times 10^6$ cells/mL, and seeded into a 96-well cell culture plate at 90 μL/well. The antibody samples diluted in a gradient (including antibodies of the present disclosure: Hu23-11.IgG4AA, Hu32a-85.IgG4AA and Hu33-6.IgG4AA, a positive control antibody H005-1, and a negative control IgG4 protein, antibody gradient dilution concentrations of 0.3 mg/mL, 3 mg/mL, 30 mg/mL) with PBS (B320, Shanghai BasalMedia) was added at 10 μL/well. The cell culture plate was incubated in an incubator at 37 °C with 5% $CO_2$ for 3 days. The cell culture plate was taken out and centrifuged (4000 rpm, 10 min), and the cell culture supernatant was collected and determined for the level of IFN-γ by ELISA (human IFN-γ assay kit (EHC102g.96, NeoBioscience)). The kit instructions were referred to for specific operations.

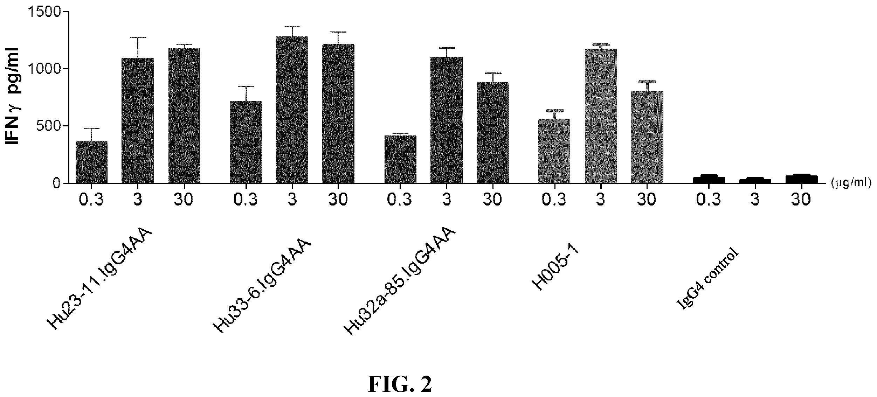

The results are shown in FIG. 2. The results showed that the anti-PD-1 antibodies Hu23-11.IgG4AA, Hu32a-85.IgG4AA and Hu33-6.IgG4AA of the present disclosure were all able to effectively activate the secretion of IFN-γ.

**Test Example 5. Effect of Anti-PD-1 Antibodies on Transgenic PD-1 Mouse Colon Cancer Model MC38**

MC38 cells were inoculated subcutaneously into the right flank of 90 hPD-1 TG mice (Biocytogen) at $5 \times 10^5$ cells/mouse/100 μL. After 10 days, animals with too great or too small tumor volume were removed, and mice were randomly divided into the following 7 groups according to the mean tumor volume of about 120 $mm^3$: blank control Vehicle (PBS), positive control H005-1 3 mpk, Hu32a-85.IgG4AA 1 mpk, Hu32a-85.IgG4AA 3 mpk, Hu23-11.IgG4AA 1 mpk, Hu23-11.IgG4AA 3 mpk and Hu33-6.IgG4AA 3 mpk, with 8 mice in each group. Each group of antibodies was intraperitoneally injected three times a week from Day 0. After the end of the first week of administration, it was found that tumors were significantly inhibited, and the frequency of administration was adjusted to once a week for a total of 5 times in the second and third weeks. The tumor volumes and body weights of animals were monitored twice a week, and data were recorded. The tumor-bearing animals were euthanized as the experimental endpoint when the tumor volume exceeded 2000 $mm^3$ or when most tumors showed rupture or when the tumor-bearing animals showed 20% of body weight loss.

$$\text{Tumor volume (TV)} = 1/2 \times L_{long} \times L_{short}^2$$

$$\text{Tumor proliferation rate (T/C\%)} = (T - T0)/(C - C0) \times 100\%$$

$$\text{Tumor growth inhibition (TGI\%)} = 1 - T/C \%$$

T and T0 represent the tumor volumes of the antibody administration group at the end of the experiment and at the beginning of the experiment, respectively, and C and C0 represent the tumor volumes of the blank control group at the end of the experiment and at the beginning of the experiment.

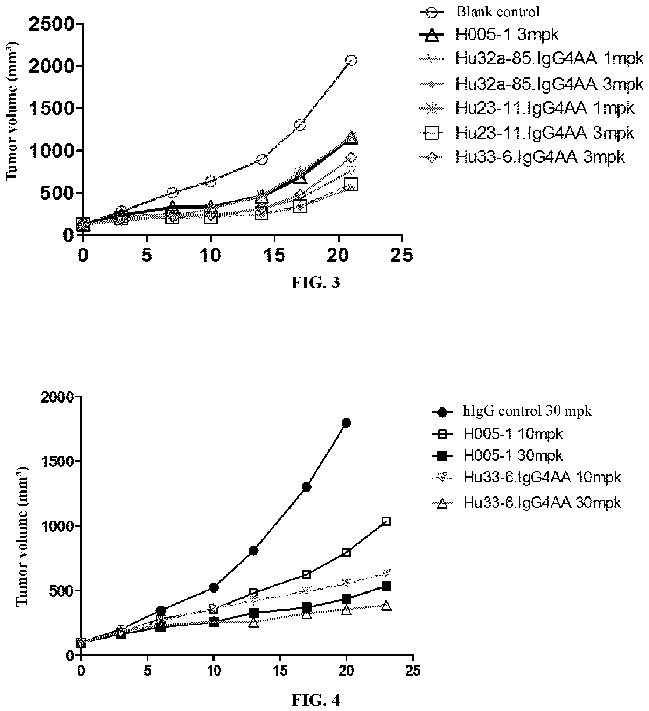

The test results are shown in Table 19 and FIG. 3. The test results showed that compared with the blank control group, the antibodies of the present disclosure were all able to significantly inhibit the growth of mouse colon cancer MC38-induced xenograft tumor, wherein the Hu32a-85.IgG4AA-3 mpk group had the highest tumor growth inhibition rate, with the tumor growth inhibition rate being 77.64% at the last measurement. After the administration frequency was three times a week for a total of 3 times, the results showed that the tumor growth inhibition rates of the antibodies of the present disclosure were significantly superior to those of the positive control antibody H005-1 when detected on the seventh day; after the administration frequency was decreased to once a week for a total of 2 times (Day 21), the antibodies of the present disclosure showed gradually significant differences in efficacy and showed a dose-dependent relationship, wherein Hu32a-85.IgG4AA was significantly superior to H005-1 at an equivalent dose ($p < 0.05$). Moreover, the tumor-bearing mice were able to well tolerate all of the anti-PD-1 antibodies, with the body weights stably increased during the whole treatment process, and no significant symptoms such as weight loss caused by medicaments observed.

Table 19. Effect of anti-PD-1 antibodies on tumor growth inhibition of mouse colon cancer MC38 ($mm^3$)

| Days after first administration | H005-1 | Hu32a-85.IgG4AA | | Hu23-11.IgG4AA | | Hu33-6.IgG4AA |
|---|---|---|---|---|---|---|
| | 3mpk | 1mpk | 3mpk | 1mpk | 3mpk | 3mpk |
| 21 | 46.82% | 67.12% | 77.64% | 45.12% | 75.32% | 59.31% |

**Test Example 6. Effect of Anti-PD-1 Antibodies on Transgenic PD-1 Mouse Colon Cancer Model MC38**

Transgenic PD-1 mice were derived from a fifth generation of purchased transgenic PD-1 mice (ISIS INNOVATION LIMITED, University Offices, Wellington Square, Oxford OX1 2JD, England) bred in Cephorim Biosciences, Inc. MC38 cells were inoculated subcutaneously into the posterior of the right flank of hPD-1 transgenic mice (half male and half female) at $5 \times 10^5$ cells/100 μL/mouse. When the mean tumor volume of the mice reached 80-100 mm³, the animals with too heavy or too light body weight or with too large or too small tumor were removed, and tumor-bearing mice were randomly divided into the following 5 groups (8 mice in each group) according to the tumor volume: negative control hIgG control 30 mpk, H005-1 10 mpk, H005-1 30 mpk, Hu33-6.IgG4AA 10 mpk, and Hu33-6.IgG4AA 30 mpk. The day of grouping and administration was set as Day 0. After the grouping, each group of drugs was administered intraperitoneally once every two days for 11 times with a 22-day administration cycle. The tumor volumes and body weights were measured twice a week, and data were recorded. The body weights and tumor volumes of each group of animals were expressed as mean ± standard deviation (Mean ± SEM) plotted using Graphpad Prism 5 and Excel software, and statistically analyzed using student t test.

$$\text{Tumor volume (TV)} = 0.5236 \times L_{long} \times L_{short}^2$$

$$\text{Tumor proliferation rate T/C\%} = (T - T0)/(C - C0) \times 100\%$$

$$\text{Tumor growth inhibition TGI\%} = 1 - \text{T/C \%}$$

T and T0 represent the tumor volumes of the antibody administration group at the end of the experiment and at the beginning of the experiment, respectively, and C and C0 represent the tumor volumes of the blank control group at the end of the experiment and at the beginning of the experiment.

The test results are shown in Table 20 and FIG. 4. The test results showed that compared with the control group, the antibodies of the present disclosure were able to significantly inhibit the growth of mouse colon cancer MC38-induced xenograft tumor, wherein the Hu33-6.IgG4AA 30 mpk group had the highest tumor growth inhibition rate, with the tumor growth inhibition rate being 80.4% when measured on day 20. In the low-dose group (10 mpk), Hu33-6.IgG4AA-10 mpk had better efficacy than the positive control H005-1-10 mpk.

Table 20. Effect of anti-PD-1 antibodies on volume of mouse colon cancer MC38 tumor

| Days after first administration | hIgG control group | H005-1 10 mpk group | H005-1 30 mpk group | Hu33-6.IgG4AA 10 mpk group | Hu33-6.IgG4AA 30 mpk group |
|---|---|---|---|---|---|
| 0 | 96.2 | 96.1 | 95.3 | 94.7 | 94.5 |
| 20 | 1797.2 | 794.0 | 434.9 | 550.1 | 352.8 |
| 23 | | 1034.2 | 534.1 | 632.4 | 387.6 |
| Note: the mean tumor volume in each group is given in the following unit: mm³. | | | | | |

**Test Example 7. Pharmacokinetic Assay of Anti-PD-1 Antibodies in Cynomolgus Monkey**

Six laboratory cynomolgus monkeys, male, 2-5 years old, 2-5 kg, were purchased from Guangdong Qianyan Biological Science and Technology Co., Ltd., with production license number of SCXK (Guangdong) 2015-0037, and animal certification number of 44613900000219.

Housing environment: the room temperature was controlled at 18-26 °C, the relative humidity was 40-70%, and the illumination was in a 12/12 light/dark cycle. The monkeys had unlimited access to feed and water except in the case of fasting.

Animals were weighed before administration, with the body weight controlled at 2.81-3.52 kg. Each group was administered to forelimb or hind limb by subcutaneous intravenous infusion using a syringe pump, at a dose of 1 mg/kg (1 mpk) and at a rate of 0.1 mL/kg/min for about 30 min. Whole blood was collected from the hind limb veins of animals before the administration, and 5 min, 0.25 h, 0.5 h (immediately after the administration ended), 1 h, 2 h, 4 h, 8 h, 1 d, 2 d, 3 d, 4 d, 5 d, 7 d, 10 d, 13 d, 14 d, 21 d and 28 d after the beginning of intravenous infusion, and serum was isolated.

About 2 mL of whole blood was collected before the administration, and 14 d, 21 d and 28 d after the beginning of intravenous infusion, and about 1 mL of whole blood was collected at the other blood collection points. The plasma concentration in serum was determined by ELISA for PK analysis. The results are shown in Table 21.

Table 21. Pharmacokinetics of humanized anti-PD-1 antibodies in cynomolgus monkeys

|  | Hu23-11.IgG4AA | Hu33-6.IgG4AA |
|---|---|---|
| t1/2 (day) | 5.5±0.7 | 4.6±1.3 |
| Cmax ($\mu$g/mL) | 23.75±2.29 | 21.47±2.13 |
| AUC (h*$\mu$g/mL) | 2775±241 | 2319±518 |
| CL (mL/day/kg) | 8.7±0.7 | 10.7±2.3 |
| Vz (mL/kg) | 69±3.6 | 67.9±3.4 |

The results showed that Hu23-11.IgG4AA and Hu33-6.IgG4AA had good pharmacokinetic activity.

**The stable anti-PD-1 antibody formulations were prepared by the following exemplary experiments, and the anti-PD-1 antibody in the following formulation implementation was Hu23-11.IgG4AA described above. The equipment used in the preparation process and the methods for calculating the results were as follows:** SEC molecular exclusion chromatography: a method for analyzing the separation of a solute by the relative relationship between the pore size of the gel pores and the size of the polymer sample molecule coil. SEC monomer content percentage = A monomer/A total $\times$ 100% (A monomer represents the peak area of the main peak monomer in the sample, and A total represents the sum of areas of all peaks). Instrument for SEC determination: Agilent 1260; column: waters, XBrige BEH200Å SEC (300 $\times$ 7.8 mm 3.5 $\mu$m).

CE capillary gel electrophoresis: a method of electrophoresis in which a gel is transferred into a capillary tube as a supporting medium, and a sample is separated according to its molecular weight under a certain voltage. Non-reduced CE (NR-CE) purity percentage = A main peak/A total $\times$ 100% (A main peak represents the peak area of the main peak in the sample, and A total represents the sum of areas of all peaks). Instrument for CE determination: Beckman, model: plus800.

iCIEF imaged capillary isoelectric focusing (iCE for short): a technique for separating according to the difference of isoelectric points pI of proteins. iCIEF neutral peak content percentage = neutral peak area/total area $\times$ 100% (total area represents the sum of areas of acidic, neutral and basic peaks). Instrument for iCIEF determination: simple protein, model: muarice.

Osmotic pressure: the freezing point method is used for determining the osmotic pressure. The freezing point of a solution is determined by using a high-sensitivity temperature-sensing element on the basis of the proportional relation between the freezing point depression value and the molar concentration of the solution, and then converted into the osmotic pressure through electric quantity. Manufacturer of instrument: Loser, model: OM815.

**Test Example 8. Screening of Buffer Systems and pH for Anti-PD-1 Antibody Formulations**

The anti-PD-1 antibody (Hu23-11.IgG4AA) formulations containing 80 mg/mL sucrose and 0.6 mg/mL polysorbate 80 (PS80) at a protein concentration of 100 mg/mL were prepared using the following buffer:

1) 10 mM sodium acetate (AA for short), pH 5.0;
2) 10 mM sodium acetate, pH 5.2;
3) 10 mM sodium acetate, pH 5.5;
4) 10 mM sodium acetate, pH 5.7;
5) 10 mM sodium succinate (SA for short), pH 5.2; or
6) 10 mM sodium citrate (CA for short), pH 5.2.

The prepared formulations were filtered and filled into containers, which were plugged and capped. The stability of the samples under a forced degradation condition (40 °C M1, i.e., storing at a high temperature of 40 °C for 1 month) and an accelerated condition (25 °C M6, i.e., storing at a temperature of 25 °C for 6 months) was investigated, and the stability of the formulations was investigated with appearance, SEC and non-reduced CE-SDS as evaluation indices. The experimental results are shown in Table 22.

Under the forced degradation condition of 40 °C M1, the appearance of the protein formulations in the AA and SA buffer systems was superior to that of the protein formulations in the CA system; the purity of the protein formulations in the AA buffer systems at pH 5.2-5.7 was superior to that of the protein formulations in the AA buffer system at pH 5.0 and the SA buffer system at pH 5.2; under the accelerated condition of 25 °C M6, there was no significant difference in appearance among groups.

Table 22. Experimental results of screening of pH and buffer systems

| No. | Conditions | Appearance | SEC monomer (%) | Non-reduced CE-SDS (%) |
|---|---|---|---|---|
| 01 | D0 | Clarified | 95.6 | 94.5 |
| | 40 °CM1 | Clarified | 89.0 | 85.1 |
| | 25 °CM6 | Turbid | 91.0 | 91.3 |
| 02 | D0 | Clarified | 95.5 | 94.5 |
| | 40 °CM1 | Clarified | 90.2 | 87.3 |
| | 25 °CM6 | Turbid | 92.6 | 92.0 |
| 03 | D0 | Clarified | 95.5 | 94.6 |
| | 40 °CM1 | Clarified | 90.5 | 88.4 |
| | 25 °CM6 | Turbid | 91.5 | 91.2 |
| 04 | D0 | Clarified | 95.5 | 94.7 |
| | 40 °CM1 | Clarified | 91.0 | 87.1 |
| | 25 °CM6 | Turbid | 91.6 | 92.5 |
| 05 | D0 | Clarified | 95.5 | 94.6 |
| | 40 °CM1 | Clarified | 88.3 | 84.7 |
| | 25 °CM6 | Turbid | 91.0 | 91.1 |
| 06 | D0 | Clarified | 95.4 | 94.9 |
| | 40 °CM1 | Turbid | 91.8 | 86.1 |
| | 25 °CM6 | Turbid | 93.2 | 92.4 |
| Note: D0 indicates the time at the beginning of the experiment, and "M" indicates month, e.g., M1 indicates one month. | | | | |

In addition, for another batch of anti-PD-1 antibodies (Hu23-11.IgG4AA), the anti-PD-1 antibody (Hu23-11.IgG4AA) formulations containing 80 mg/mL sucrose and 0.6 mg/mL polysorbate 80 (PS80) at a protein concentration of 100 mg/mL were prepared using the following buffer:

1) 10 mM sodium acetate, pH 5.2;
2) 10 mM sodium succinate, pH 5.2; or
3) 10 mM histidine acetate (His-AA for short), pH 5.2.

The prepared formulations were filtered and filled into containers, which were plugged and capped. The stability of the samples under an accelerated condition (25 °C M6, i.e., storing at a temperature of 25 °C for 6 months) was investigated, and the stability of the formulations was investigated according to the investigated SEC and iCIEF indices of the formulations. The experimental results are shown in Table 23.

Under the accelerated condition of 25 °C M6, the SEC/iCIEF data for AA at pH 5.2 and His-AA at pH 5.2 were higher than those for the SA group at pH 5.2, and iCIEF data for the AA buffer system at pH 5.2 was slightly higher than that for the His-AA buffer system at pH 5.2. Upon comprehensive consideration, the buffer system of the formulations is preferably acetate and histidine salt buffer systems, and is preferably an AA or His-AA buffer system.

Table 23. Experimental results of screening of pH and buffer systems

| No. | Conditions | SEC monomer (%) | Neutral peak iCIEF (%) |
|---|---|---|---|
| 01 | D0 | 99.6 | 54.4 |
| | 25 °CM6 | 97.0 | 42.7 |

(continued)

| No. | Conditions | SEC monomer (%) | Neutral peak iCIEF (%) |
|---|---|---|---|
| 02 | D0 | 99.2 | 54.6 |
| | 25 °CM6 | 95.8 | 39.1 |
| 03 | D0 | 99.7 | 54.0 |
| | 25 °CM6 | 97.5 | 40.7 |

Note: in the table, "D" indicates day, D0 indicates the time at the beginning of the experiment, and "M" indicates month, e.g., M1 indicates one month.

**Test Example 9. Screening of Ionic Strengths for Anti-PD-1 Antibody Formulations**

The anti-PD-1 antibody (Hu23-11.IgG4AA) formulations containing 80 mg/mL sucrose and 0.6 mg/mL polysorbate 80 at a protein concentration of 120 mg/mL were prepared using sodium acetate buffer systems at pH 5.2 with ionic strengths of 10 mM and 30 mM, respectively. Excipient composition:

1) 10 mM pH 5.2 AA, 0.6 mg/mL PS80 and 80 mg/mL sucrose; or
2) 30 mM pH 5.2 AA, 0.6 mg/mL PS80 and 80 mg/mL sucrose;

The prepared formulations were filtered and filled into containers, which were plugged and capped. The stability of the samples under a forced degradation condition (40 °C M1) was investigated, and the stability of the formulations was investigated with SEC as an evaluation index. The experimental results are shown in Table 24.

The experimental data showed that under the forced degradation condition of 40 °C M1, the SEC data for the 10 mM ionic strength group were slightly higher than those for the 30 mM ionic strength group. Therefore, the ionic strength of the sodium acetate buffer system at pH 5.2 is preferably 10 mM.

Table 24. Experimental results of screening of ionic strengths

| Group | Conditions | SEC monomer (%) |
|---|---|---|
| 01 | D0 | 97.4 |
| | 40 °CM1 | 91.7 |
| 02 | D0 | 97.1 |
| | 40 °CM1 | 89.4 |

Note: in the table, "D" indicates day, D0 indicates the time at the beginning of the experiment, and "M" indicates month, e.g., M1 indicates one month.

**Test Example 10. Screening of Surfactants for Anti-PD-1 Antibody Formulations**

The anti-PD-1 antibody (Hu23-11.IgG4AA) formulations containing different concentrations of tween 80 of the following 1)-5) were prepared in a 10 mM sodium acetate salt buffer at pH 5.2. The protein concentration was 100 mg/mL. Other excipients are as follows:

1) 80 mg/mL sucrose and 0.2 mg/mL polysorbate 80 (PS80);
2) 80 mg/mL sucrose and 0.4 mg/mL PS80;
3) 80 mg/mL sucrose and 0.6 mg/mL PS80;
4) 80 mg/mL sucrose and 0.8 mg/mL PS80; and
5) 80 mg/mL sucrose and 0.6 mg/mL polysorbate 20 (PS20).

The prepared formulations were filtered and filled into containers, which were plugged and capped. The samples were subjected to shaking (25 °C, 300 rpm, 10 days) and long-term stability (2-8 °C for 6 months) experiments, and the stability of the formulations was investigated with appearance, SEC and non-reduced CE-SDS as evaluation indices. The experimental results are shown in Table 25.

The experimental results showed that under the condition of shaking D10, the appearances of the 0.6 mg/mL PS80, 0.8 mg/mL PS80 and 0.6 mg/mL PS20 groups were better than those of the 0.2 mg/mL PS80 and 0.4 mg/mL PS80 groups in which a large number of particles appeared; when the samples were stored at 2-8 °C for 6 months, the appearances of 0.6 mg/mL PS80 and 0.8 mg/mL PS80 were better than that of the 0.6 mg/mL PS20 group in which turbidity appeared, and there was no significant difference in the purities of these groups. Therefore, the surfactant is preferably polysorbate 80, preferably at a concentration of 0.6 mg/mL.

Table 25. Experimental results of screening of surfactants for formulations

| No. | Conditions | Appearance | SEC monomer (%) | Non-reduced CE-SDS (%) |
|---|---|---|---|---|
| 01 | D0 | Clarified | 95.5 | 95.1 |
| | Shaking D10 | A large number of particles | N/A | N/A |
| | 4 °CM6 | Clarified | 94.8 | 95.7 |
| 02 | D0 | Clarified | 95.4 | 94.6 |
| | Shaking D10 | A large number of particles | N/A | N/A |
| | 4 °CM6 | Clarified | 95.0 | 96.2 |
| 03 | D0 | Clarified | 95.5 | 94.4 |
| | Shaking D10 | A small number of particles | N/A | N/A |
| | 4 °CM6 | Clarified | 94.7 | 95.8 |
| 04 | D0 | Clarified | 95.5 | 94.5 |
| | Shaking D10 | A small number of particles | N/A | N/A |
| | 4 °CM6 | Clarified | 94.6 | 95.7 |
| 05 | D0 | Clarified | 95.5 | 94.6 |
| | Shaking D10 | A small number of particles | N/A | N/A |
| | 4 °CM6 | Turbid | 95.0 | 96.2 |

Note: in the table, "D" indicates day, e.g., D10 indicates 10 days; d0 indicates the time at the beginning of the experiment, and "M" indicates month, e.g., M6 indicates six months.

**Test Example 11. Screening of Osmotic Pressure Regulators for Anti-PD-1 Antibody Formulations**

The anti-PD-1 antibody (Hu23-11.IgG4AA) formulations at a protein concentration of 100 mg/mL were prepared in 10 mM sodium acetate buffers at pH 5.2 containing various kinds of excipients of the following 1)-7). The specific excipients are as follows:

1) 0.6 mg/mL polysorbate 80 (PS80);
2) 80 mg/mL sucrose and 0.6 mg/mL PS80;
3) 80 mg/mL trehalose and 0.6 mg/mL PS80;
4) 50 mg/mL sorbitol and 0.6 mg/mL PS80;
5) 100 mM arginine and 0.6 mg/mL PS80;
6) 100 mM glycine and 0.6 mg/mL PS80; and
7) 100 mM NaCl and 0.6 mg/mL PS80.

The prepared formulations were filtered and filled into containers, which were plugged and capped. The stability of the samples under a forced degradation condition (40 °C D22 (storing at a high temperature of 40 °C for 22 days)) was investigated, and the stability of the formulations was investigated with appearance, SEC and non-reduced CE-SDS as evaluation indices. The experimental results are shown in Table 26.

Under the forced degradation condition of 40 °C D22, there was no significant difference in appearance among the groups; from the purity data, it could be seen that the SEC and non-reduced CE-SDS of the formulations in the 80 mg/mL sucrose, 80 mg/mL trehalose and 50 mg/mL sorbitol groups were slightly higher than those of the formulations in the other groups, and there was no significant difference in purity among the formulations in the three groups. When the administration route is subcutaneous injection, the osmotic pressure of the formulations is controlled at 280-320 mOsm. Therefore, the content of sucrose is preferably 70-90 mg/mL. The osmotic pressure of the formulation containing 80 mg/mL sucrose is about 300 mOsm. The content of sucrose is preferably 80 mg/mL.

Table 26. Experimental results of screening of osmotic pressure for formulations

| No. | Conditions | Appearance | SEC monomer (%) | Non-reduced CE-SDS (%) |
|---|---|---|---|---|
| 01 | D0 | Clarified | 97.0 | 95.5 |
| | 40 °CD22 | Clarified | 90.2 | 87.8 |
| 02 | D0 | Clarified | 97.0 | 95.5 |
| | 40 °CD22 | Clarified | 91.3 | 89.7 |
| 03 | D0 | Clarified | 97.0 | 95.2 |
| | 40 °CD22 | Clarified | 91.2 | 89.6 |
| 04 | D0 | Clarified | 97.0 | 95.2 |
| | 40 °CD22 | Clarified | 92.0 | 90.0 |
| 05 | D0 | Clarified | 97.0 | 95.3 |
| | 40 °CD22 | Clarified | 91.8 | 87.4 |
| 06 | D0 | Clarified | 97.0 | 95.5 |
| | 40 °CD22 | Clarified | 90.1 | 88.9 |
| 07 | D0 | Clarified | 96.9 | 95.5 |
| | 40 °CD22 | Clarified | 91.0 | 87.7 |
| Note: in the table, "D" indicates day, e.g., D22 indicates 22 days, and D0 indicates the time at the beginning of the experiment. | | | | |

## Test Example 12. Screening of Protein Concentrations for Anti-PD-1 Antibody Formulations

The anti-PD-1 antibody (Hu23-11.IgG4AA) formulations containing 80 mg/mL sucrose and 0.6 mg/mL polysorbate 80 (PS80) at a protein concentration of 100 mg/mL or 120 mg/mL were prepared with a 10 mM sodium acetate buffer system at pH 5.2.

1) 100 mg/mL protein concentration, 0.6 mg/mL PS80 and 80 mg/mL sucrose; or
2) 120 mg/mL protein concentration, 0.6 mg/mL PS80 and 80 mg/mL sucrose.

Each of the formulations was filtered and filled in to a container, which was plugged and capped. The stability of the samples under a forced degradation condition (40 °C M1) was investigated, and the stability of the formulations was investigated with appearance, SEC, non-reduced CE-SDS and ICE as evaluation indices. The experimental results are shown in Table 27.

The experimental data showed that under the forced degradation condition of 40 °C M1, there was no significant difference in the appearance and purity between the 100 mg/mL and 120 mg/mL formulation groups.

Table 27. Experimental results of comparison of different concentrations of formulations

| Group | Conditions | Appearance | SEC monomer (%) | Non-reduced CE-SDS (%) | ICE neutral peak (%) |
|---|---|---|---|---|---|
| 01 | D0 | Clarified | 95.5 | 94.5 | 55.1 |
| | 40 °CM1 | Clarified | 90.2 | 87.3 | 39.6 |
| 02 | D0 | Clarified | 95.6 | 94.0 | 55.2 |
| | 40 °CM1 | Clarified | 90.1 | 86.6 | 39.9 |
| Note: in the table, "D" indicates day, D0 indicates the time at the beginning of the experiment, and "M" indicates month, e.g., M1 indicates one month. | | | | | |

**Test Example 13. Stability Test of Anti-PD-1 Antibody Formulations**

The anti-PD-1 antibody (Hu23-11.IgG4AA) formulations containing 10 mM sodium acetate at pH 5.2, 80 mg/mL sucrose and 0.6 mg/mL PS80 at a protein concentration of 120 mg/mL were prepared.

The prepared formulations were filtered and filled into containers, which were plugged and capped. The stability of the samples under a forced degradation condition (40 °C M1), 5 freeze-thaw cycles and a shaking D7 condition (25 °C, 300 rpm) was investigated, and the stability of the formulations was investigated with appearance, SEC, non-reduced CE-SDS and IEC as evaluation indices. The experimental results are shown in Table 28.

The experimental results showed that from the appearance, it could be seen that the final formulations remained clarified under each of the forced degradation conditions; from the purity data, it could be seen that there was no significant under five freeze-thaw cycles or the shaking D7 condition (25 °C, 300 rpm), and the SEC was reduced by 1.9%, the NR-CE was reduced by 2.5% and the IEC was reduced by 14.8% under the forced degradation condition of 40 °C M1, as compared to those on D0. Therefore, the formulations have good stability.

Table 28. Experimental results of stability of formulations

| Conditions | Appearance | SEC monomer (%) | Non-reduced CE-SDS (%) | IEC neutral peak (%) |
|---|---|---|---|---|
| D0 | Clarified | 99.1 | 90.9 | 63.7 |
| 5 freeze-thaw cycles | Clarified | 99.1 | 90.8 | 62.3 |
| Shaking D7 | Clarified | 98.8 | 91.9 | 62.2 |
| 40 °CM1 | Clarified | 97.2 | 88.4 | 48.9 |
| Note: in the table, "D" indicates day, D0 indicates the time at the beginning of the experiment, and "M" indicates month, e.g., M1 indicates one month. | | | | |

In addition, the stability of the formulations under accelerated degradation conditions of 25 °C M6 (storing at a temperature of 25 °C for 6 months) and 4 °C M6 (storing at a temperature of 4 °C for 6 months) was also investigated. The experimental results are shown in Table 29. The results showed that the formulations of the present disclosure have good stability under a long-term condition of 4 °C M6, and even under an accelerated degradation condition of 25 °C M6. For the formulations, the SEC was reduced by only 2.2%, the non-reduced CE-SDS was reduced by only 2.7%, and the IEC was reduced by 9.3%, as compared to those on D0.

Table 29. Experimental results of stability of formulations

| Conditions | Appearance | SEC monomer (%) | Non-reduced CE-SDS (%) | IEC neutral peak (%) |
|---|---|---|---|---|
| D0 | Clarified | 99.1 | 90.9 | 63.7 |
| 25 °CM6 | Clarified | 96.9 | 88.2 | 54.4 |
| 4 °CM6 | Clarified | 98.1 | 91.0 | 62.8 |

**Test Example 14. Experiment on Formula Optimization of Anti-PD-1 Antibody Formulations**

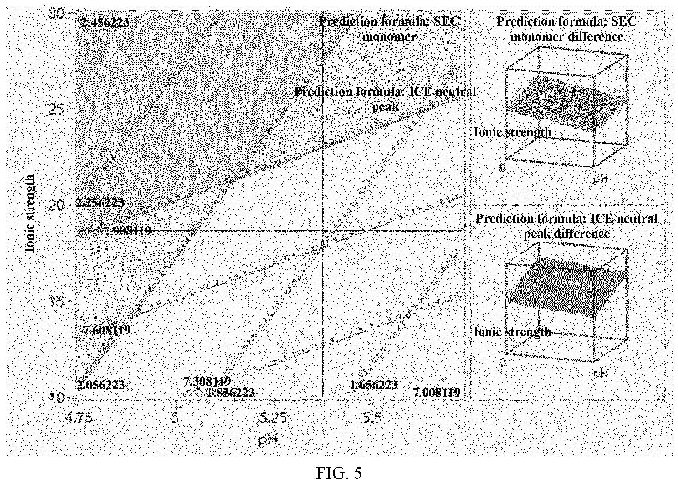

A DOE experiment was designed with the ionic strength, pH value and protein concentration of sodium acetate as variables, and the factors and levels for the DOE experiment were set as follows: ionic strength: 10-30 mM, pH: 4.7-5.7, and the concentration of the anti-PD-1 antibody (Hu23-11.IgG4AA): 90-150 mg/mL. A series of formulas were designed (see table 30) and tested under a forced degradation condition (storing at a high temperature of 40 °C for one month), and the results were statistically analyzed with appearance, SEC and IEC as evaluation indices by the least-squares method. The results are shown in Table 31 and FIG. 5.

The result showed that after the formulations were stored at a high temperature of 40 °C for one month, the appearance of each of the formulas was clarified, and in terms of purity, the SEC was reduced by 1.1%-2.7%, and the iCIEF was reduced by 5.6%-8.5%, which were within the acceptable range under the forced degradation condition. The difference among the groups was within the detection error range of the instrument. Therefore, the anti-PD-1 antibody samples have good stability in the ranges of protein concentration of 90-150 mg/mL, ionic strength of 10-30 Mm and pH of 4.7-5.7. The data were fitted, and the purity was observed to be in a safer range at low ionic strength. Therefore, a protein concentration of 120 mg/mL, an ionic strength of 10 mM, and pH of 5.2 may be preferred.

Table 30. Formula designs for DOE formula screening experiment

| Group | pH | Protein concentration (mg/mL) | Ionic strength (mM) |
|---|---|---|---|
| 1 | 5.7 | 150 | 20 |
| 2 | 4.7 | 120 | 21.9 |
| 3 | 5.2 | 120 | 10 |
| 4 | 4.7 | 90 | 10 |
| 5 | 5.2 | 120 | 20 |
| 6 | 5.2 | 90 | 20 |
| 7 | 5.7 | 150 | 10 |
| 8 | 4.7 | 90 | 30 |
| 9 | 4.7 | 150 | 10 |
| 10 | 5.2 | 150 | 30 |
| 11 | 5.7 | 90 | 10 |
| 12 | 5.7 | 107.7 | 30 |
| Note: all formulas comprise 80 mg/mL sucrose and 0.6 mg/mL PS80. | | | |

Table 31. Results of DOE formula screening experiment

| Group | Conditions | Appearance | SEC monomer (%) | SEC monomer reduction (%) | iCIEF neutral peak (%) | iCIEF neutral peak reduction (%) |
|---|---|---|---|---|---|---|
| 01 | D0 | Clarified | 98.7 | N | 59.6 | N |
| | 40 °CM1 | Clarified | 96.6 | 2.1 | 52.6 | 7.0 |
| 02 | D0 | Clarified | 99.1 | N | 60.4 | N |
| | 40 °CM1 | Clarified | 96.7 | 2.4 | 51.9 | 8.5 |
| 03 | D0 | Clarified | 98.9 | N | 60.0 | N |
| | 40 °CM1 | Clarified | 97.1 | 1.8 | 52.8 | 7.2 |
| 04 | D0 | Clarified | 99.2 | N | 59.8 | N |
| | 40 °CM1 | Clarified | 97.6 | 1.6 | 54.2 | 5.6 |
| 05 | D0 | Clarified | 98.9 | N | 60.2 | N |
| | 40 °CM1 | Clarified | 96.7 | 2.2 | 52.5 | 7.7 |
| 06 | D0 | Clarified | 99.1 | N | 60.1 | N |
| | 40 °CM1 | Clarified | 97.3 | 1.8 | 53.4 | 6.7 |
| 07 | D0 | Clarified | 98.7 | N | 59.3 | N |
| | 40 °CM1 | Clarified | 96.6 | 2.1 | 53.4 | 5.9 |
| 08 | D0 | Clarified | 99.2 | N | 59.4 | N |
| | 40 °CM1 | Clarified | 97.0 | 2.2 | 52.4 | 7.0 |
| 09 | D0 | Clarified | 99.1 | N | 59.9 | N |
| | 40 °CM1 | Clarified | 96.8 | 2.3 | 52.5 | 7.4 |
| 10 | D0 | Clarified | 98.9 | N | 59.9 | N |
| | 40 °CM1 | Clarified | 96.2 | 2.7 | 52.3 | 7.6 |

(continued)

| Group | Conditions | Appearance | SEC monomer (%) | SEC monomer reduction (%) | iCIEF neutral peak (%) | iCIEF neutral peak reduction (%) |
|---|---|---|---|---|---|---|
| 11 | D0 | Clarified | 98.7 | N | 59.6 | N |
| | 40 °CM1 | Clarified | 97.6 | 1.1 | 53.3 | 6.3 |
| 12 | D0 | Clarified | 98.7 | N | 60.7 | N |
| | 40 °CM1 | Clarified | 97.3 | 1.4 | 52.8 | 7.9 |

Note: in the table, "D" indicates day, D0 indicates the time at the beginning of the experiment, "M" indicates month, e.g. M1 indicates one month; N indicates not applicable to this table; SEC monomer reduction (%) = SEC monomer (%) of the formulation on D0 - SEC monomer (%) of the formulation at 40 °C M1; iCIEF neutral peak reduction (%) = iCIEF neutral peak (%) on D0 - iCIEF neutral peak (%) at 40 °C M1.

**Test Example 15. Freeze-Drying of Anti-PD-1 Antibody Formulations**

The anti-PD-1 antibody (Hu23-11.IgG4AA) formulations containing 100 mg/mL antibody, 80 mg/mL sucrose and 0.6 mg/mL PS80 were prepared in a 10 mM AA buffer at pH 5.2, and the formulation samples were freeze-dried according to the procedures of pre-freezing, primary drying, and secondary drying (see Table 32 for parameters). After the freeze-drying procedures were completed, plugging was performed in vacuum. The samples were reconstituted for comparison of formulations before and after freeze-drying. The results showed that the reconstituted solution was able to retain good performance of the liquid formulation.

Table 32. Freeze-drying procedures

| Freeze-drying procedures | Set temperature | Set time (min) | Retention time (min) | Degree of vacuum (mBar) |
|---|---|---|---|---|
| Pre-freezing | 5 °C | 10 | 60 | N/A |
| | -45 °C | 50 | 120 | N/A |
| Primary drying | -10 °C | 120 | 1800 | 0.10 |
| Secondary drying | 25 °C | 60 | 1 | 0.10 |
| Secondary drying | 25 °C | 1 | 360 | 0.01 |

Although the foregoing invention has been described in detail by way of drawings and examples for purposes of clarity of understanding, the description and examples should not be construed as limiting the scope of the present disclosure. The disclosures of all patents and scientific literature cited herein are clearly incorporated by reference in their entirety.

**Claims**

1. A pharmaceutical composition comprising an anti-PD-1 antibody and a buffer, wherein,

the buffer is an acetate buffer, a histidine salt buffer or a succinate buffer, and preferably, the buffer has a pH of about 4.5 to about 6.0, preferably a pH of about 4.7 to about 5.7, and more preferably a pH of about 5.2; wherein the acetate buffer is preferably an acetic acid-sodium acetate buffer; the histidine salt buffer is preferably a histidine-acetate buffer, and the succinate buffer is preferably a succinic acid-sodium succinate buffer; the anti-PD-1 antibody comprises a heavy chain variable region and a light chain variable region, wherein the heavy chain variable region comprises an HCDR1 set forth in SEQ ID NO: 65, an HCDR2 set forth in SEQ ID NO: 66, and an HCDR3 set forth in SEQ ID NO: 67, and the light chain variable region comprises an LCDR1 set forth in SEQ ID NO: 68, an LCDR2 set forth in SEQ ID NO: 12, and an LCDR3 set forth in SEQ ID NO: 69; preferably, the heavy chain variable region comprises an HCDR1 set forth in SEQ ID NO: 8, an HCDR2 set forth in SEQ ID NO: 9, and an HCDR3 set forth in SEQ ID NO: 10, and the light chain variable region comprises an LCDR1 set forth in SEQ ID NO: 49, an LCDR2 set forth in SEQ ID NO: 12, and an LCDR3 set forth in SEQ ID NO: 13.

2. The pharmaceutical composition according to claim 1, wherein the buffer has a concentration of about 5 mM to about 30 mM, preferably about 10 mM to about 30 mM, and more preferably about 10 mM.

3. The pharmaceutical composition according to claim 1 or 2, wherein the anti-PD-1 antibody has a concentration of about 1 mg/mL to about 150 mg/mL, preferably about 90 mg/mL to about 150 mg/mL, and more preferably about 120 mg/mL or about 100 mg/mL.

4. The pharmaceutical composition according to any one of claims 1 to 3, further comprising a surfactant, wherein the surfactant is preferably polysorbate, and more preferably polysorbate 80; the surfactant preferably has a concentration of about 0.2 mg/mL to about 0.8 mg/mL, more preferably about 0.6 mg/mL to about 0.8 mg/mL, and most preferably about 0.6 mg/mL.

5. The pharmaceutical composition according to any one of claims 1 to 4, further comprising an osmotic pressure regulator, wherein preferably, the osmotic pressure regulator is a sugar, an amino acid and/or a salt; and more preferably, the osmotic pressure regulator is selected from one or more of the group consisting of sucrose, trehalose, sorbitol, arginine, glycine and sodium chloride; the osmotic pressure regulator preferably has a concentration of about 50 mg/mL to about 100 mg/mL, more preferably about 70 mg/mL to about 90 mg/mL; and most preferably about 80 mg/mL.

6. The pharmaceutical composition according to any one of claims 1 to 5, wherein the anti-PD-1 antibody comprises a heavy chain variable region set forth in SEQ ID NO: 27 or having at least 90% sequence identity to SEQ ID NO: 27, and a light chain variable region set forth in SEQ ID NO: 55 or having at least 90% sequence identity to SEQ ID NO: 55; preferably, the anti-PD-1 antibody has a heavy chain variable region set forth in SEQ ID NO: 27 and a light chain variable region set forth in SEQ ID NO: 55.

7. The pharmaceutical composition according to any one of claims 1 to 6, wherein the anti-PD-1 antibody comprises a heavy chain constant region and/or a light chain constant region; wherein preferably, the heavy chain constant region is selected from the group consisting of constant regions of human IgG1, IgG2, IgG3 and IgG4 and conventional variants thereof, and the light chain constant region is selected from the group consisting of constant regions of human antibody κ and λ chains and conventional variants thereof; more preferably, the anti-PD-1 antibody comprises a heavy chain constant region set forth in SEQ ID NO: 72 and a light chain constant region set forth in SEQ ID NO: 73; and most preferably, the anti-PD-1 antibody comprises: a heavy chain set forth in SEQ ID NO: 74 or having at least 85% sequence identity to SEQ ID NO: 74, and a light chain set forth in SEQ ID NO: 75 or having at least 85% sequence identity to SEQ ID NO: 75.

8. The pharmaceutical composition according to any one of claims 1 to 7, comprising:

a) an anti-PD-1 antibody having a concentration of about 1 mg/mL to about 150 mg/mL,
b) an acetate buffer having a concentration of about 5 mM to about 30 mM and a pH of about 4.5 to about 6.0,
c) an osmotic pressure regulator having a concentration of about 50 mg/mL to about 100 mg/mL, wherein the osmotic pressure regulator is selected from the group consisting of sucrose, trehalose, sorbitol, arginine, glycine and sodium chloride; and
d) polysorbate having a concentration of about 0.2 mg/mL to about 0.8 mg/mL; wherein preferably, the pharmaceutical composition comprises:

A1) an anti-PD-1 antibody having a concentration of about 90 mg/mL to about 150 mg/mL,
B1) an acetate buffer having a concentration of about 10 mM to about 30 mM and a pH of about 4.7 to about 5.7,
C1) sucrose having a concentration of about 70 mg/mL to about 90 mg/mL, and
D1) polysorbate 80 having a concentration of about 0.6 mg/mL to about 0.8 mg/mL; and more preferably, the pharmaceutical composition comprises: an acetic acid-sodium acetate buffer having a concentration of about 10 mM and a pH of about 5.2, an anti-PD-1 antibody having a concentration of about 120 mg/mL, sucrose having a concentration of about 80 mg/mL, and polysorbate 80 having a concentration of about 0.6 mg/mL.

9. A pharmaceutical composition, comprising: an acetic acid-sodium acetate buffer having a concentration of 10 mM and a pH of 5.2, an anti-PD-1 antibody having a concentration of 120 mg/mL, sucrose having a concentration of 80 mg/mL, and polysorbate 80 having a concentration of 0.6 mg/mL; wherein the anti-PD-1 antibody has a heavy chain set forth in SEQ ID NO: 74 and a light chain set forth in SEQ ID NO: 75.

10. A method for preparing the pharmaceutical composition according to any one of claims 1 to 9, comprising the step of buffer-exchanging an anti-PD-1 antibody stock solution.

11. A lyophilized formulation comprising an anti-PD-1 antibody, obtained by freeze-drying the pharmaceutical composition according to any one of claims 1 to 9.

12. A reconstituted solution comprising an anti-PD-1 antibody, obtained by reconstituting the lyophilized formulation according to claim 11.

13. A lyophilized formulation comprising an anti-PD-1 antibody, being able to form the pharmaceutical composition according to any one of claims 1 to 9 after reconstitution.

14. An article of manufacture, comprising a container containing the pharmaceutical composition according to any one of claims 1 to 9, or the lyophilized formulation according to claim 11 or 13, or the reconstituted solution according to claim 12.

15. A method for treating or preventing a disease or disorder, comprising administering to a subject an effective amount of the pharmaceutical composition according to any one of claims 1 to 9, or the lyophilized formulation according to claim 11 or 13, or the reconstituted solution according to claim 12, or the article of manufacture according to claim 14; wherein preferably, the disease or disorder is a tumor; more preferably, the disease is selected from the group consisting of: head and neck squamous cell carcinoma, head and neck cancer, brain cancer, neuroglioma, glioblastoma multiforme, neuroblastoma, central nervous system carcinoma, neuroendocrine tumor, throat cancer, nasopharyngeal cancer, esophageal cancer, thyroid cancer, malignant pleural mesothelioma, lung cancer, breast cancer, liver cancer, hepatoma, hepatobiliary cancer, pancreatic cancer, stomach cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, leukemia, lymphoma, bone cancer, chondrosarcoma, myeloma, multiple myeloma, myelodysplastic syndrome, myeloproliferative tumor, squamous cell carcinoma, Ewing's sarcoma, systemic light chain amyloidosis and Merkel cell carcinoma; and most preferably, the disease is selected from the group consisting of PD-L1-positive melanoma, lung cancer, non-small cell lung cancer, breast cancer, stomach cancer, kidney cancer, bladder cancer, intestinal cancer and colon cancer; optionally, the disease is a PD-1-related disease.

FIG. 1

**FIG. 2**

**FIG. 3**

**FIG. 4**

FIG. 5

## INTERNATIONAL SEARCH REPORT

| | International application No. |
|---|---|
| | **PCT/CN2021/109438** |

**A. CLASSIFICATION OF SUBJECT MATTER**

A61K 39/395(2006.01)i;  A61P 35/00(2006.01)i

According to International Patent Classification (IPC) or to both national classification and IPC

**B. FIELDS SEARCHED**

Minimum documentation searched (classification system followed by classification symbols)

A61K, A61P

Documentation searched other than minimum documentation to the extent that such documents are included in the fields searched

Electronic data base consulted during the international search (name of data base and, where practicable, search terms used)

CNABS, CNTXT, DWPI, SIPOABS, CATXT, USTXT, EPTXT, WOTXT, CPEA, CNKI, PUBMED, WEB OF SCIENCE, ELSEVIER, 百度学术, GenBank, 中国专利生物序列检索系统: anti-PD-1, PD-1, 抗体, 表面活性剂, 缓冲液, 聚山梨酯80, 蔗糖, 海藻糖, 渗透压调节剂, 等渗剂, antibody, surfactant, buffer, buffering, polysorbate 80, tween, sucrose, trehalose, osmotic, isotonic, search based on SEQ ID NOs: 8-10, 12, 13, 27, 49, 55, 65-69, 74 and 75

**C. DOCUMENTS CONSIDERED TO BE RELEVANT**

| Category* | Citation of document, with indication, where appropriate, of the relevant passages | Relevant to claim No. |
|---|---|---|
| A | CN 110787292 A (SHANGHAI HENLIUS BIOTECH CO., LTD.) 14 February 2020 (2020-02-14)<br>see entire document | 1-15 |
| A | WO 2019206987 A1 (MEDIMMUNE LTD.) 31 October 2019 (2019-10-31)<br>see entire document | 1-15 |
| A | CN 109966487 A (SHANGHAI HENLIUS BIOLOGICAL PHARMACEUTICAL CO., LTD. et al.) 05 July 2019 (2019-07-05)<br>see entire document | 1-15 |
| A | WO 2017054646 A1 (JIANGSU HENGRUI MEDICINE CO., LTD. et al.) 06 April 2017 (2017-04-06)<br>see entire document | 1-15 |
| A | CN 110869002 A (MERCK SHARP & DOHME CORP.) 06 March 2020 (2020-03-06)<br>see entire document | 1-15 |

☑ Further documents are listed in the continuation of Box C.   ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **18 October 2021** | **04 November 2021** |

| Name and mailing address of the ISA/CN | Authorized officer |
|---|---|
| **China National Intellectual Property Administration (ISA/ CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2021/109438** |

### C. DOCUMENTS CONSIDERED TO BE RELEVANT

| Category* | Citation of document, with indication, where appropriate, of the relevant passages | Relevant to claim No. |
| --- | --- | --- |
| A | CN 106390115 A (SHANGHAI JUNSHI BIOSCIENCES CO., LTD. et al.) 15 February 2017 (2017-02-15) see entire document | 1-15 |
| A | WO 2020097139 A1 (MERCK SHARP & DOHME et al.) 14 May 2020 (2020-05-14) see entire document | 1-15 |
| A | CN 110974958 A (BEIJING ORIENTAL 100 TAI BIOTECHNOLOGY CO., LTD.) 10 April 2020 (2020-04-10) see entire document | 1-15 |

Form PCT/ISA/210 (second sheet) (January 2015)

**INTERNATIONAL SEARCH REPORT**

International application No.

**PCT/CN2021/109438**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

| International application No. |
| --- |
| **PCT/CN2021/109438** |

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **15**
   because they relate to subject matter not required to be searched by this Authority, namely:

   [1]  Claim 15 relates to a method for treating or preventing diseases or conditions. The subject matters thereof are all subject matters that do not warrant an international search according to the criteria set out in PCT Rule 39.1(iv). The subject matter of claim 15 can be amended by reasonable expectation to be a use of an effective dose of the pharmaceutical composition described in any one of claims 1-9, the lyophilized preparation described in claim 11 or 13, the reconstituted solution described in claim 12, or the product described in claim 14 in preparing a drug for treating or preventing diseases or conditions. The international search is provided on the basis of such reasonable expectation.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Patent document cited in search report | | | Publication date (day/month/year) | Patent family member(s) | | | Publication date (day/month/year) |
|---|---|---|---|---|---|---|---|
| CN | 110787292 | A | 14 February 2020 | CN | 110787292 | B | 24 April 2020 |
| WO | 2019206987 | A1 | 31 October 2019 | US | 2021054079 | A1 | 25 February 2021 |
| | | | | IL | 278151 | D0 | 30 November 2020 |
| | | | | BR | 112020020390 | A2 | 19 January 2021 |
| | | | | CN | 112004553 | A | 27 November 2020 |
| | | | | AU | 2019258330 | A1 | 10 December 2020 |
| | | | | EP | 3784279 | A1 | 03 March 2021 |
| | | | | SG | 11202010324 T | A | 27 November 2020 |
| | | | | AR | 115365 | A1 | 13 January 2021 |
| | | | | CA | 3096993 | A1 | 31 October 2019 |
| | | | | EA | 202092508 | A1 | 16 March 2021 |
| | | | | KR | 20210005096 | A | 13 January 2021 |
| CN | 109966487 | A | 05 July 2019 | None | | | |
| WO | 2017054646 | A1 | 06 April 2017 | TW | I721020 | B | 11 March 2021 |
| | | | | AU | 2016329960 | A1 | 26 April 2018 |
| | | | | CA | 2999079 | A1 | 06 April 2017 |
| | | | | BR | 112018005349 | A2 | 09 October 2018 |
| | | | | CN | 106999591 | A | 01 August 2017 |
| | | | | RU | 2018110333 | A | 28 October 2019 |
| | | | | CN | 106999591 | B | 23 February 2021 |
| | | | | KR | 20180054791 | A | 24 May 2018 |
| | | | | RU | 2731418 | C2 | 02 September 2020 |
| | | | | EP | 3357508 | A1 | 08 August 2018 |
| | | | | US | 2018339045 | A1 | 29 November 2018 |
| | | | | MX | 2018003306 | A | 16 May 2018 |
| | | | | RU | 2018110333 | A3 | 30 December 2019 |
| | | | | US | 2021000954 | A1 | 07 January 2021 |
| | | | | TW | 201711699 | A | 01 April 2017 |
| | | | | US | 10786567 | B2 | 29 September 2020 |
| | | | | EP | 3357508 | A4 | 24 April 2019 |
| | | | | JP | 2018532730 | A | 08 November 2018 |
| CN | 110869002 | A | 06 March 2020 | PH | 12019502484 | A1 | 20 July 2020 |
| | | | | NI | 201900112 | A | 04 February 2020 |
| | | | | JO | P20190260 | A1 | 16 June 2017 |
| | | | | AU | 2018263862 | A1 | 21 November 2019 |
| | | | | SG | 11201910182 R | A | 28 November 2019 |
| | | | | MX | 2019013028 | A | 05 February 2020 |
| | | | | CO | 2019012151 | A2 | 07 February 2020 |
| | | | | JP | 2020518599 | A | 25 June 2020 |
| | | | | US | 2020147213 | A1 | 14 May 2020 |
| | | | | EP | 3618808 | A1 | 11 March 2020 |
| | | | | CA | 3062160 | A1 | 08 November 2018 |
| | | | | WO | 2018204368 | A1 | 08 November 2018 |
| | | | | PE | 20200513 | A1 | 05 March 2020 |
| | | | | EC | SP19078502 | A | 27 December 2019 |
| | | | | CL | 2019003142 | A1 | 10 July 2020 |
| | | | | CR | 20190497 | A | 20 January 2020 |
| | | | | EP | 3618808 | A4 | 26 May 2021 |
| | | | | KR | 20190142392 | A | 26 December 2019 |
| | | | | EA | 201992590 | A1 | 08 April 2020 |

Form PCT/ISA/210 (patent family annex) (January 2015)

**INTERNATIONAL SEARCH REPORT**
Information on patent family members

International application No.

**PCT/CN2021/109438**

| Patent document cited in search report | | | Publication date (day/month/year) | Patent family member(s) | | | Publication date (day/month/year) |
|---|---|---|---|---|---|---|---|
| | | | | BR | 112019022972 | A2 | 26 May 2020 |
| CN | 106390115 | A | 15 February 2017 | | None | | |
| WO | 2020097139 | A1 | 14 May 2020 | AU | 2019377456 | A1 | 27 May 2021 |
| | | | | CA | 3118144 | A1 | 14 May 2020 |
| CN | 110974958 | A | 10 April 2020 | CN | 110974958 | B | 21 August 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2020074098 W
- US 4683195 A
- WO 2015085847 A

### Non-patent literature cited in the description

- *J. biol. chem,* 1968, vol. 243, 3558
- **WARD et al.** *Nature,* 1989, vol. 341, 544-546
- **BIRD et al.** *Science,* 1988, vol. 242, 423-426
- **HUSTON et al.** *Proc. Natl. Acad. Sci USA,* 1988, vol. 85, 5879-5883
- **KABAT et al.** Sequences of Proteins of Immunological Interest. Public Health Service, National Institutes of Health, 1991
- **AL-LAZIKANI et al.** *JMB,* 1997, vol. 273, 927-948
- **LEFRANC M. P.** *Immunologist,* 1999, vol. 7, 132-136
- **LEFRANC, M. P. et al.** *Dev. Comp. Immunol.,* 2003, vol. 27, 55-77
- **KABAT, E. A. et al.** Sequences of Proteins of Immunological Interest. 1991
- Epitope Mapping Protocols in Methods in Molecular Biology. 1996, vol. 66
- **STAHLI et al.** *Methods in Enzymology,* 1983, vol. 9, 242-253
- **KIRKLAND et al.** *J. Immunol.,* 1986, vol. 137, 3614-3619
- **HARLOW ; LANE.** Antibodies: A Laboratory Manual. Cold Spring Harbor Press, 1988
- **MOREL et al.** *Molec. Immunol.,* 1988, vol. 25, 7-15
- **CHEUNG et al.** *Virology,* 1990, vol. 176, 546-552
- **MOLDENHAUER et al.** *Scand. J. Immunol.,* 1990, vol. 32, 77-82
- Antibodies: A Laboratory Manual. Cold Spring Harbor Press
- **WATSON et al.** Molecular Biology of the Gene. The Benjamin/Cummings Pub. Co, 1987, 224
- **ALTSCHUL, S.F. et al.** *J. Mol. Biol.,* 1990, vol. 215, 403-410
- **GISH, W. et al.** *Nature Genet.,* 1993, vol. 3, 266-272
- **MADDEN, T.L. et al.** *Meth. Enzymol.,* 1996, vol. 266, 131-141
- **ALTSCHUL, S.F. et al.** *Nucleic Acids Res.,* 1997, vol. 25, 3389-3402
- **ZHANG, J. et al.** *Genome Res.,* 1997, vol. 7, 649-656
- **MULLIS et al.** *Cold Spring Harbor Symp. Quant. Biol.,* 1987, vol. 51, 263
- PCR TECHNOLOGY. Stockton Press, 1989